# EUROPÄISCHE PATENTSCHRIFT

(11) **EP 3 114 102 B1**
(45) Veröffentlichungstag und Bekanntmachung des Hinweises auf die Patenterteilung: **06.12.2017**
(21) Anmeldenummer: 15703460.4
(22) Anmeldetag: 09.02.2015
(51) Int. Cl.: C07C 15/60, H01L 51/50, H01L 51/00, C07C 211/61, C09K 11/06, C07D 333/76, C07C 209/60, C07C 209/68, C07C 211/54, C07D 209/86, C07D 307/91

(54) **MATERIALIEN FÜR ELEKTRONISCHE VORRICHTUNGEN**
MATERIALS FOR ELECTRONIC DEVICES
MATERIAUX POUR DISPOSITIFS ELECTRONIQUES

(30) Priorität: 07.03.2014 EP 14000814
(43) Veröffentlichungstag der Anmeldung: 11.01.2017
(73) Patentinhaber: Merck Patent GmbH, 64293 Darmstadt (DE)
(72) Erfinder: MUJICA-FERNAUD, Teresa, 64283 Darmstadt (DE); MONTENEGRO, Elvira, 69469 Weinheim (DE); PFISTER, Jochen, 64665 Alsbach-Haehnlein (DE)
(86) Internationale Anmeldenummer: PCT/EP2015/000271
(87) Internationale Veröffentlichungsnummer: WO 2015/131976

(56) Entgegenhaltungen:
- WO-A1-2005/104264
- WO-A1-2011/136482
- WO-A1-2011/136484
- WO-A1-2013/182263
- CN-A- 101 987 822
- DE-A1- 10 109 463
- US-A1- 2010 109 555
- US-A1- 2012 199 820

## Beschreibung

Die vorliegenden Anmeldung betrifft eine Phenanthrenyl-Arylaminoverbindung einer unten näher definierten Formel (I). Die Verbindung wird bevorzugt in einer elektronischen Vorrichtung verwendet, besonders bevorzugt in einer organischen Elektrolumineszenzvorrichtung (OLED).

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs verstanden.

Der Aufbau von OLEDs, in denen organische Verbindungen als funktionelle Materialien eingesetzt werden, ist beispielsweise in US 4539507, US 5151629, EP 0676461 und WO 98/27136 beschrieben. Allgemein werden unter der Bezeichnung OLEDs elektronische Vorrichtungen verstanden, welche eine oder mehrere Schichten enthaltend organische Verbindungen aufweisen und unter Anlegen von elektrischer Spannung Licht emittieren.

Einen großen Einfluss auf die Leistungsdaten von elektronischen Vorrichtungen haben Schichten mit lochtransportierender Funktion, wie beispielsweise Lochinjektionsschichten, Lochtransportschichten, Elektronenblockierschichten und emittierende Schichten.

Es ist im Stand der Technik bekannt, Triarylamine als Materialien mit lochtransportierenden Eigenschaften in den oben genannten Schichten einzusetzen. Diese können Mono-Triarylamine darstellen, wie beispielsweise in JP 1995/053955, WO 2006/123667 und JP 2010/222268 beschrieben, oder Bis- oder andere Oligoamine darstellen, wie beispielsweise in US 7504163 oder US 2005/0184657 beschrieben. Bekannte Beispiele für Triarylamin-Verbindungen als Materialien mit lochtransportierenden Eigenschaften für OLEDs sind unter anderem Tris-p-biphenyl-amin, N,N'-Di-1-naphthyl-N,N'-diphenyl-1,1'-biphenyl-4,4'-diamin (NPB) und 4,4',4"-Tris-(3-methylphenylphenylamino)triphenylamin (MTDATA).

Weiterhin im Stand der Technik bekannt ist die Verwendung von Phenanthrenyl-Arylaminoverbindungen in OLEDs, unter anderem als Lochtransportmaterialien (WO 2013/182263 und C. Schmitz et al., Advanced Materials 2011, 11, 821). Die in den genannten Dokumenten offenbarten Phenanthren-Derivate sind alle in der 3-Position des Phenanthrens mit einer Arylaminogruppe substituiert.

Auch wenn die in den oben genannten Dokumenten offenbarten Verbindungen gut geeignet zur Verwendung in elektronischen Vorrichtungen sind, besteht doch weiterhin Bedarf an neuen Verbindungen für diese Verwendung. Insbesondere besteht Bedarf an Verbindungen, die zu einer Verbesserung der Leistungsdaten der elektronischen Vorrichtung, insbesondere zu einer Verbesserung von Lebensdauer, Effizienz und Betriebsspannung, führen. Insbesondere zur Verwendung in lochtransportierenden Schichten der elektronischen Vorrichtungen werden kontinuierlich neue Materialien mit entsprechenden Eigenschaften gesucht.

Im Rahmen von Untersuchungen zu neuartigen Materialien für diese Verwendung wurde nun überraschend gefunden, dass sich Phenanthren-Derivate, die mindestens eine Arylaminogruppe in 1- oder in 4-Position am Phenanthren-Grundgerüst aufweisen und in untenstehender Formel (I) näher definiert sind, hervorragend zur Verwendung in OLEDs eignen, insbesondere in einer lochtransportierenden Schicht.

Die gefundenen Verbindungen weisen eine oder mehrere Eigenschaften gewählt aus sehr guten lochleitenden Eigenschaften, sehr guten elektronenblockierenden Eigenschaften, hoher Glasübergangstemperatur, hoher Oxidationsstabilität, guter Löslichkeit, geringer Kristallinität und hoher Temperaturstabilität auf.

Gegenstand der vorliegenden Erfindung ist eine Verbindung der Formel (I) 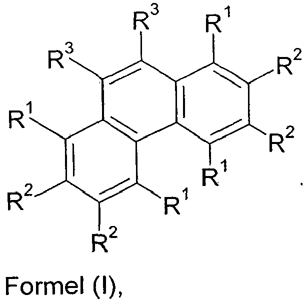 wobei für die auftretenden Symbole gilt:
- R¹: ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁶ substituiert sein können, heteroaromatischen Ringsystemen mit 5 bis 40 aromatischen Ringatomen, die mit einem oder mehreren Resten R⁶ substituiert sein können, Gruppen der Formel (A) 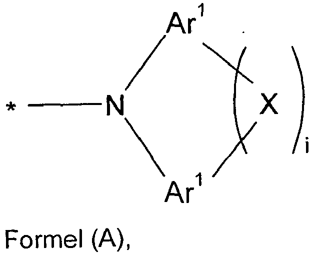 und Gruppen der Formel (B) 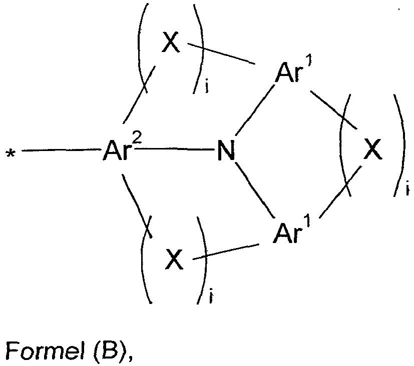 wobei die genannten Alkyl-, Alkoxy-, Alkenyl- und Alkinylgruppen jeweils mit einem oder mehreren Resten R⁶ substituiert sein können,und
wobei eine oder mehrere CH₂-Gruppen in den genannten Alkyl-, Alkoxy-, Alkenyl- und Alkinylgruppen durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können, und
wobei die mit * markierte Bindung in Formel (A) und Formel (B) jeweils die Bindung an die Phenanthren-Einheit bezeichnet;
- R²,R³: sind bei jedem Auftreten gleich oder verschieden gewählt aus H, D, F, C(=O)R⁶, CN, Si(R⁶)₃, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁶ substituiert
sein können, und heteroaromatischen Ringsystemen mit 5 bis 40 aromatischen Ringatomen, die mit einem oder mehreren Resten R⁶ substituiert sein können,
wobei die genannten Alkyl-, Alkoxy-, Alkenyl- und Alkinylgruppen jeweils mit einem oder mehreren Resten R⁶ substituiert sein können,und
wobei eine oder mehrere CH₂-Gruppen in den genannten Alkyl-, Alkoxy-, Alkenyl- und Alkinylgruppen durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
- R⁶: ist bei jedem Auftreten gleich oder verschieden gewählt aus H, D, F, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclische nAlkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁷ substituiert sein können, und heteroaromatischen Ringsystemen mit 5 bis 40 aromatischen Ringatomen, die mit einem oder mehreren Resten R⁷ substituiert sein können,
wobei die genannten Alkyl-, Alkoxy-, Alkenyl- und Alkinylgruppen jeweils mit einem oder mehreren Resten R⁷ substituiert sein können,
wobei eine oder mehrere CH₂-Gruppen in den genannten Alkyl-, Alkoxy-, Alkenyl- und Alkinylgruppen durch -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können, und
wobei zwei oder mehr Substituenten R⁶ miteinander verknüpft sein können und einen Ring bilden können;
- R⁷: ist bei jedem Auftreten gleich oder verschieden gewählt aus H, D, F, CN, und aliphatischen, aromatischen oder heteroaromatischen organischen Resten mit 1 bis 20 C-Atomen, in denen auch ein oder mehrere H-Atome durch D, F oder CN ersetzt sein können;
- Ar¹: ist bei jedem Auftreten gleich oder verschieden gewählt aus aromatischen Ringsystemen mit 6 bis 40 aromatischen Ringatomen, die mit einem oder mehreren Resten R⁴ substituiert sein können, und heteroaromatischen Ringsystemen mit 5 bis 40 aromatischen Ringatomen, die mit einem oder mehreren Resten R⁴ substituiert sein können;
- Ar²: ist bei jedem Auftreten gleich oder verschieden gewählt aus aromatischen Ringsystemen mit 6 bis 40 aromatischen Ringatomen, die mit einem oder mehreren Resten R⁴ substituiert sein können, und heteroaromatischen Ringsystemen mit 5 bis 40 aromatischen Ringatomen, die mit einem oder mehreren Resten R⁴ substituiert sein können;
- R⁴: ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁶ substituiert sein können, und heteroaromatischen Ringsystemen mit 5 bis 40 aromatischen Ringatomen, die mit einem oder mehreren Resten R⁶ substituiert sein können,
wobei die genannten Alkyl-, Alkoxy-, Alkenyl- und Alkinylgruppen jeweils mit einem oder mehreren Resten R⁶ substituiert sein können,
wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können, und
wobei zwei oder mehr Substituenten R⁴ miteinander verknüpft sein können und einen Ring bilden können;
- X: ist bei jedem Auftreten gleich oder verschieden gewählt aus Einfachbindungen, BR⁵, C(R⁵)₂, -C(R⁵)₂-C(R⁵)₂-, -C(R⁵)=C(R⁵)-, - C(R⁵)₂-O-, -C(R⁵)₂-NR⁵-, Si(R⁵)₂, C=O, NR⁵, PR⁵, P(=O)R⁵, O, S, S=O, SO₂ und wahlweise mit Resten R⁵ substituiertem ortho-Phenylen;
- R⁵: ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclische nAlkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁶ substituiert sein können, und heteroaromatischen Ringsystemen mit 5 bis 40 aromatischen Ringatomen, die mit einem oder mehreren Resten R⁶ substituiert sein können,
wobei die genannten Alkyl-, Alkoxy-, Alkenyl- und Alkinylgruppen jeweils mit einem oder mehreren Resten R⁶ substituiert sein können,
wobei eine oder mehrere CH₂-Gruppen in den genannten Alkyl-, Alkoxy-, Alkenyl- und Alkinylgruppen durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können, und
wobei zwei oder mehr Substituenten R⁵ miteinander verknüpft sein können und einen Ring bilden können;
- i: ist bei jedem Auftreten gleich oder verschieden 0 oder 1,
wobei mindestens ein Rest R¹ in der Verbindung der Formel (I) gewählt ist aus Gruppen der Formel (A) und Gruppen der Formel (B).

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol,.1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von den oben unter Aryl- und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyt, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Es ist bevorzugt, dass die Verbindung der Formel (I) neben der Phenanthren-Gruppe keine weitere kondensierte Arylgruppe mit mehr als 10 aromatischen Ringatomen enthält. Besonders bevorzugt enthält die Verbindung der Formel (I) neben der Phenanthren-Gruppe keine weitere kondensierte Arylgruppe mit mehr als 10 aromatischen Ringatomen und keine kondensierte Heteroarylgruppe mit mehr als 12 aromatischen Ringatomen.

Unter dem Begriff kondensierte Aryl- bzw. Heteroarylgruppe wird im Sinne der vorliegenden Anmeldung eine Gruppe verstanden, die aus zwei oder mehr miteinander kondensierten einfachen aromatischen bzw. heteroaromatischen Ringen besteht. Beispiele für eine kondensierte Arylgruppe sind Naphthalin, Anthracen und Phenanthren. Beispiele für eine kondensierte Heteroarylgruppe sind Acridin und Phenanthridin.

Es ist bevorzugt, dass mindestens ein Rest R¹ in der Verbindung der Formel (I) gewählt ist aus Gruppen der Formel (A). Besonders bevorzugt ist genau ein Rest R¹ in der Verbindung der Formel (I) gewählt aus Gruppen der Formel (A), oder es sind genau zwei Reste R¹ in der Verbindung der Formel (I) gewählt aus Gruppen der Formel (A).

Es ist bevorzugt, dass genau ein Rest R¹ in der Verbindung der Formel (I) gewählt ist aus Gruppen der Formel (A) und Gruppen der Formel (B), oder dass genau zwei Reste R¹ in der Verbindung der Formel (I) gewählt sind aus Gruppen der Formel (A) und Gruppen der Formel (B).

Für den Fall, dass genau zwei Reste R¹ in der Verbindung der Formel (I) gewählt sind aus Gruppen der Formel (A) und Gruppen der Formel (B), ist es bevorzugt, dass genau einer dieser Reste R¹ in 1-Position am Phenanthren gebunden ist und genau einer dieser Reste R¹ in 4-Position am Phenanthren gebunden ist.

Weiterhin ist es bevorzugt, dass der Fall ausgeschlossen ist, in dem sowohl ein Rest R¹ in 4-Position am Phenanthren als auch ein Rest R¹ in 5-Position am Phenanthren ausgewählt ist aus Gruppen der Formel (A) und Gruppen der Formel (B).

Weiterhin ist es bevorzugt, dass der Fall ausgeschlossen ist, in dem sowohl ein Rest R¹ in 1-Position am Phenanthren als auch ein Rest R¹ in 8-Position am Phenanthren ausgewählt ist aus Gruppen der Formel (A) und Gruppen der Formel (B).

Die Nummerierung der Positionen am Phenanthren-Grundkörper ist im Rahmen der vorliegenden Anmeldung folgende:

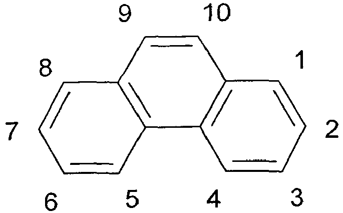

Es ist bevorzugt, dass ein oder zwei Indices i pro Gruppe der Formel (B) gleich 1 sind, und es ist besonders bevorzugt, dass genau ein Index i pro Gruppe der Formel (B) gleich 1 ist.

Ganz besonders bevorzugt ist der Index i bei jedem Auftreten gleich 0.

Bevorzugte Ausführungsformen der Gruppe (A) sind gewählt aus den folgenden Formeln (A-1) bis (A-7)

| | |
|---|---|
| 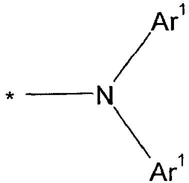 | 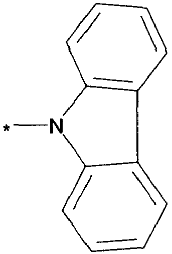 |
| Formel (A-1) | Formel (A-2) |
| | |
| 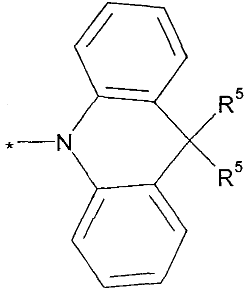 | 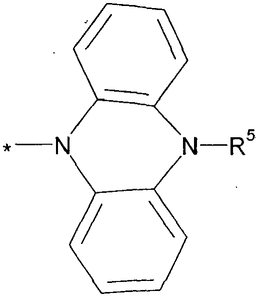 |
| Formel (A-3) | Formel (A-4) |
| 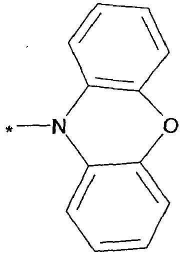 | 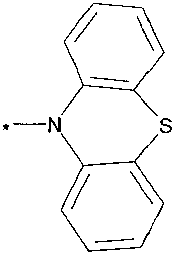 |
| Formel (A-5) | Formel (A-6) |
| | |
| 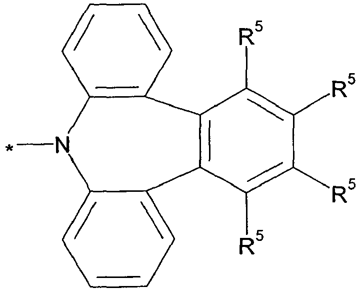 | |
| Formel (A-7) | |

welche an den freien Positionen jeweils mit Resten R⁴ substituiert sein können, und wobei die mit * markierte Bindung die Anbindungsposition an den Phenanthrenyl-Grundkörper darstellt.

Besonders bevorzugt ist darunter die Formel (A-1).

Bevorzugte Ausführungsformen der Gruppe (B) sind gewählt aus den folgenden Formeln (B-1) bis (B-15)

| | |
|---|---|
| 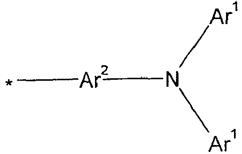 | 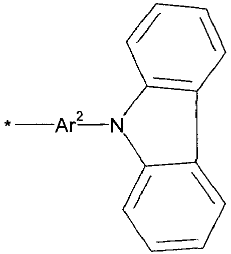 |
| Formel (B-1) | Formel (B-2) |
| 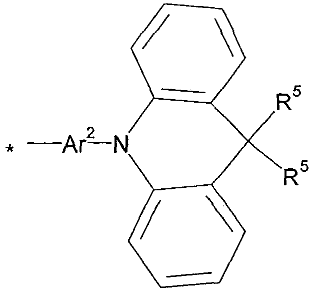 | 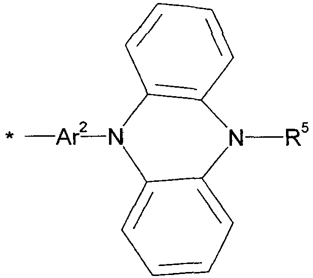 |
| Formel (B-3) | Formel (B-4) |
| | |
| 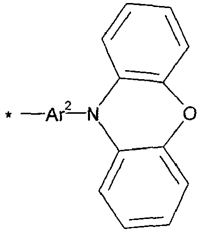 | 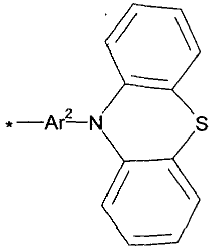 |
| Formel (B-5) | Formel (B-6) |
| | |
| 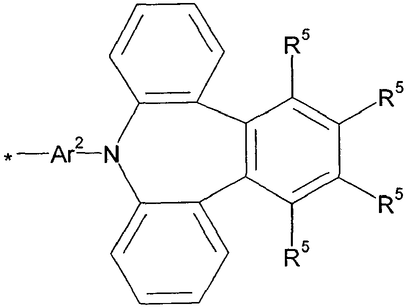 | 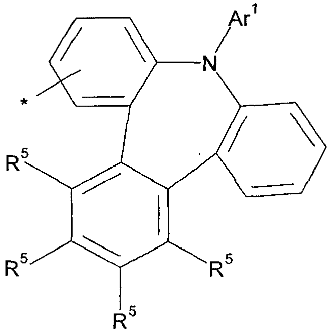 |
| Formel (B-7) | Formel (B-8) |
| 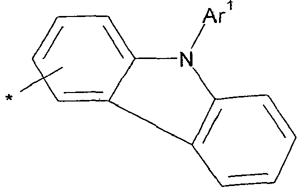 | 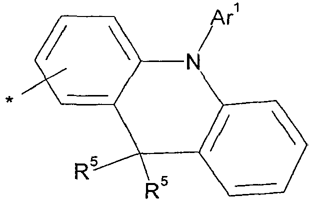 |
| Formel (B-9) | Formel (B-10) |
| | |
| 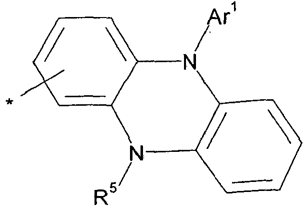 | 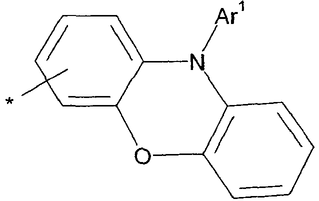 |
| Formel (B-11) | Formel (B-12) |
| | |
| 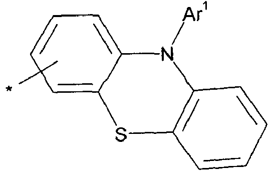 | 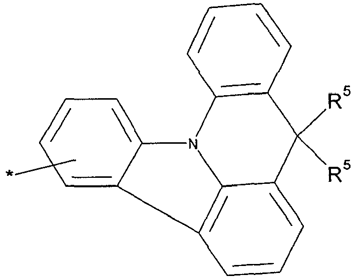 |
| Formel (B-13) | Formel (B-14) |
| | |
| 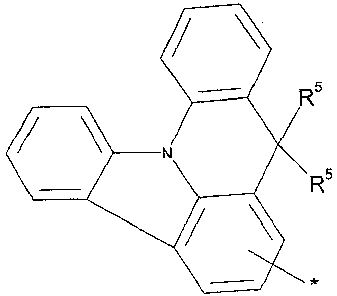 | |
| Formel (B-15) | |

welche an den freien Positionen jeweils mit Resten R⁴ substituiert sein können, und wobei die mit * markierte Bindung die Anbindungsposition an den Phenanthrenyl-Grundkörper darstellt.

Besonders bevorzugt ist darunter die Formel (B-1).

Es ist bevorzugt, dass Ar¹ bei jedem Auftreten gleich oder verschieden gewählt ist aus aromatischen Ringsystemen mit 6 bis 24 aromatischen Ringatomen, die mit einem oder mehreren Resten R⁴ substituiert sein können, und heteroaromatischen Ringsystemen mit 5 bis 24 aromatischen Ringatomen, die mit einem oder mehreren Resten R⁴ substituiert sein können. Besonders bevorzugt ist Ar¹ bei jedem Auftreten gleich oder verschieden gewählt aus aromatischen Ringsystemen mit 12 bis 24 aromatischen Ringatomen, die mit einem oder mehreren Resten R⁴ substituiert sein können.

Bevorzugt sind zwei Gruppen Ar¹ in Formel (A) bzw. Formel (B), die an dasselbe Stickstoffatom gebunden sind, nicht gleich gewählt.

Bevorzugt enthalten die Gruppen Ar¹ jeweils mindestens eine Gruppe gewählt aus Benzol, Naphthalin, Phenanthren, Fluoranthen, Biphenyl, Terphenyl, Quaterphenyl, Fluoren, Spirobifluoren, Furan, Benzofuran, Isobenzofuran, Dibenzofuran, Thiophen, Benzothiophen, Isobenzothiophen, Dibenzothiophen, Indol, Isoindol, Carbazol, Indolocarbazol, Indenocarbazol, Pyridin, Chinolin, Isochinolin, Acridin, Phenanthridin, Benzimidazol, Pyrimidin, Pyrazin, und Triazin, wobei die genannten Gruppen jeweils mit einem oder mehreren Resten R⁴ substituiert sein können.

Bevorzugt ist Ar¹ gewählt aus Gruppen der folgenden Formeln (Ar¹-1) bis (Ar¹-59) 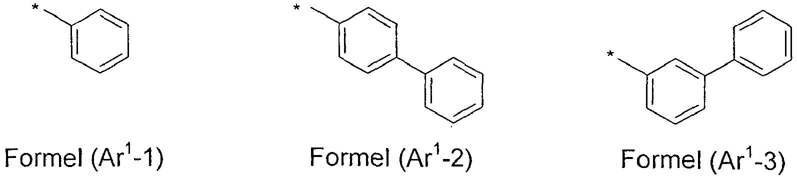 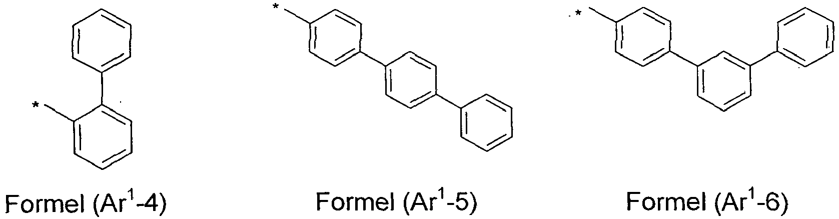 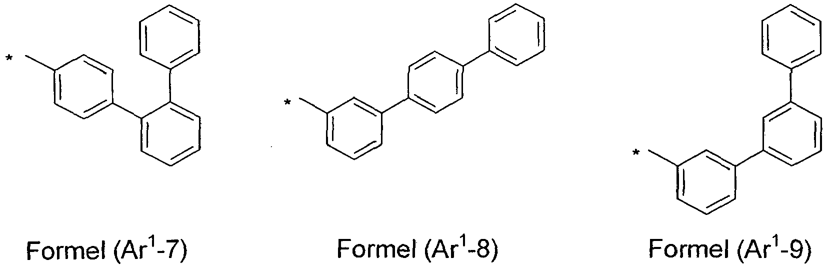 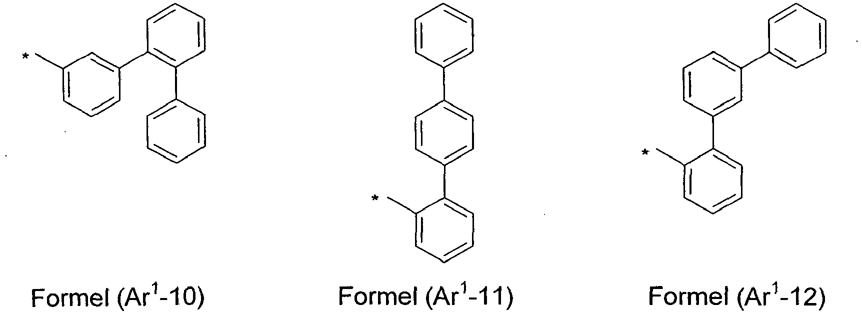 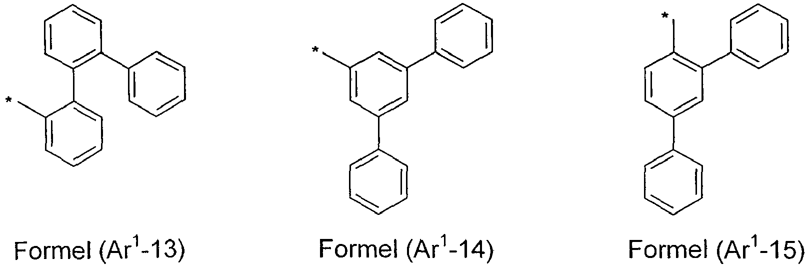 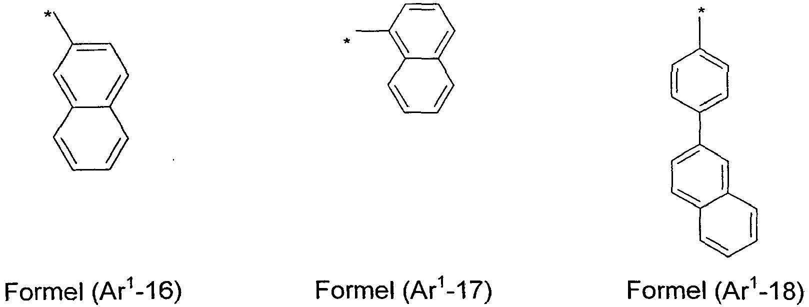 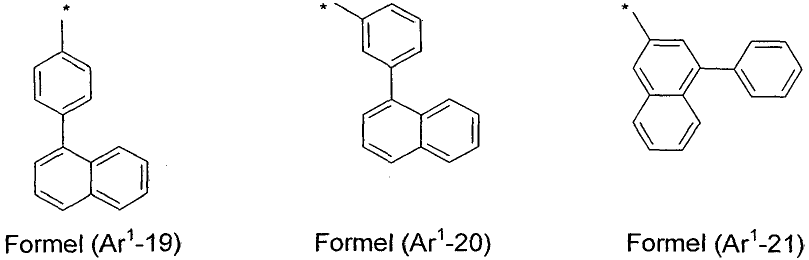 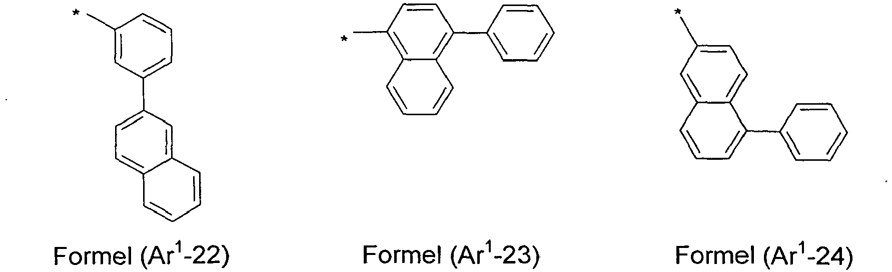 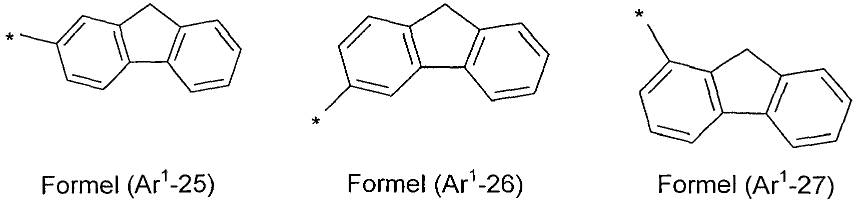 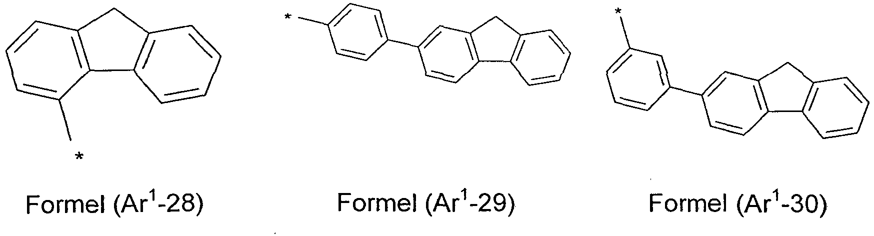 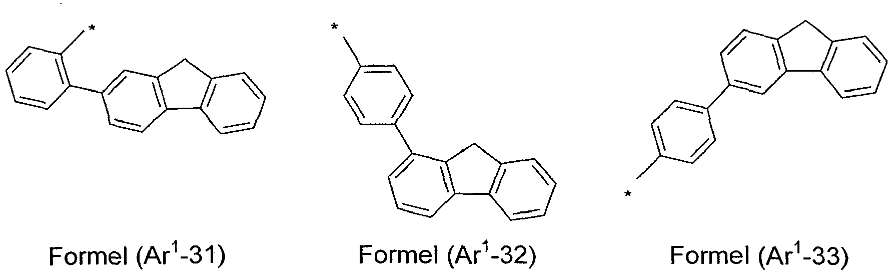 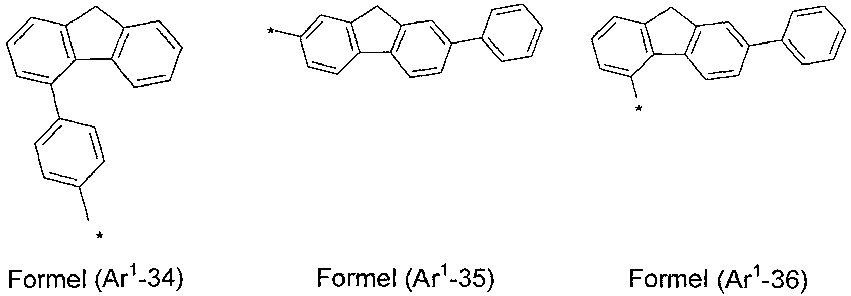 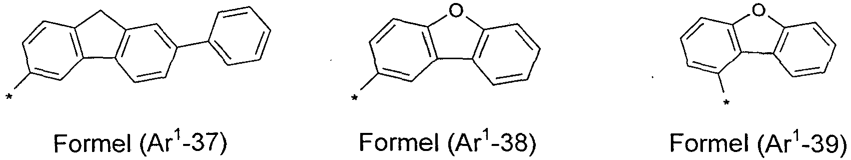 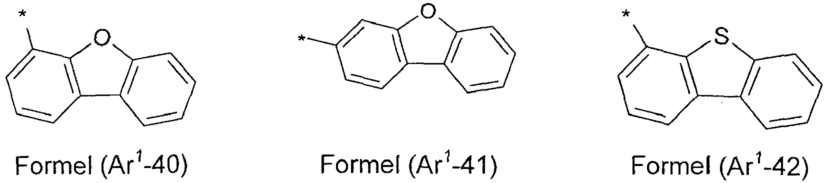 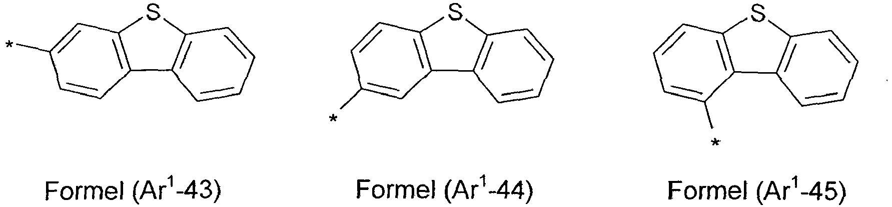 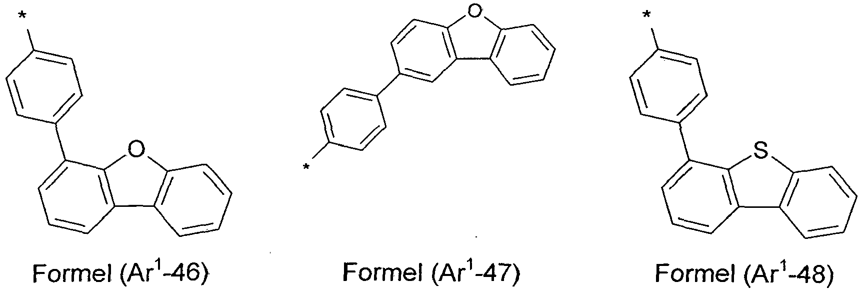 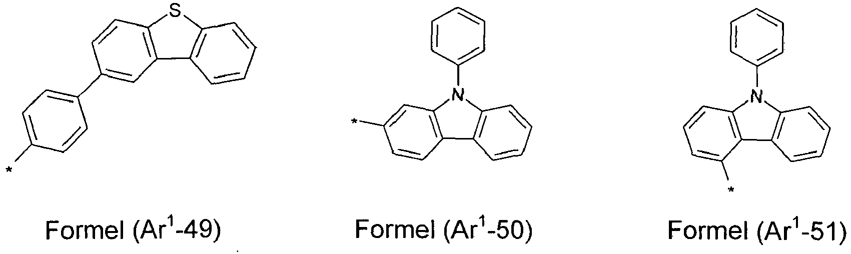 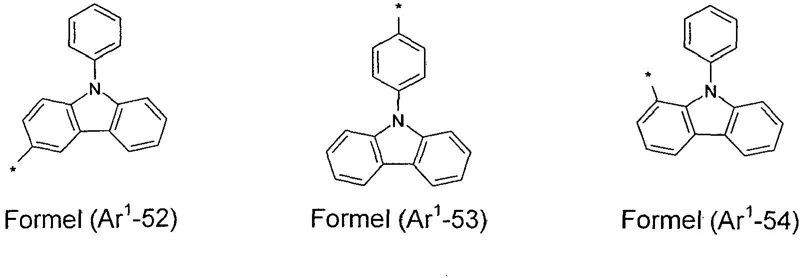 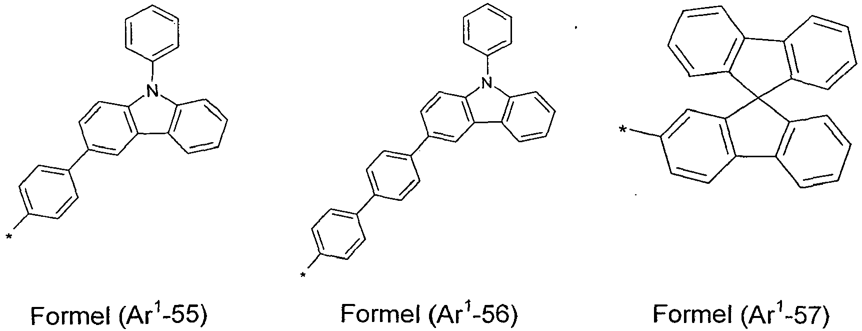 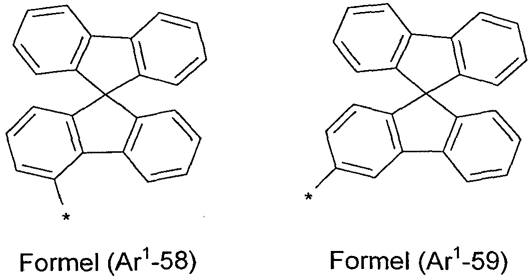 wobei die mit * markierte Bindung jeweils die Bindung zum Stickstoffatom darstellt, und wobei die Gruppen an ihren freien Positionen Reste R⁴ tragen können.

Es ist bevorzugt, dass Ar² bei jedem Auftreten gleich oder verschieden gewählt ist aus aromatischen Ringsystemen mit 6 bis 24 aromatischen Ringatomen, die mit einem oder mehreren Resten R⁴ substituiert sein können, und heteroaromatischen Ringsystemen mit 5 bis 24 aromatischen Ringatomen, die mit einem oder mehreren Resten R⁴ substituiert sein können. Besonders bevorzugt ist Ar² bei jedem Auftreten gleich oder verschieden gewählt aus aromatischen Ringsystemen mit 6 bis 13 aromatischen Ringatomen, die mit einem oder mehreren Resten R⁴ substituiert sein können.

Bevorzugt ist Ar² gewählt aus Gruppen der folgenden Formeln (Ar²-1) bis (Ar²-14)

| | |
|---|---|
| 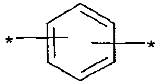 | 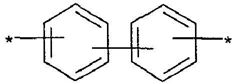 |
| Formel (Ar²-1) | Formel (Ar²-2) |
| | |
| 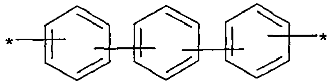 | 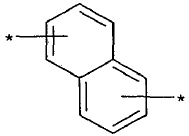 |
| Formel (Ar²-3) | Formel (Ar²-4) |
| | |
| 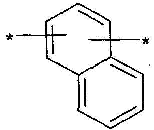 | 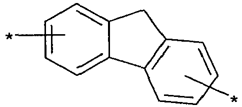 |
| Formel (Ar²-5) | Formel (Ar²-6) |
| | |
| 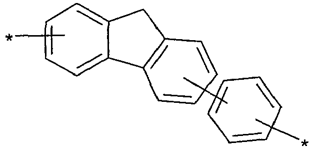 | 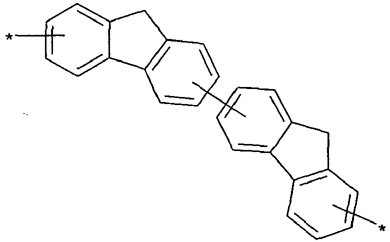 |
| Formel (Ar²-7) | Formel (Ar²-8) |
| | |
| 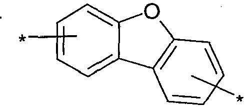 | 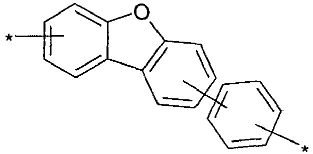 |
| Formel (Ar²-9) | Formel (Ar²-10) |

| | |
|---|---|
| 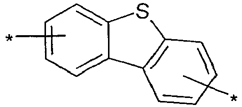 | 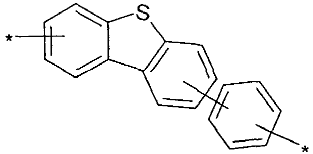 |
| Formel (Ar²-11) | Formel (Ar²-12) |
| | |
| 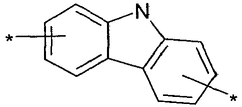 | 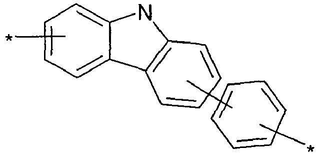 |
| Formel (Ar²-13) | Formel (Ar²-14) |

wobei die Gruppen an den freien Positionen jeweils mit Resten R⁴ substituiert sein können und wobei die mit * markierten Bindungen jeweils die Bindungen an den Rest der Verbindung kennzeichnen.

Gruppen X sind bevorzugt gewählt aus Einfachbindungen, C(R⁵)₂, -C(R⁵)₂-C(R⁵)₂-, -C(^{R}5)=C(^{R}5)-, Si(R⁵)₂, C=O, NR⁵, O, S, S=O, SO₂ und wahlweise mit Resten R⁵ substituiertem ortho-Phenylen.

R¹ ist bevorzugt bei jedem Auftreten gleich oder verschieden gewählt aus H, D, F, CN, Si(R⁶)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 24 aromatischen Ringatomen, die mit einem oder mehreren Resten R⁶ substituiert sein können, heteroaromatischen Ringsystemen mit 5 bis 24 aromatischen Ringatomen, die mit einem oder mehreren Resten R⁶ substituiert sein können, Gruppen der Formel (A), wie oben definiert, und Gruppen der Formel (B),
wobei die genannten Alkyl- und Alkoxygruppen jeweils mit einem oder mehreren Resten R⁶ substituiert sein können und wobei in den genannten Alkyl- und Alkoxygruppen eine oder mehrere CH₂-Gruppen durch -C=C-, -R⁶C=CR⁶-, Si(R⁶)₂, C=O, C=NR⁶, -NR⁶-, -O-, -S-, -C(=O)O- oder -C(=O)NR⁶- ersetzt sein können.

R², R³ sind bevorzugt bei jedem Auftreten gleich oder verschieden gewählt aus H, D, F, CN, Si(R⁶)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 24 aromatischen Ringatomen, die mit einem oder mehreren Resten R⁶ substituiert sein können, und heteroaromatischen Ringsystemen mit 5 bis 24 aromatischen Ringatomen, die mit einem oder mehreren Resten R⁶ substituiert sein können,
wobei die genannten Alkyl- und Alkoxygruppen jeweils mit einem oder mehreren Resten R⁶ substituiert sein können und wobei in den genannten Alkyl- und Alkoxygruppen eine oder mehrere CH₂-Gruppen durch -C≡C-, -R⁶C=CR⁶-, Si(R⁶)₂, C=O, C=NR⁶, -NR⁶-, -O-, -S-, -C(=O)O- oder -C(=O)NR⁶- ersetzt sein können.

R⁴ und R⁵ sind bevorzugt bei jedem Auftreten gleich oder verschieden gewählt aus H, D, F, CN, Si(R⁶)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 24 aromatischen Ringatomen, die mit einem oder mehreren Resten R⁶ substituiert sein können, und heteroaromatischen Ringsystemen mit 5 bis 24 aromatischen Ringatomen, die mit einem oder mehreren Resten R⁶ substituiert sein können,
wobei die genannten Alkyl- und Alkoxygruppen jeweils mit einem oder mehreren Resten R⁶ substituiert sein können und wobei in den genannten Alkyl- und Alkoxygruppen eine oder mehrere CH₂-Gruppen durch -C≡C-, -R⁶C=CR⁶-, Si(R⁶)₂, C=O, C=NR⁶, -NR⁶-, -O-, -S-, -C(=O)O- oder -C(=O)NR⁶- ersetzt sein können.

Gruppen R⁵, die Bestandteile einer Gruppe C(R⁵)₂, -C(R⁵)₂-C(R⁵)₂-, -C(R⁵)=C(R⁵)-, Si(R⁵)₂, und NR⁵ sind, sind bevorzugt gewählt aus geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 24 aromatischen Ringatomen, die mit einem oder mehreren Resten R⁶ substituiert sein können, und heteroaromatischen Ringsystemen mit 5 bis 24 aromatischen Ringatomen, die mit einem oder mehreren Resten R⁶ substituiert sein können.

R⁶ ist bevorzugt bei jedem Auftreten gleich oder verschieden gewählt aus H, D, F, CN, Si(R⁷)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 24 aromatischen Ringatomen, die mit einem oder mehreren Resten R⁷ substituiert sein können, und heteroaromatischen Ringsystemen mit 5 bis 24 aromatischen Ringatomen, die mit einem oder mehreren Resten R⁷ substituiert sein können,
wobei die genannten Alkyl- und Alkoxygruppen jeweils mit einem oder mehreren Resten R⁷ substituiert sein können und wobei in den genannten Alkyl- und Alkoxygruppen eine oder mehrere CH₂-Gruppen durch -C≡C-, -R⁷C=CR⁷-, Si(R⁷)₂, C=O, C=NR⁷, -NR⁷-, -O-, -S-, -C(=O)O- oder -C(=O)NR⁷- ersetzt sein können.

Bevorzugte Ausführungsformen der Verbindung der Formel (I) entsprechen den folgenden Formeln (I-1) bis (I-24)

| | |
|---|---|
| 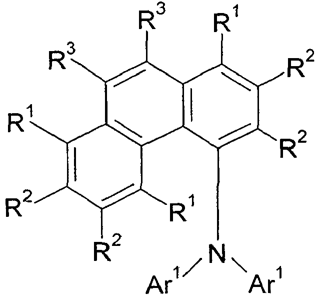 | 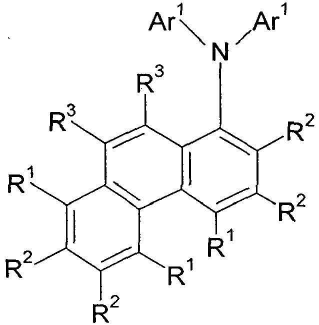 |
| Formel (I-1) | Formel (I-2) |
| 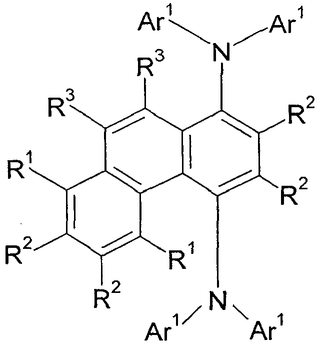 | 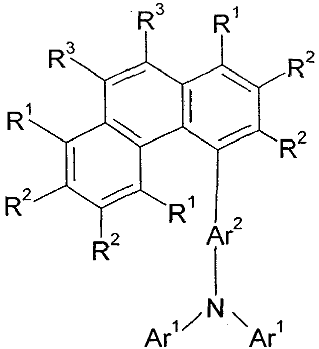 |
| Formel (I-3) | Formel (I-4) |
| | |
| 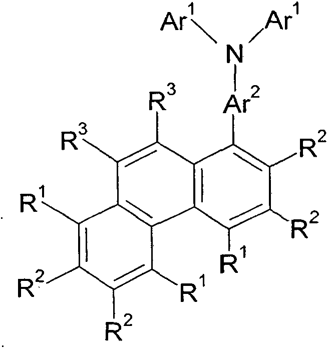 | 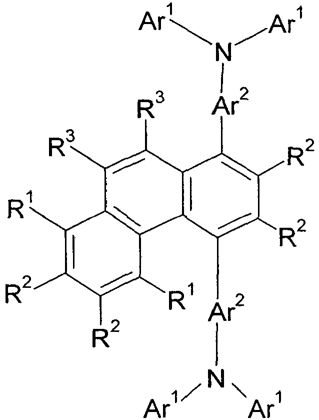 |
| Formel (I-5) | Formel (I-6) |
| 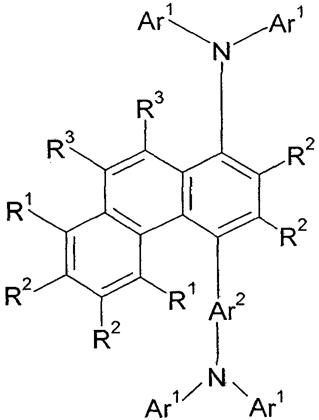 | 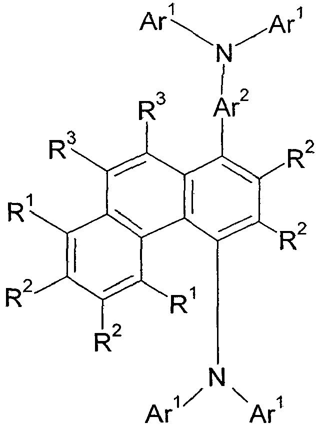 |
| Formel (I-7) | Formel (I-8) |
| | |
| 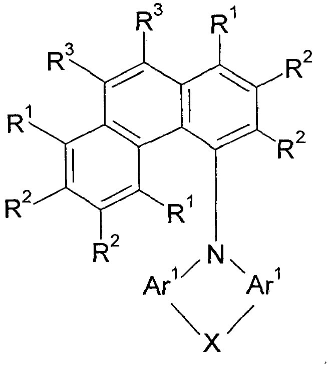 | 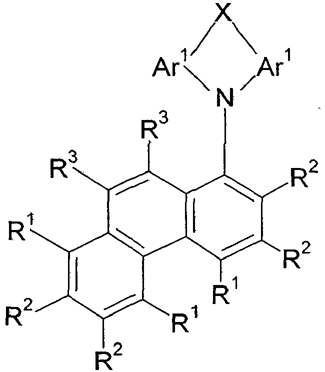 |
| Formel (I-9) | Formel (I-10) |
| 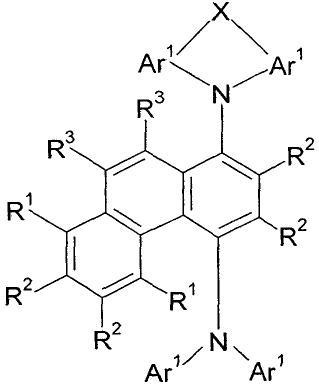 | 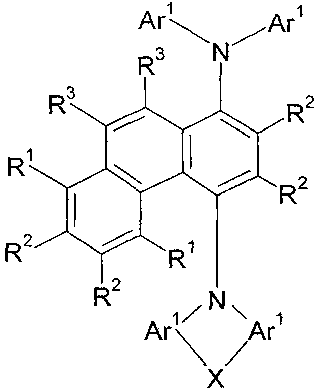 |
| Formel (I-11) | Formel (I-12) |
| | |
| 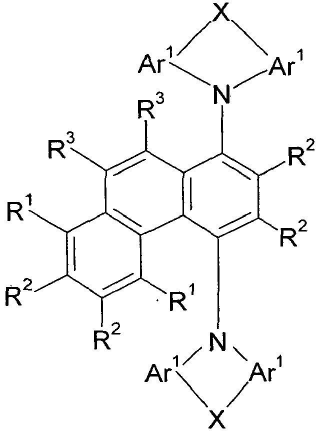 | 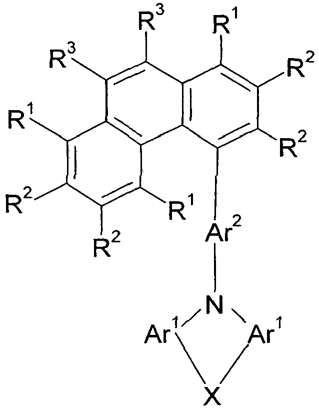 |
| Formel (I-13) | Formel (I-14) |
| 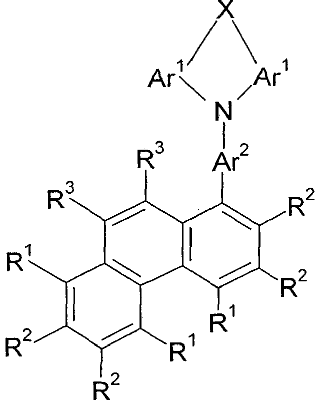 | 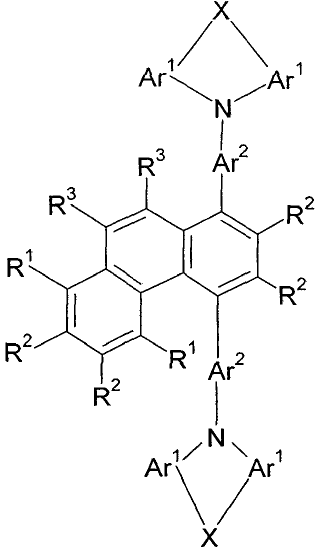 |
| Formel (I-15) | Formel (I-16) |
| | |
| 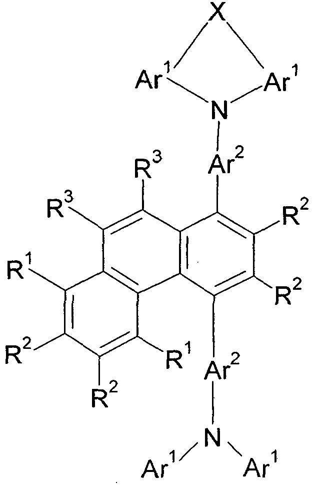 | 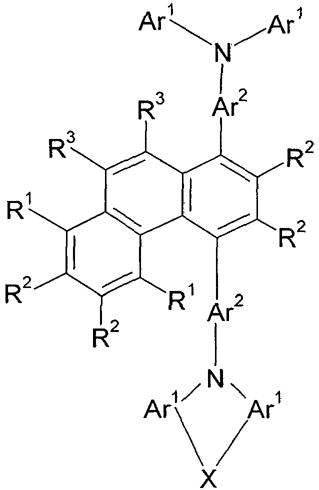 |
| Formel (I-17) | Formel (I-18) |
| 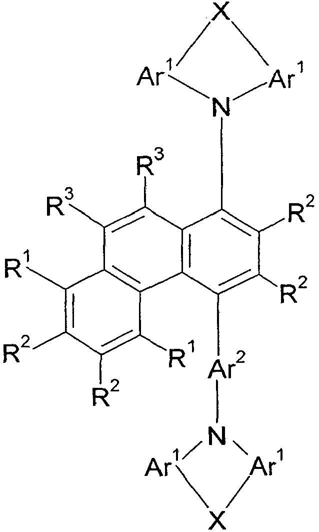 | 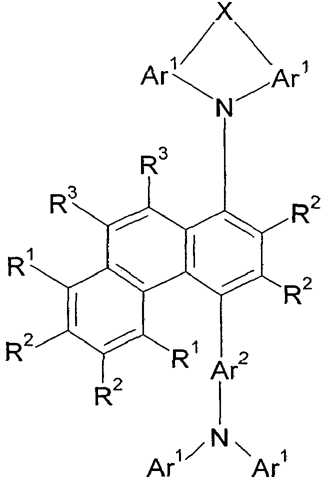 |
| Formel (I-19) | Formel (I-20) |
| | |
| 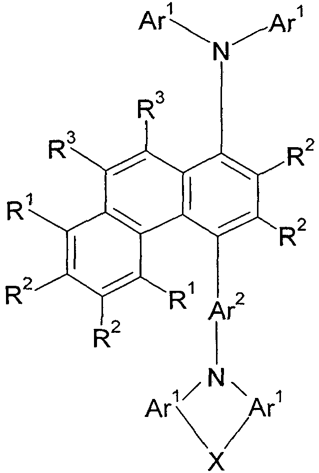 | 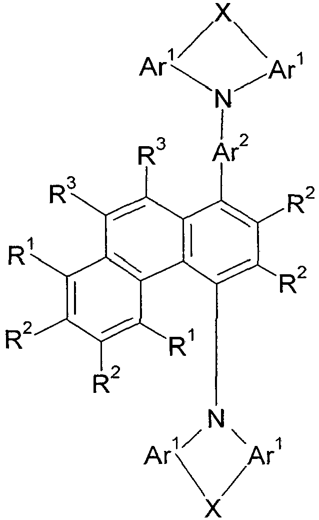 |
| Formel (I-21) | Formel (I-22) |
| 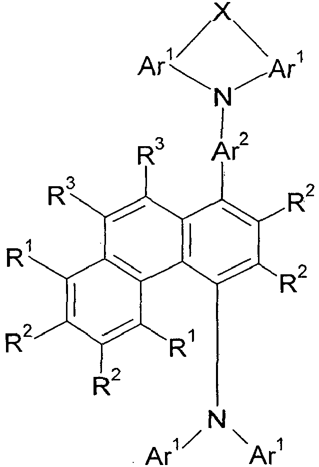 | 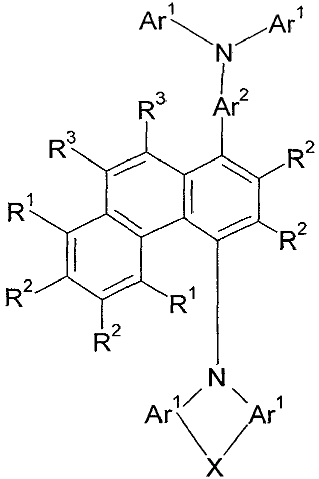 |
| Formel (I-23) | Formel (I-24) |

wobei die auftretenden Gruppen definiert sind wie oben, und R¹ bei jedem Auftreten gleich oder verschieden gewählt ist aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl-oder Alkinylgruppen mit 2 bis 20 C-Atomen, aromatischen Ringsystemen mit 6 bis 40 aromatischen Ringatomen, die mit einem oder mehreren Resten R⁶ substituiert sein können, und heteroaromatischen Ringsystemen mit 5 bis 40 aromatischen Ringatomen, die mit einem oder mehreren Resten R⁶ substituiert sein können,
wobei die genannten Alkyl-, Alkoxy-, Alkenyl- und Alkinylgruppen jeweils mit einem oder mehreren Resten R⁶ substituiert sein können und wobei eine oder mehrere CH₂-Gruppen in den genannten Alkyl-, Alkoxy-, Alkenyl- und Alkinylgruppen durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können.

Bevorzugt sind unter den Formeln (I-1) bis (I-24) die Formeln (I-1), (I-2), (I-9) und (I-10), besonders bevorzugt sind die Formeln (I-1) und (I-2).

Die Kombination der bevorzugten Formeln (I-1) bis (I-24) mit den bevorzugten Ausführungsformen der Gruppen Ar¹, Ar², X, R¹ bis R⁶ ist besonders bevorzugt.

Für die Formeln (I-1) bis (I-24) ist es besonders bevorzugt, dass die Gruppen R¹, R² und R³ bei jedem Auftreten gleich oder verschieden gewählt sind aus H, D, F, CN, Si(R⁶)₃, geradkettigen Alkyl- oder Alkoxygruppen mit 1 bis 20 C-Atomen, verzweigten oder cyclischen Alkyl-oder Alkoxygruppen mit 3 bis 20 C-Atomen, aromatischen Ringsystemen mit 6 bis 24 aromatischen Ringatomen, die mit einem oder mehreren Resten R⁶ substituiert sein können, und heteroaromatischen Ringsystemen mit 5 bis 24 aromatischen Ringatomen, die mit einem oder mehreren Resten R⁶ substituiert sein können,
wobei die genannten Alkyl- und Alkoxygruppen jeweils mit einem oder mehreren Resten R⁶ substituiert sein können und wobei in den genannten Alkyl- oder Alkoxygruppen eine oder mehrere CH₂-Gruppen durch -C=C-, - R⁶C=CR⁶-, Si(R⁶)₂, C=O, C=NR⁶, -NR⁶-, -O-, -S-, -C(=O)O- oder - C(=O)NR⁶- ersetzt sein können. Ganz besonders bevorzugt ist es, dass die Gruppen R¹, R² und R³ bei jedem Auftreten gleich oder verschieden gewählt sind aus H, F, CN, Si(R⁶)₃, geradkettigen Alkyl- oder Alkoxygruppen mit 1 bis 20 C-Atomen, verzweigten oder cyclischen Alkyl-oder Alkoxygruppen mit 3 bis 20 C-Atomen, aromatischen Ringsystemen mit 6 bis 24 aromatischen Ringatomen, die mit einem oder mehreren Resten R⁶ substituiert sein können, und heteroaromatischen Ringsystemen mit 5 bis 24 aromatischen Ringatomen, die mit einem oder mehreren Resten R⁶ substituiert sein können.

Beispiele für Verbindungen gemäß Formel (I) sind im Folgenden abgebildet:

| | | |
|---|---|---|
| 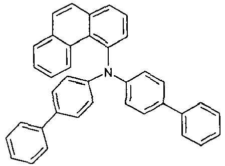 | 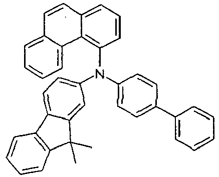 | 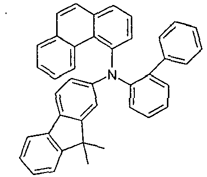 |
| 1 | 2 | 3 |
| 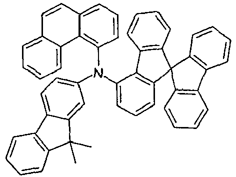 | 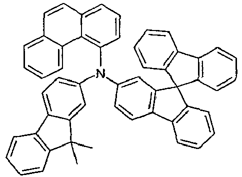 | 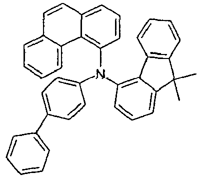 |
| 4 | 5 | 6 |
| 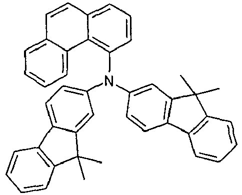 | 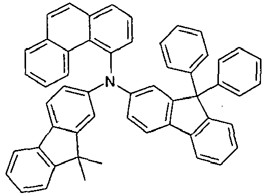 | 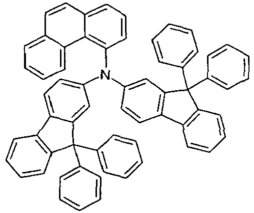 |
| 7 | 8 | 9 |
| 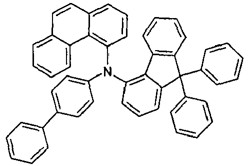 | 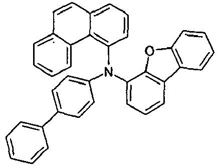 | 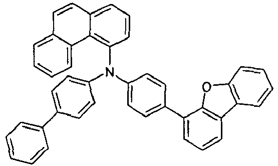 |
| 10 | 11 | 12 |
| 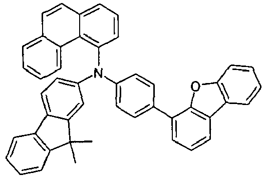 | 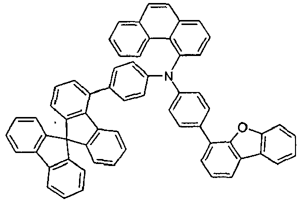 | 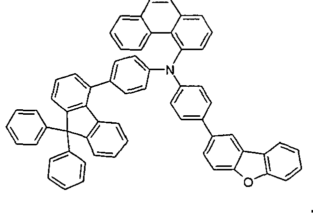 |
| 13 | 14 | 15 |
| 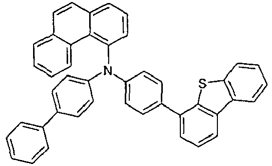 | 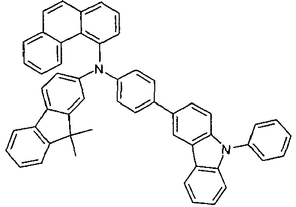 | 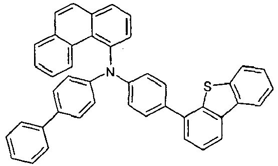 |
| 16 | 17 | 18 |
| 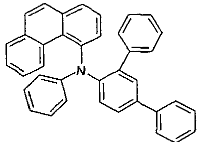 | 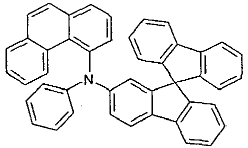 | 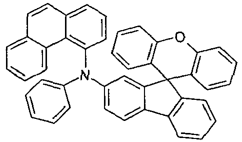 |
| 19 | 20 | 21 |
| 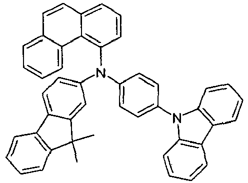 | 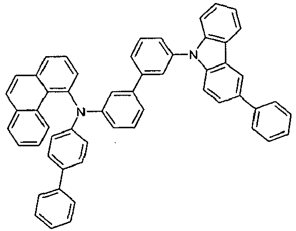 | 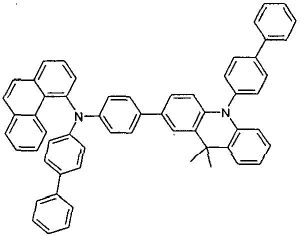 |
| 22 | 23 | 24 |
| 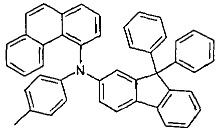 | 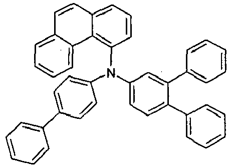 | 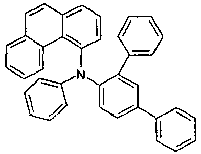 |
| 25 | 26 | 27 |
| 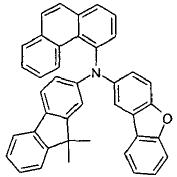 | 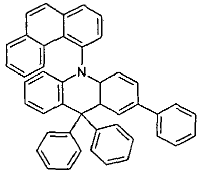 | 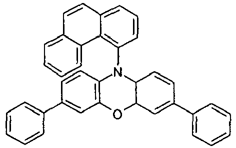 |
| 28 | 29 | 30 |
| 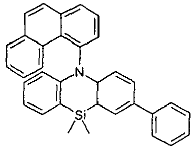 | 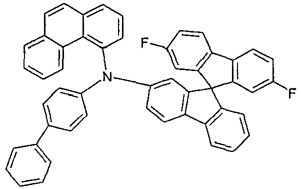 | 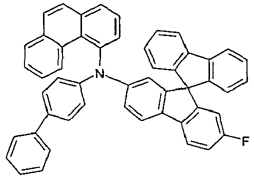 |
| 31 | 32 | 33 |
| 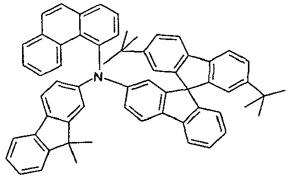 | 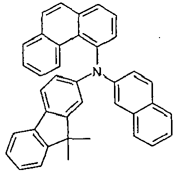 | 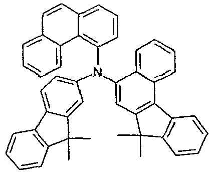 |
| 34 | 35 | 36 |
| 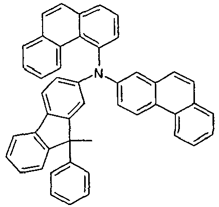 | 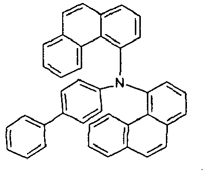 | 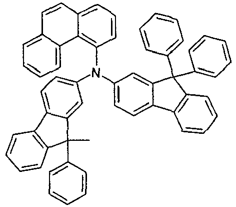 |
| 37 | 38 | 39 |
| 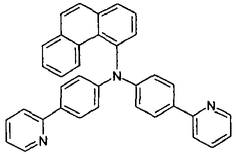 | 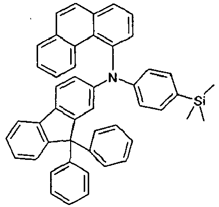 | 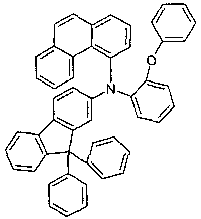 |
| 40 | 41 | 42 |
| 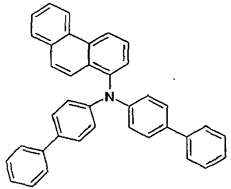 | 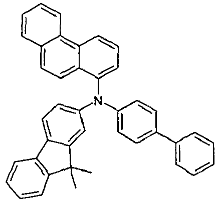 | 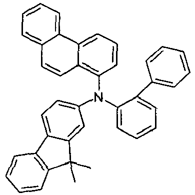 |
| 43 | 44 | 45 |
| 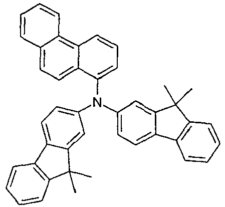 | 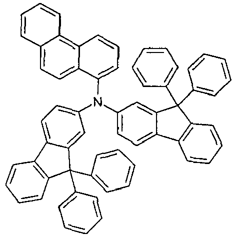 | 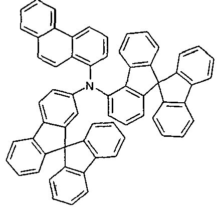 |
| 46 | 47 | 48 |
| 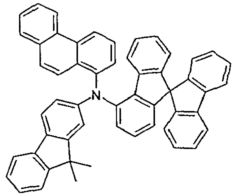 | 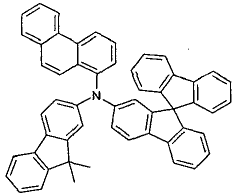 | 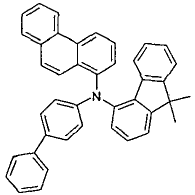 |
| 49 | 50 | 51 |
| 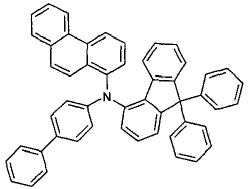 | 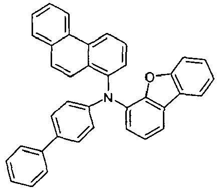 | 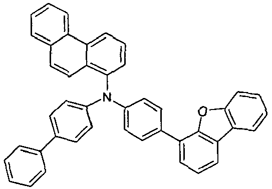 |
| 52 | 53 | 54 |
| 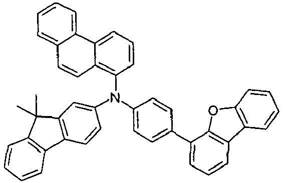 | 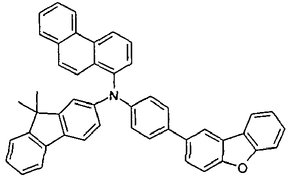 | 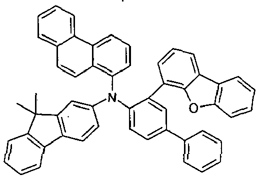 |
| 55 | 56 | 57 |
| 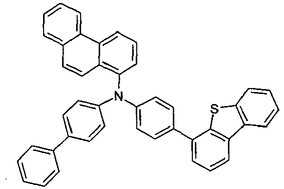 | 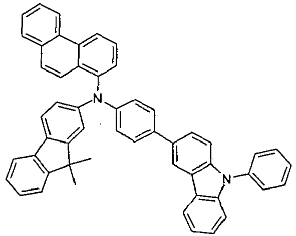 | 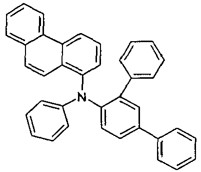 |
| 58 | 59 | 60 |
| 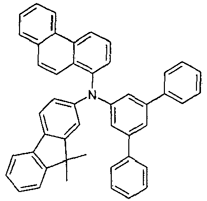 | 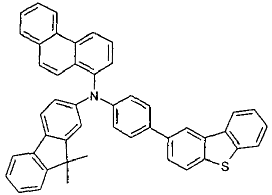 | 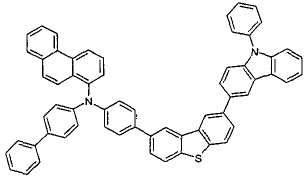 |
| 61 | 62 | 63 |
| 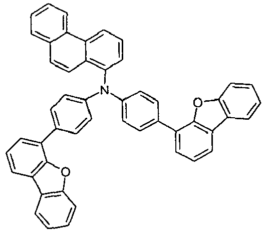 | 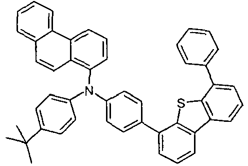 | 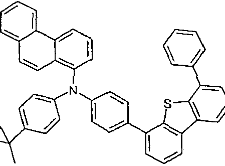 |
| 64 | 65 | 66 |
| 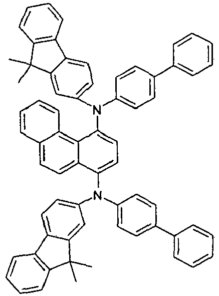 | 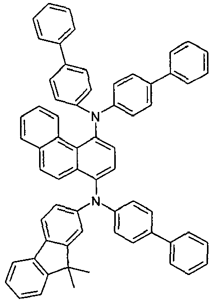 | 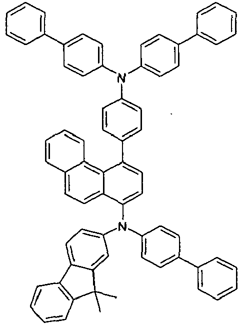 |
| 67 | 68 | 69 |
| 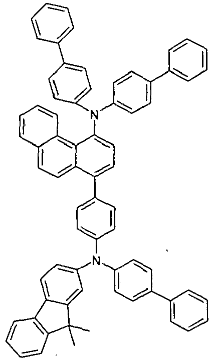 | 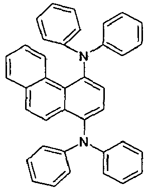 | 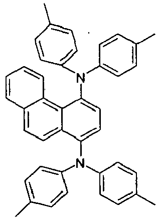 |
| 70 | 71 | 72 |
| 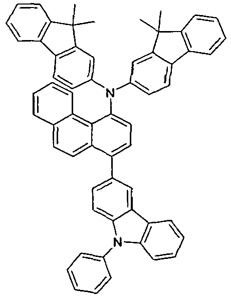 | 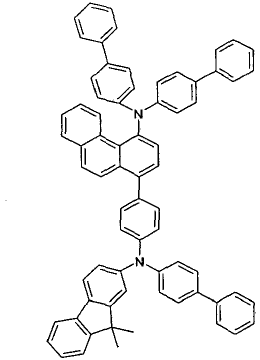 | 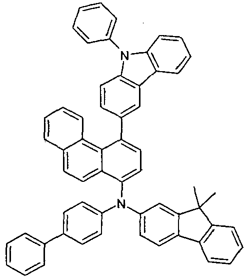 |
| 73 | 74 | 75 |
| 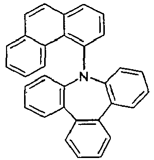 | 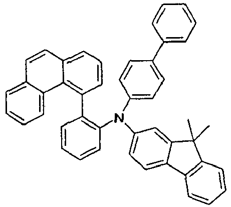 | 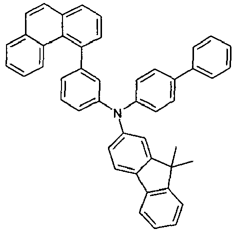 |
| 76 | 77 | 78 |
| 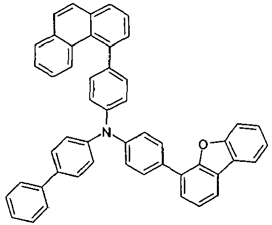 | 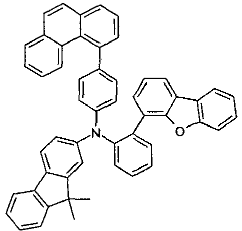 | 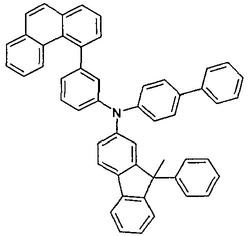 |
| 79 | 80 | 81 |
| 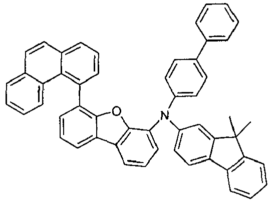 | 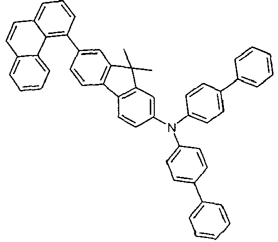 | 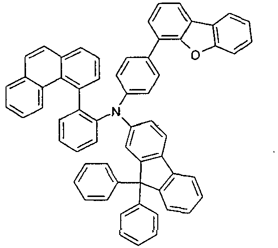 |
| 82 | 83 | 84 |

Die Synthese der erfindungsgemäßen Verbindungen kann gemäß im Stand der Technik bekannten Verfahren und Reaktionstypen, beispielsweise Halogenierung, Buchwald-Kupplung, Ullmann-Kupplung und Suzuki-Kupptung, erfolgen.

Schema 1 zeigt einen bevorzugten Syntheseweg zur Herstellung der erfindungsgemäßen Verbindungen. Dazu wird die PhenanthrenVerbindung A in einer Buchwald-Kupplung mit einem Amin B der Formel Ar-NH-Ar umgesetzt, oder sie wird in einer Suzuki-Kupplung mit einem Boronsäure Derivat C der Formel Ar₂N-Ar-B(OR)₃ umgesetzt.

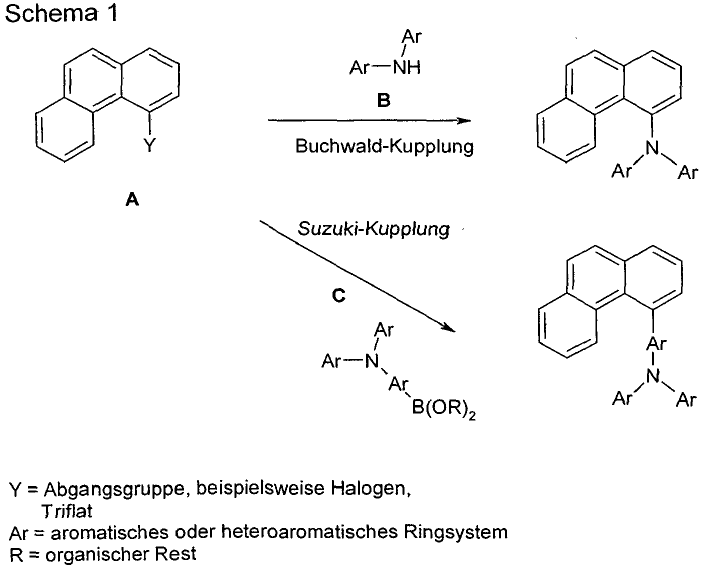

Auf analogem Weg (Schema 2) können erfindungsgemäße Verbindungen hergestellt werden, die in 1-Position am Phenanthren mit einer Arylaminogruppe substituiert sind oder die in dieser Position mit einer Arylgruppe, die eine Arylaminogruppe trägt, substituiert sind. Hierzu wird von einem in 1-Position mit einer Abgangsgruppe substituierten Phenanthren-Derivat D ausgegangen.

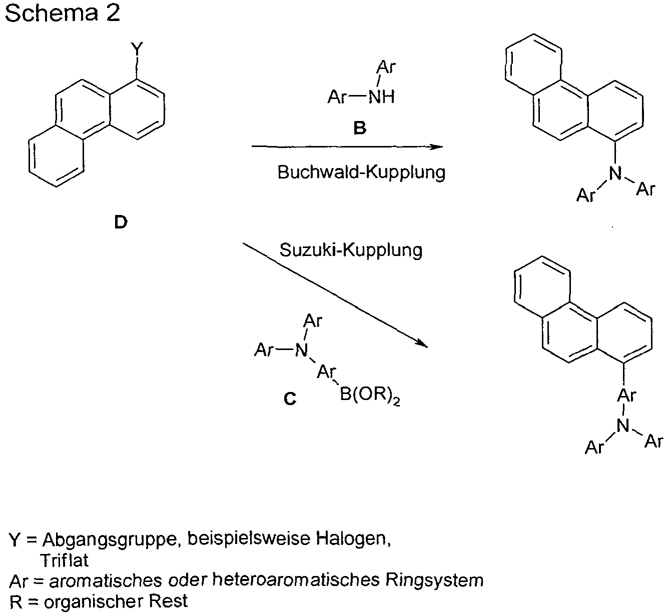

Synthesewege für die Ausgangsverbindungen A und D, welche in der Synthese der erfindungsgemäßen Verbindungen eingesetzt werden, sind dem Fachmann bekannt. Weiterhin werden in den Ausführungsbeispielen einige explizite Syntheseverfahren im Detail dargestellt.

Wie in den Ausführungsbeispielen gezeigt, können durch Verwendung von zwei Abgangsgruppen, je eine in 1-Position und je eine in 4-Position am Phenanthren, Verbindungen der Formel (I) mit zwei Arylaminogruppen hergestellt werden. Durch Verwendung von zwei unterschiedlichen Abgangsgruppen können, wie ebenfalls in den Ausführungsbeispielen gezeigt, Verbindungen der Formel (I) hergestellt werden, die zwei unterschiedliche Arylaminogruppen in 1- und in 4-Position aufweisen.

Gegenstand der Erfindung ist damit weiterhin ein Verfahren zur Herstellung einer Verbindung der Formel (I), dadurch gekennzeichnet, dass eine in 1-und/oder in 4-Position mit einer Abgangsgruppe substituierte Phenanthrenverbindung in einer Kupplungsreaktion mit einer Diarylaminoverbindung oder mit einer mit einer Abgangsgruppe substituierten Triarylaminoverbindung umgesetzt wird.

Die Abgangsgruppen sind dabei bevorzugt gewählt aus Halogenid, bevorzugt Br oder I, Boronsäuregruppen, Boronsäureestergruppen und Sulfonsäureestergruppen, bevorzugt Trifluorsulfonsäureestergruppen. Boronsäuregruppen und Boronsäureestergruppen sind besonders bevorzugt bei der Reaktion von Phenanthrenverbindung mit Triarylaminoverbindung.

Die Kupplungsreaktion ist bei der Reaktion der Phenanthrenverbindung mit einer Diarylaminoverbindung bevorzugt gewählt aus Buchwald-Kupplungsreaktionen. Die Kupplungsreaktion ist bei der Reaktion der Phenanthrenverbindung mit einer Triarylaminoverbindung bevorzugt gewählt aus Suzuki-Reaktionen.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹ , R² , R³, R⁴, R⁵, R⁶ oder R⁷ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethytether, Tetraethylengtycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichteh, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung enthaltend die Verbindung der Formel (I) ist bevorzugt die folgende: Anode-Lochinjektionsschicht-Lochtransportschicht-wahlweise weitere Lochtransportschicht-wahlweise Elektronenblockierschicht-emittierende Schicht-Elektronentransportschicht-Elektroneninjektionsschicht-Kathode. Es müssen jedoch nicht alle der genannten Schichten vorhanden sein, und es können zusätzlich weitere Schichten vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen sind dabei bevorzugt in der Lochtransportschicht, Lochinjektionsschicht oder der Elektronenblockierschicht vorhanden.

Es ist erfindungsgemäß bevorzugt, wenn die Verbindung gemäß Formel (I) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht oder in einer emittierenden Schicht, enthalten sein.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierende nVerbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen. Weitere Beispiele sind in einer folgenden Tabelle aufgeführt.

Die Verbindung gemäß Formel (I) kann aber auch erfindungsgemäß in einer elektronischen Vorrichtung enthaltend eine oder mehrere fluoreszierende emittierende Verbindungen eingesetzt werden.

In einer bevorzugten Ausführungsform der Erfindung werden die Verbindungen gemäß Formel (I) als Lochtransportmaterial eingesetzt. Die Verbindungen liegen dann bevorzugt in einer Lochtransportschicht, einer Elektronenblockierschicht oder einer Lochinjektionsschicht vor.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin

Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden inbesondere die folgenden Verbindungen:

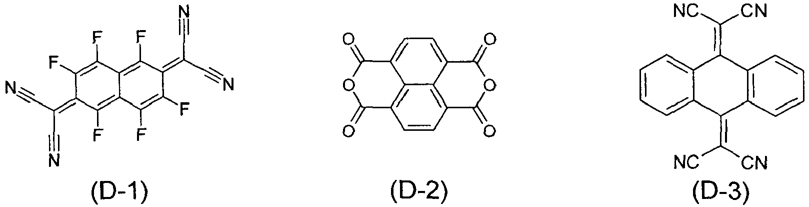

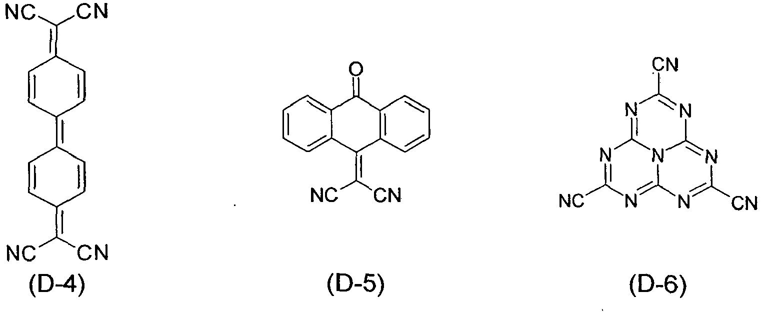

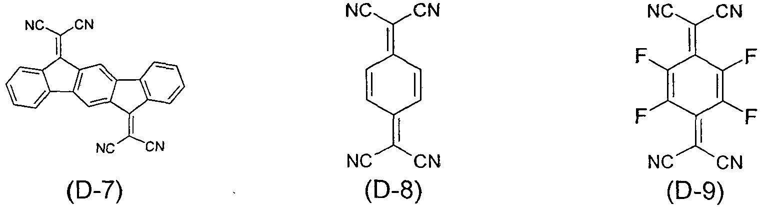

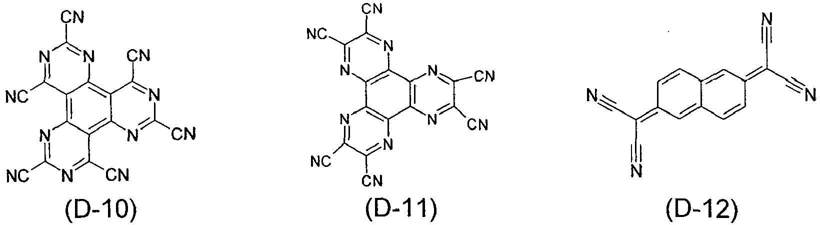

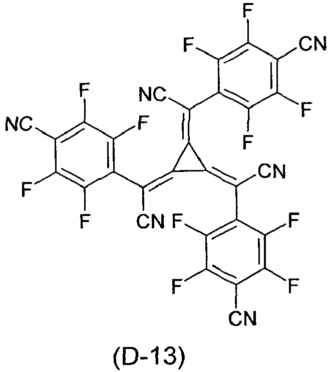

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

In einer weiteren Ausführungsform der vorliegenden Erfindung wird die Verbindung der Formel (I) als Matrixmaterial in Kombination mit einer oder mehreren emittierenden Verbindungen, vorzugsweise phosphoreszierenden emittierenden Verbindungen, eingesetzt.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) stellt dabei bevorzugt das Matrixmaterial mit lochtransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen oder den bevorzugten Matrixmaterialien für fluoreszierende emittierende Verbindungen, je nachdem welche Art von emittierender Verbindung im mixed-Matrix-System eingesetzt wird.

Bevorzugte phosphoreszierende emittierende Verbindungen zur Verwendung in Mixed-Matrix-Systemen sind in einer folgenden Tabelle aufgeführt.

In einer weiteren Ausführungsform der vorliegenden Erfindung wird die Verbindung der Formel (I) als emittierende Verbindung in einer emittierenden Schicht einer OLED eingesetzt. Bevorzugt ist sie in diesem Fall eine fluoreszierende emittierende Verbindung, besonders bevorzugt eine blau fluoreszierende emittierende Verbindung.

Wenn die Verbindung der Formel (I) als emittierende Verbindung in einer emittierenden Schicht einer OLED eingesetzt wird, so wird sie bevorzugt in Kombination mit einem oder mehreren Matrixmaterialien eingesetzt, besonders bevorzugt mit den oben genannten bevorzugten Anteilen von emittierender Verbindung und Matrixmaterial. Bevorzugte Matrixmaterialien sind in diesem Fall die üblicherweise vom Fachmann als Matrixmaterialien für fluoreszierende emittierende Verbindungen eingesetzten Verbindungen. Beispiele für bevorzugte Verbindungsklassen sind in folgenden Abschnitten aufgeführt.

Im Folgenden werden bevorzugte Ausführungsformen für die verschiedenen Funktionsmaterialien der elektronischen Vorrichtung aufgeführt.

Bevorzugte phosphoreszierende emittierende Verbindungen sind die oben genannten Verbindungen und die in der folgenden Tabelle gezeigten Verbindungen:

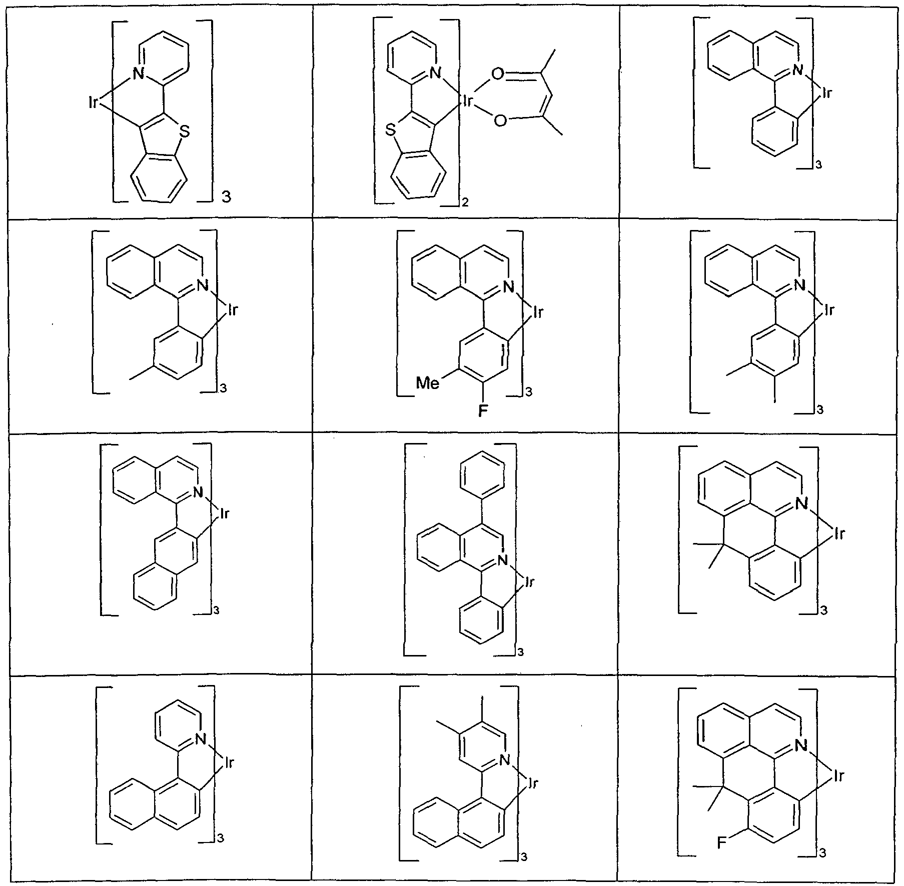

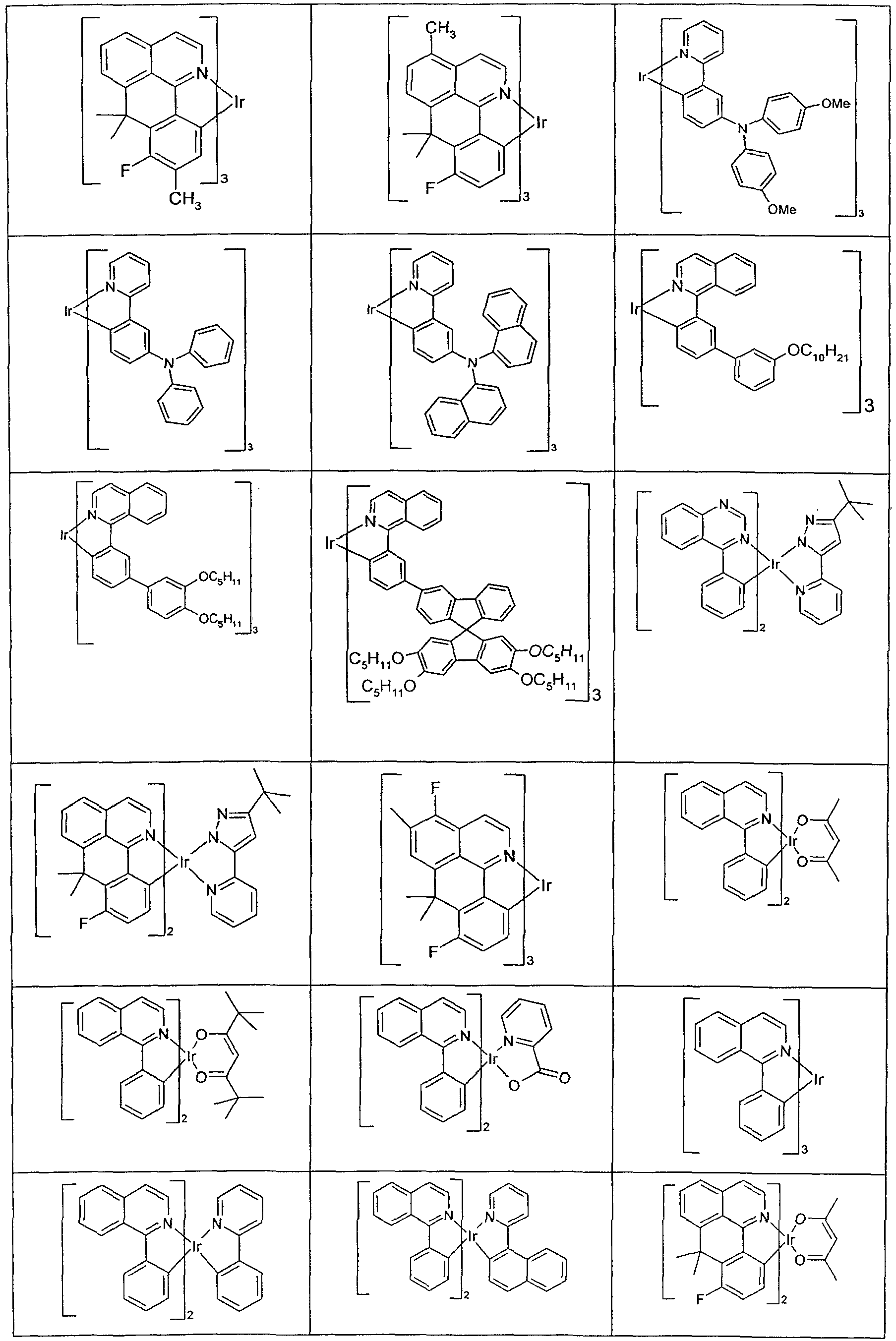

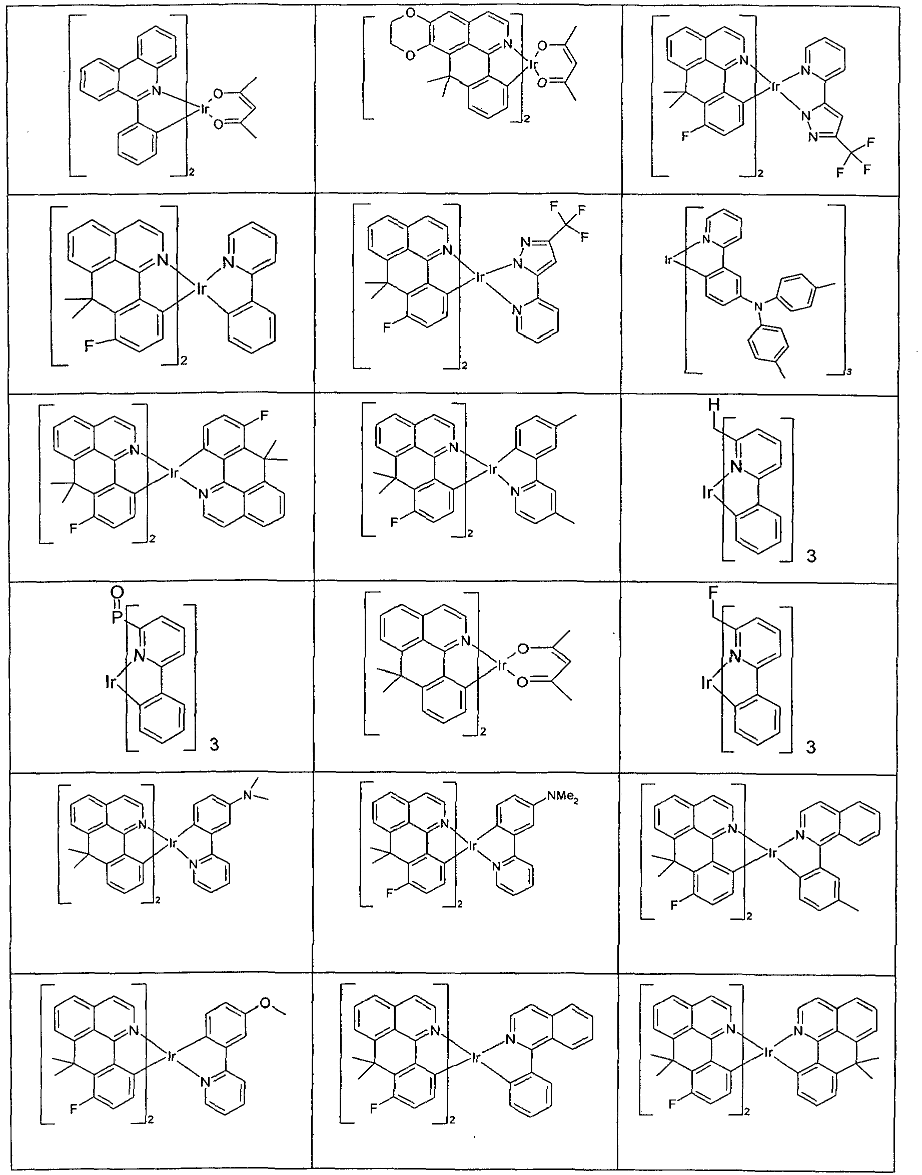

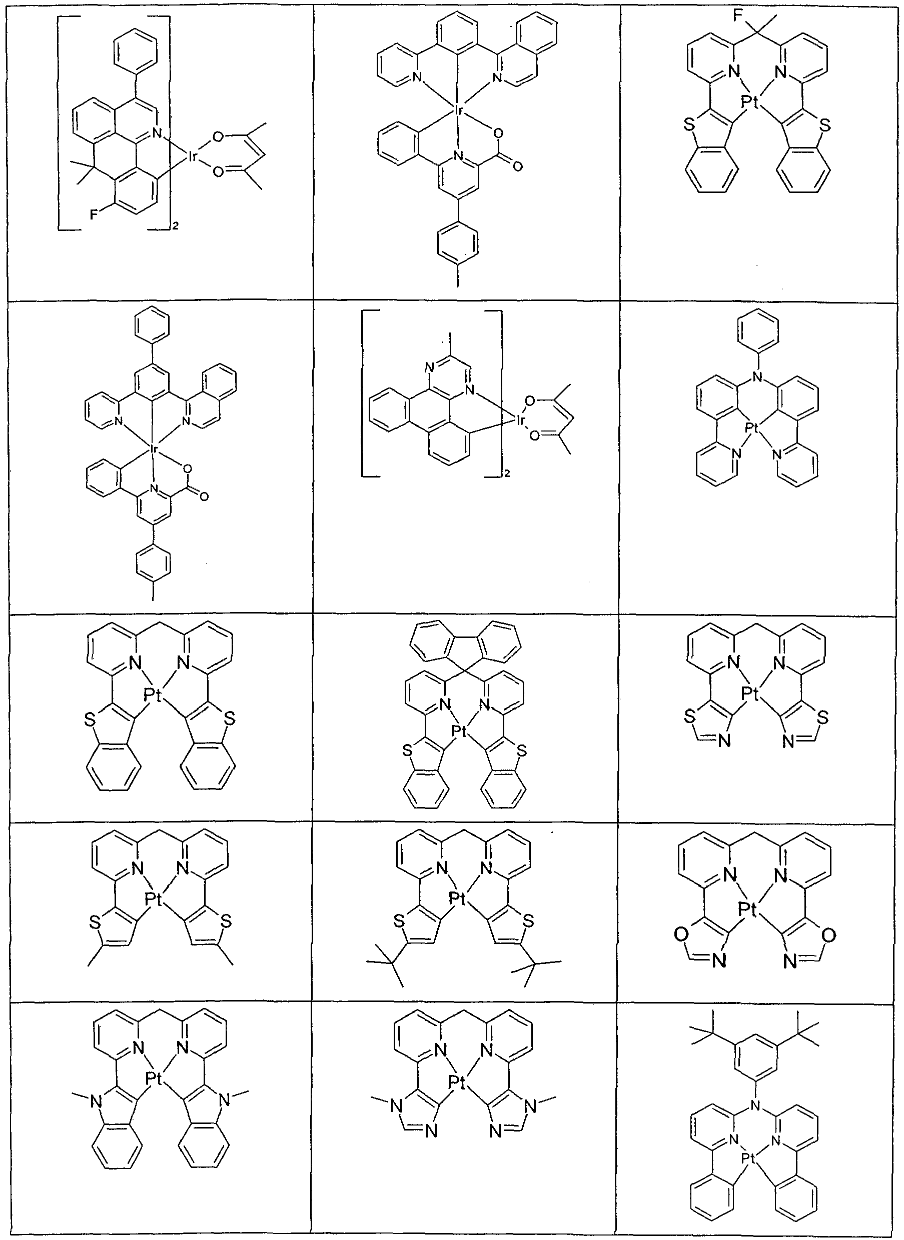

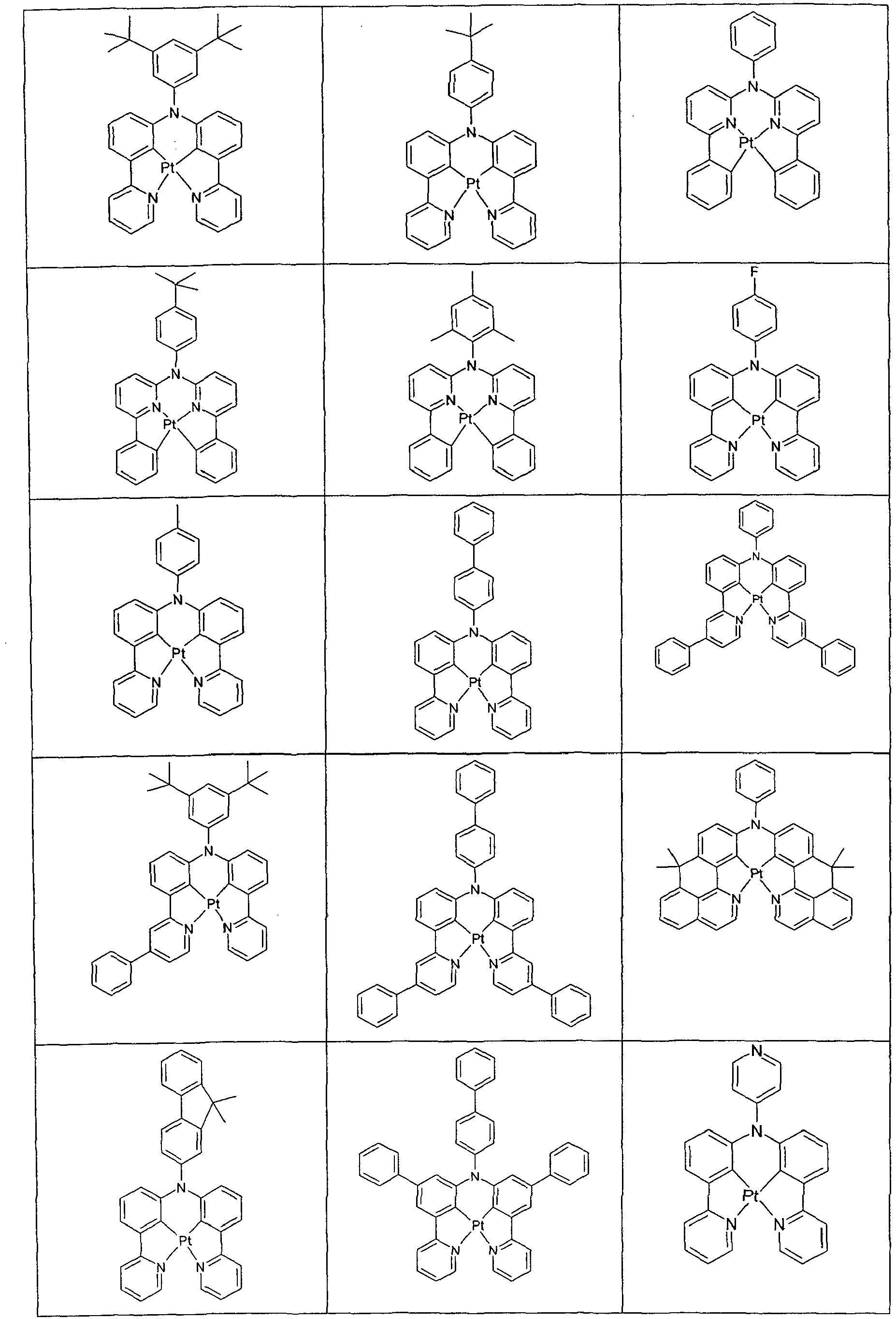

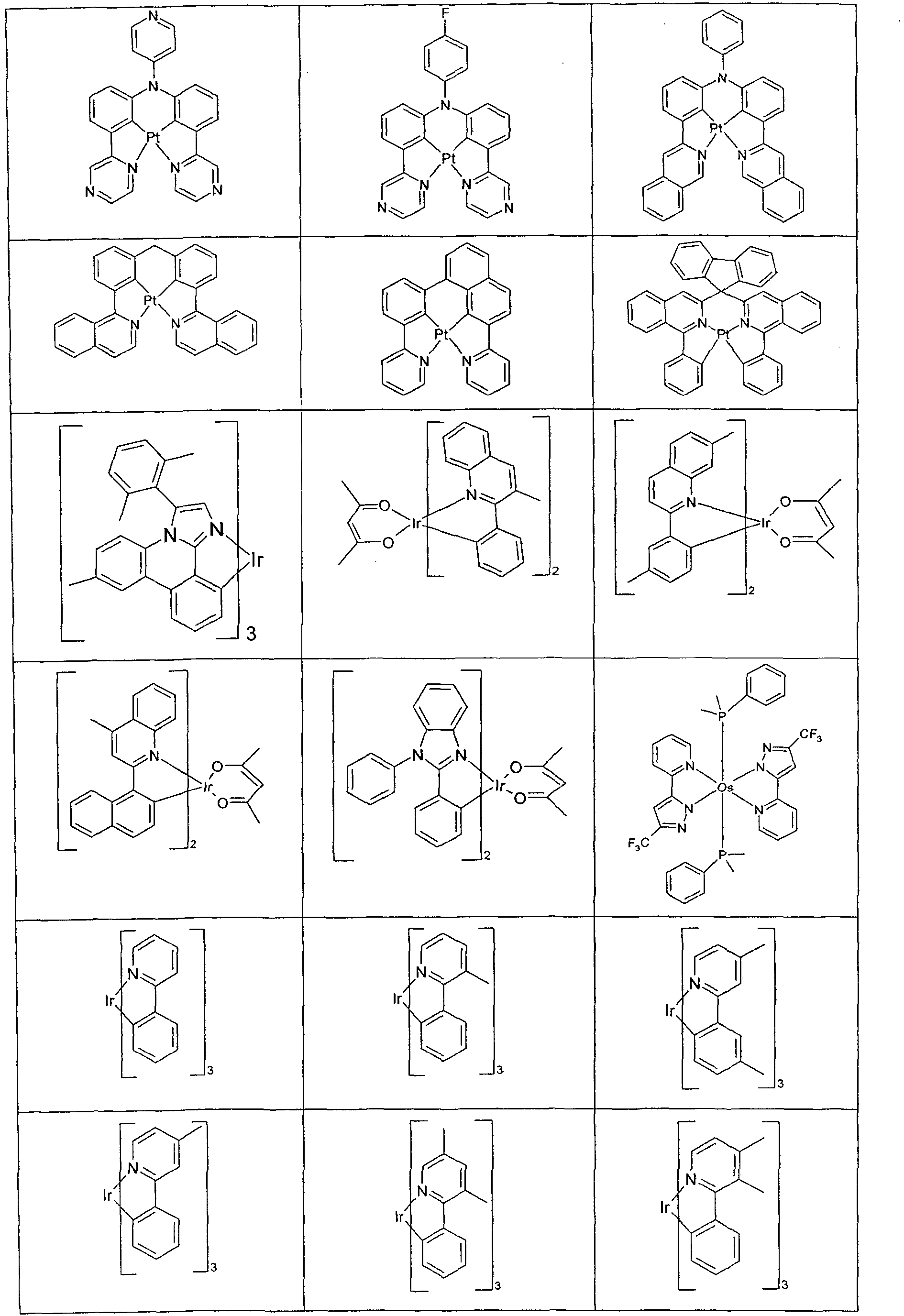

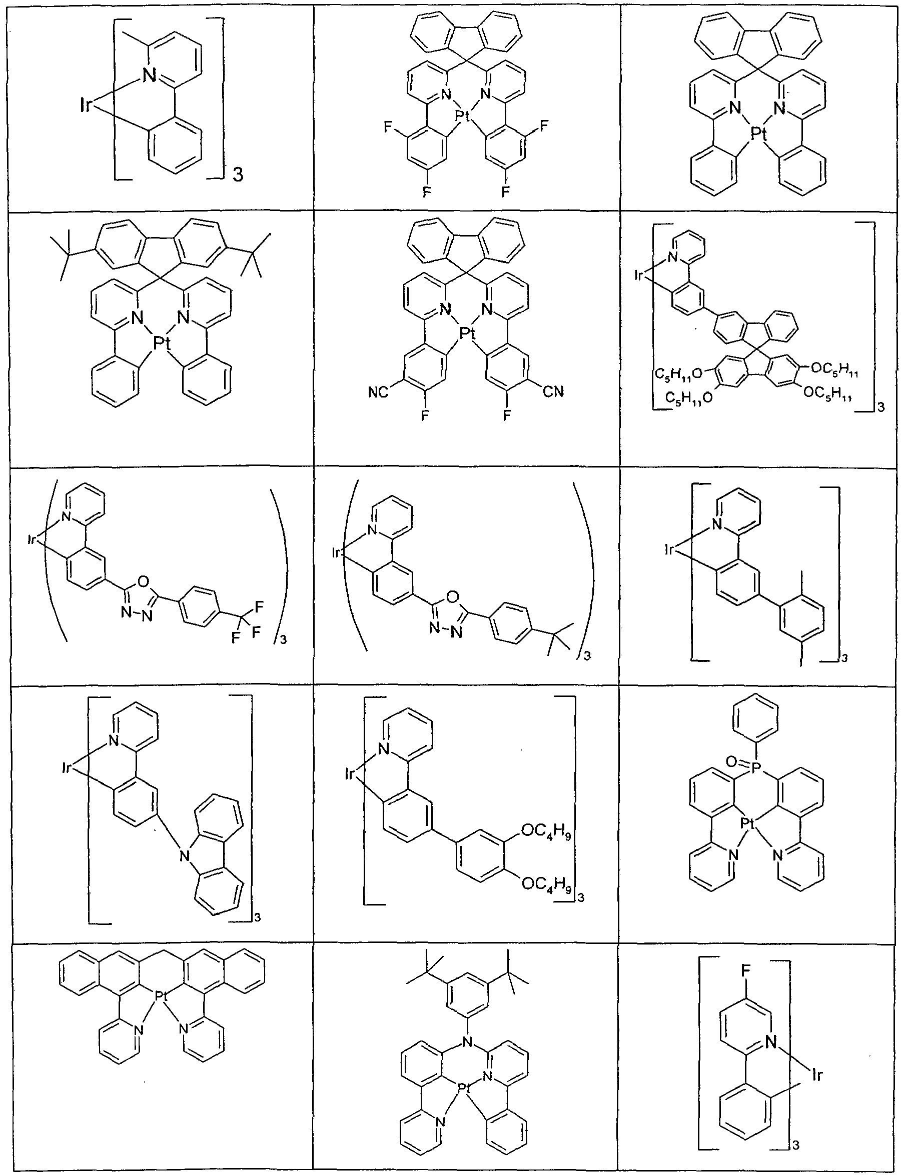

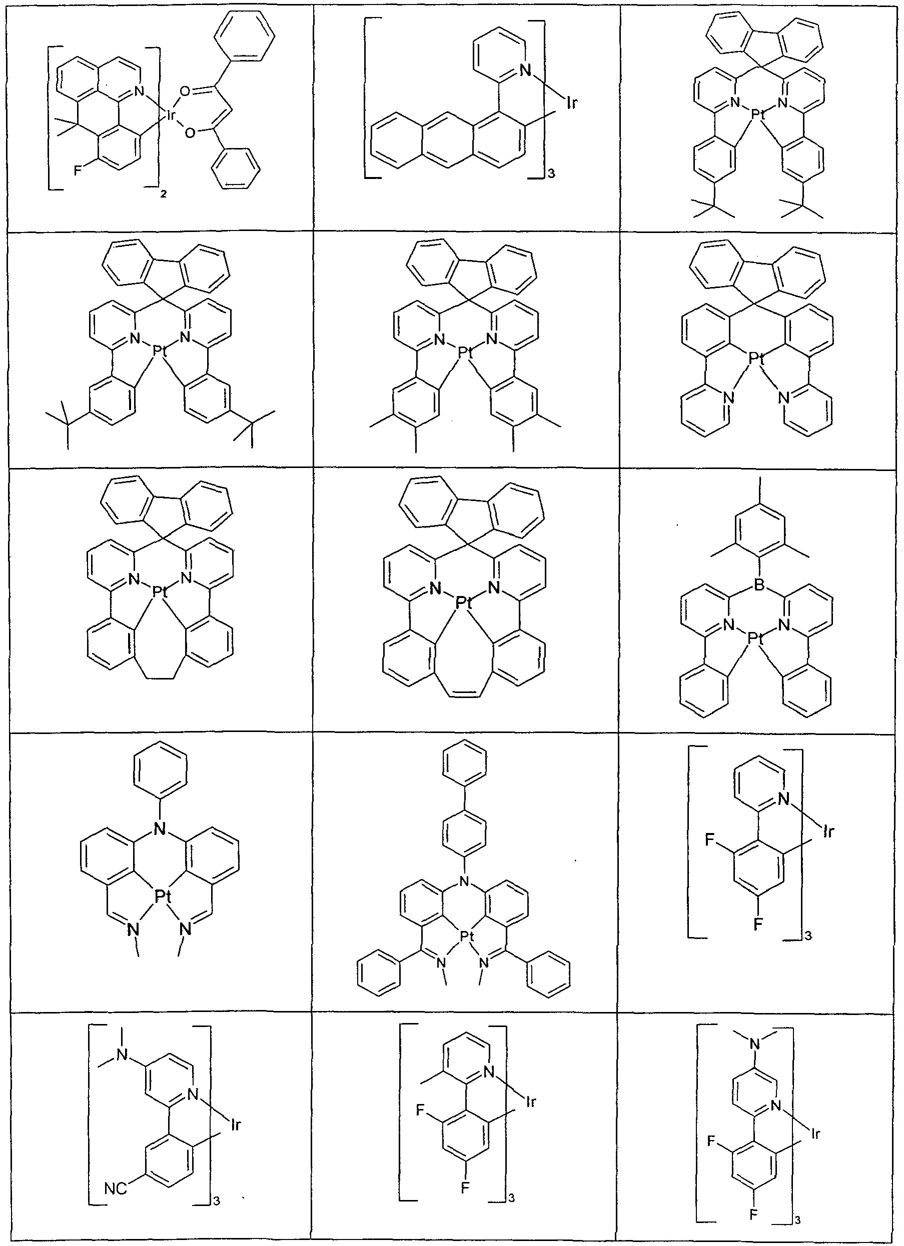

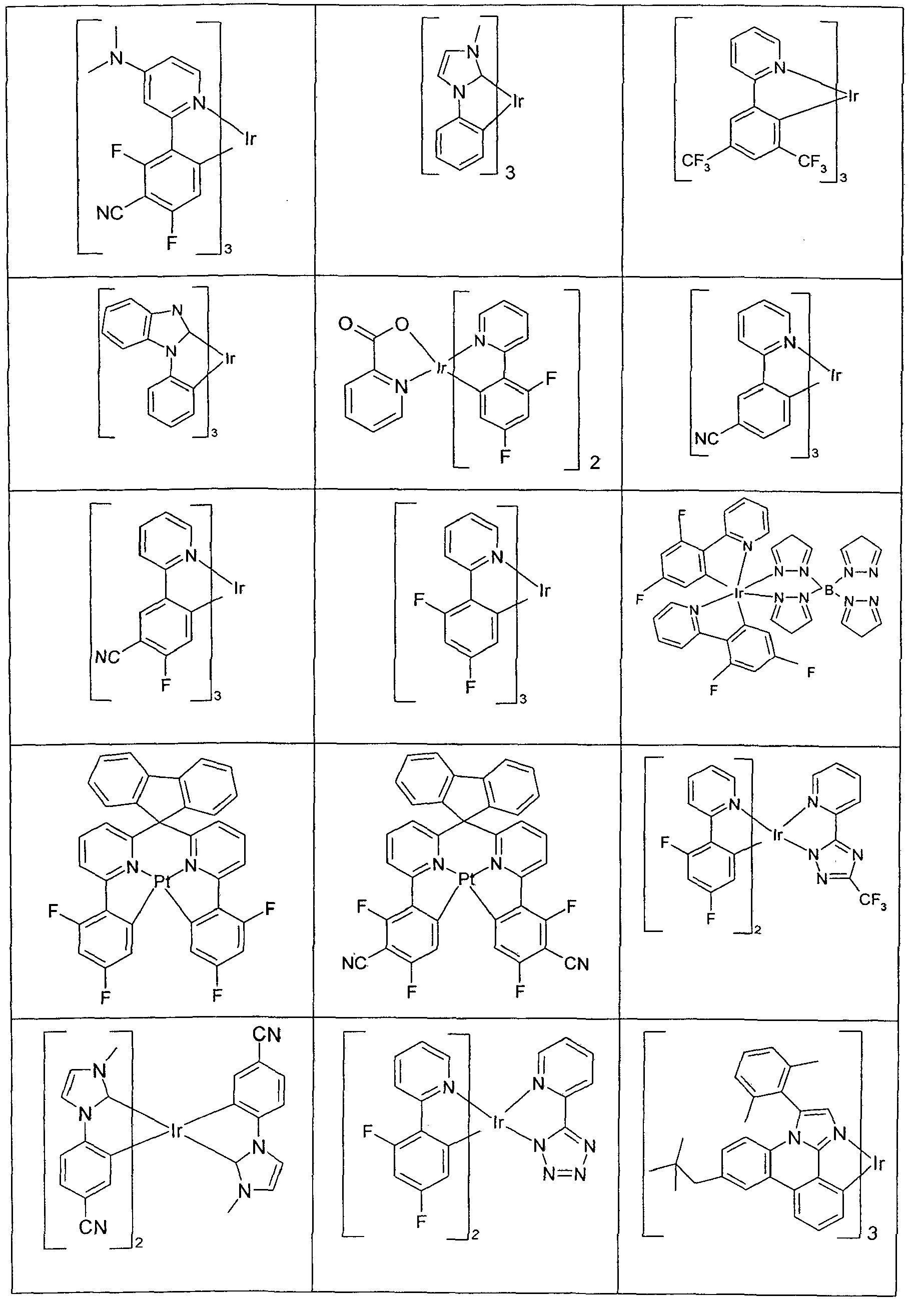

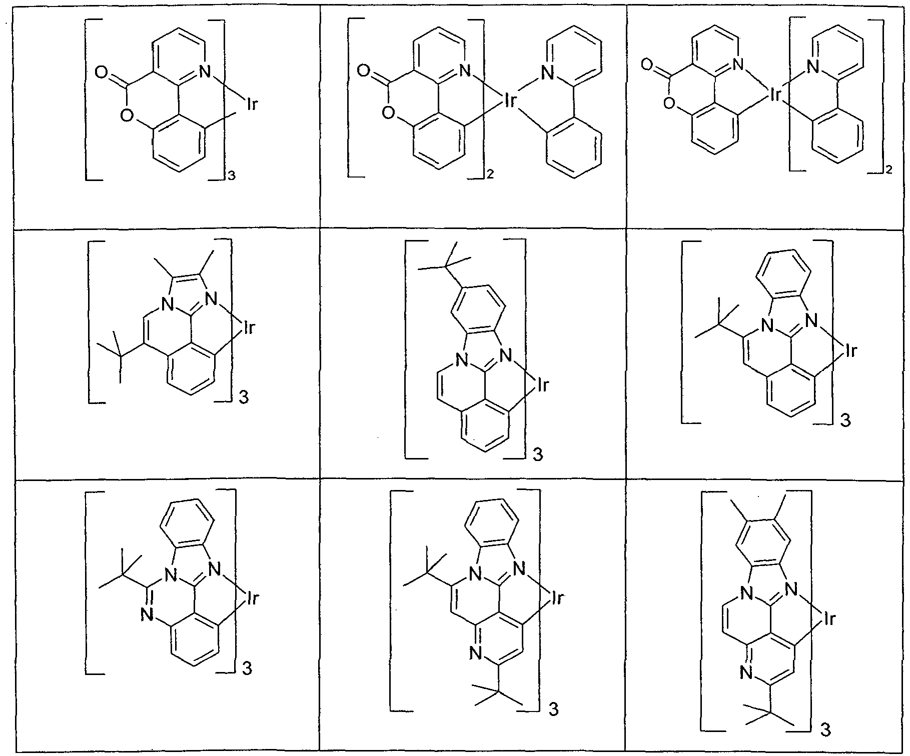

Bevorzugte fluoreszierende emittierende Verbindungen sind neben den Verbindungen der Formel (I) ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine und die in WO 2014/111269 offenbarten erweiterten Benzoindenofluorene.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen.

Bevorzugte Matrixmaterialien für phosphoreszierer:de emittierende Verbindungen sind neben den Verbindungen der Formel (I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

### A-1) Verbindungen des Typs (1)

### Synthese von Biphenyl-4-yl-(9,9-dimethyl-9H-fluoren-2-yl)-phenanthren-4-yl-amin (1-1)

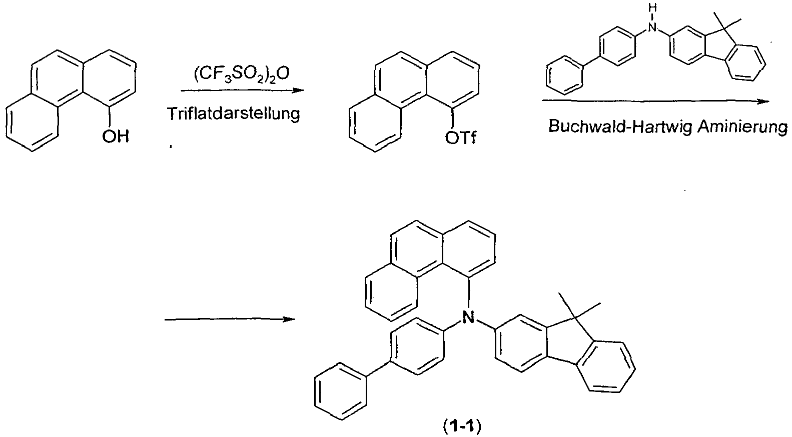

### Zwischenstufe: Trifluormethansulfonsäure-phenanthren-4-yl-ester

20 g (103 mmol) 4-Hydroxy-Phenanthren (CAS.Nr.: 7651-86-7, Synthese in Tetrahedron 2010, 66(12), 2111 beschrieben) und 42.8 mL Pyridin (309 mmol) werden in 130 mL CH₂Cl₂ gelöst. Bei 5°C werden 21.2 mL (128 mmol) Trifluoromethansulfonsäureanhydrid zugefügt. Der Ansatz wird 5 Stunden weitergerührt. Das Gemisch wird im Anschluss zwischen CH₂Cl₂ und Wasser verteilt, die organische Phase dreimal mit Wasser gewaschen und über Na₂SO₄ getrocknet und einrotiert. Nach Filtration des Rohprodukts über Kieselgel mit Toluol wird der verbleibende Rückstand aus Heptan/Toluol umkristallisiert. Die Ausbeute beträgt 29,4 g (87% der Theorie).

### Biphenyl-4-yl-(9,9-dimethyl-9H-fluoren-2-yl)-phenanthren-4-yl-amin (1-1)

29,4 g des Triflats (90 mmol) und 32,6 g Biphenyl-4-yl-(9,9-dimethyl-9H-fluoren-2-yl)-amin (90 mmol) werden in 340 mL Toluol gelöst. Die Lösung wird entgast und mit N₂ gesättigt. Danach wird sie mit 2,2 g (5,4 mmol) SPhos und 4,13 g Palladium-dba (4,5 mmol) versetzt. Anschließend werden 17,3 g Natrium-tert-butylat (180 mmol) zugegeben. Die Reaktionsmischung wird 4 h unter Schutzatmosphäre auf 85°C erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohprodukts über Kieselgel mit Toluol wird der verbleibende Rückstand aus Heptan/Toluol umkristallisiert und anschließend im Hochvakuum sublimiert. Die Reinheit beträgt 99.9%. Die Ausbeute beträgt 38 g (80% der Theorie).

Analog zu der oben beschriebenen Synthese von Verbindung (**1-1**) werden die folgenden Verbindungen (**1-2**) bis (**1-8**) hergestellt.

| | Edukt 1 | Edukt 2 | Produkt | Ausbeute |
|---|---|---|---|---|
| **1-2** | 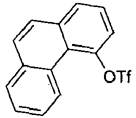 | 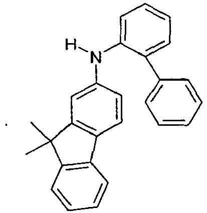 | 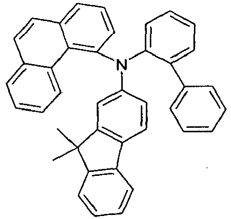 | 75 % |
| **1-3** | 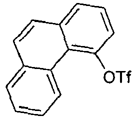 | 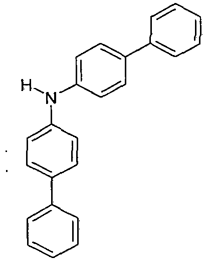 | 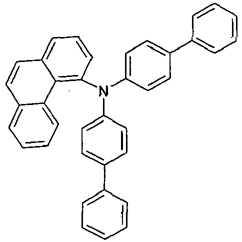 | 82% |
| **1-4** | 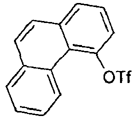 | 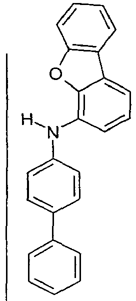 | 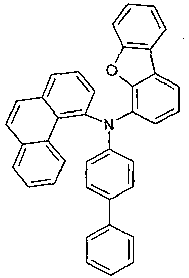 | 86% |
| **1-5** | 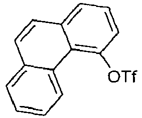 | 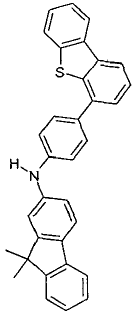 | 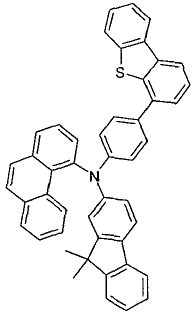 | 77% |
| **1-6** | 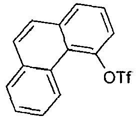 | 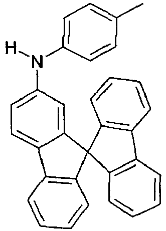 | 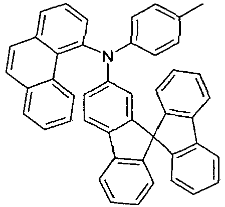 | 73% |
| **1-7** | 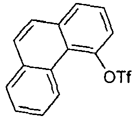 | 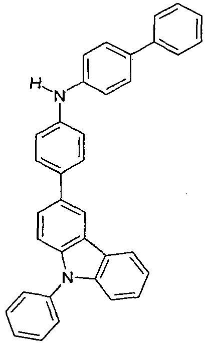 | 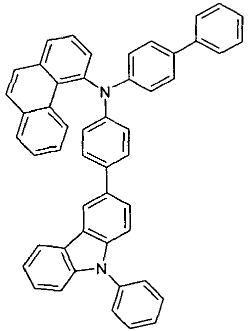 | 81% |
| **1-8** | 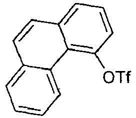 | 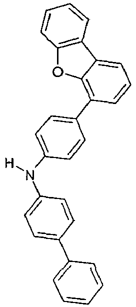 | 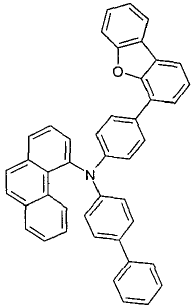 | 75% |

### A-2) Verbindungen des Types (2)

### Synthese von Biphenyl-4-yl-(9,9-dimethyl-9H-fluoren-2-yl)-(4-phenanthren-4-yl-phenyl)-amin (Verbindung (2-1))

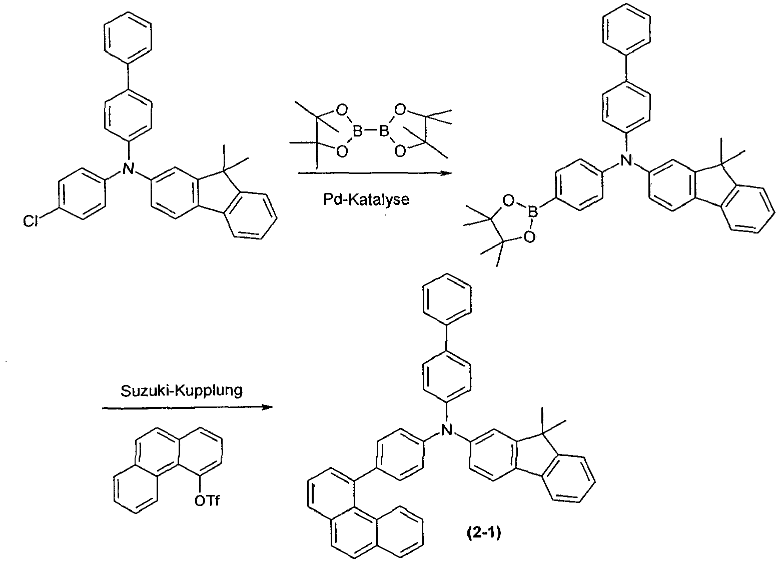

### Vorstufe: Biphenyl-4-yl-(4-chlorphenyl)-(9,9-dimethyl-9H-fluoren-2-yl)-amin

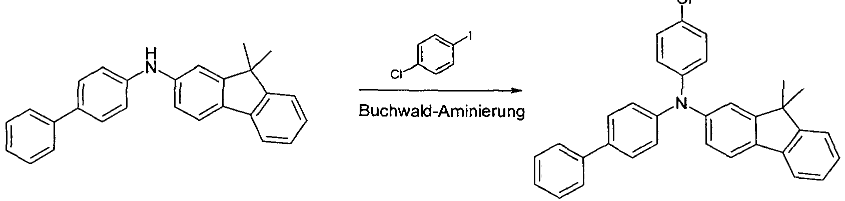

40 g g Biphenyl-4-yl-(9,9-dimethyl-9H-fluoren-2-yl)-amin (111 mmol), 26,4 g 4-Chlor-iodbenzol (111 mmol) werden in 700 mL Toluol gelöst. Die Lösung wird entgast und mit N₂ gesättigt. Danach wird sie mit 4,4 mL (4,4 mmol) einer Tri-tert-Butylphosphin 1M-Lösung und 0,5 g (2,21 mmol) Palladium(II)acetat versetzt und anschließend werden 15,9 g Natrium-tert-butylat (166 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 47 g (90% d. Th).

### Zwischenstufe: Biphenyl-4-yl-(9,9-dimethyl-9H-fluoren-2-yl)-[4-(4,4,5,5-tetramethyl-[1,3,2]dioxaborolan-2-yl)-phenyl]-amin

20g (42 mmol) Biphenyl-4-yl-(4-chloro-phenyl)-(9,9-dimethyl-9H-fluoren-2-yl)-amin, 12,5 g (50,8 mmol) Bis(pinacolato)diboran und 12,5 g (127 mmol) Kalliumacetat werden in 400 ml Dioxan suspendiert. Zu dieser Suspension werden 1,04g (1,27 mmol) 1,1-Bis(diphenylphosphino)ferrocen-dichloropalladium(II) Komplex mit DCM gegeben. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird aus Toluol umkristallisiert (21,7 g, 91% Ausbeute).

Analog dazu werden folgende Verbindungen hergestellt:

| **Edukt 1** | **Produkt** | **Ausbeute** |
|---|---|---|
| 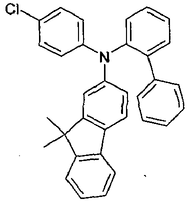 | 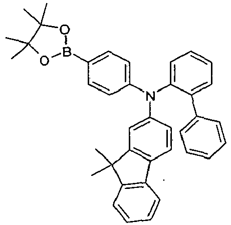 | 87 % |
| 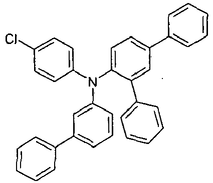 | 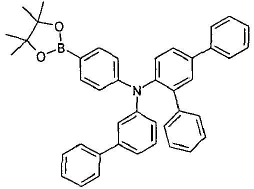 | 94% |
| 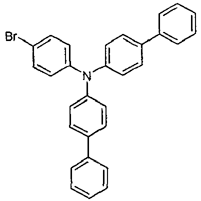 | 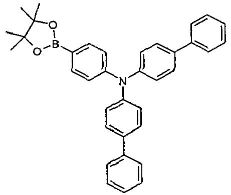 | 82% |
| 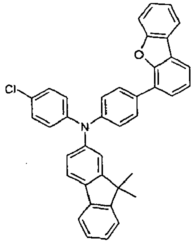 | 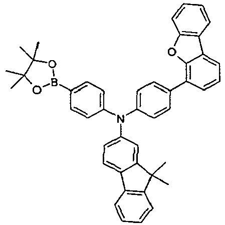 | 89% |
| 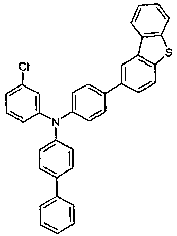 | 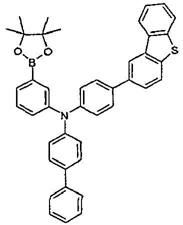 | 77% |
| 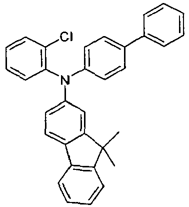 | 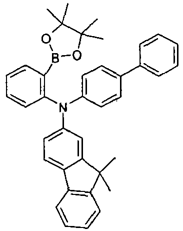 | 89% |
| 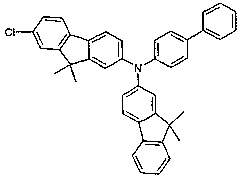 | 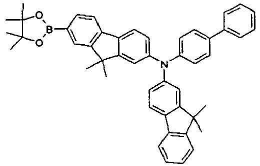 | 95% |

### Biphenyl-4-yl-(9,9-dimethyl-9H-fluoren-2-yt)-(4-phenanthren-4-yl-phenyl)-amin (Verbindung (2-1))

23g (40,8 mmol) Biphenyl-4-yl-(9,9-dimethyl-9H-fluoren-2-yl)-[4-(4,4,5,5-tetramethyl-[1,3,2]dioxaborolan-2-yl)-phenyl]-amin, 12,1 g (37,1 mmol) Trifluormethansulfonsäurephenanthren-4-yl-ester, 7,78 g Natriummetaborat (55,6 mmol) und 54 µL Hydraziniumhydroxid werden in 600 mL THF suspendiert. Zu dieser Suspension werden 0,52 g (0,742 mmol) Palladium dichlorid-bis(triphenylphosphin) gegeben, und die Reaktionsmischung wird 24 h unter Rückfluss erhitzt. Nach Erkalten wird die organische Phase abgetrennt, über Kieselgel filtriert, dreimal mit 100 mL Wasser gewaschen und anschließend zur Trockene eingeengt. Nach Filtration des Rohprodukts über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 17 g (75% d. Th).

Analog dazu werden folgende Verbindungen (2-2) bis (2-8) hergestellt:

| | **Edukt 1** | **Edukt 2** | **Produkt** | **Ausbeute** |
|---|---|---|---|---|
| **2-2** | 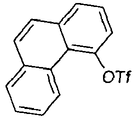 | 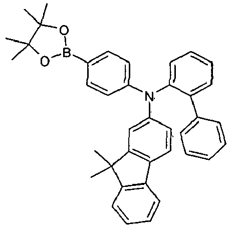 | 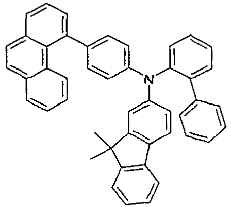 | 78 % |
| **2-3** | 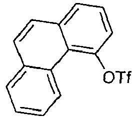 | 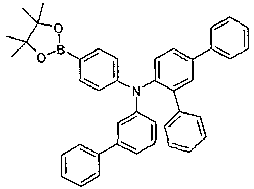 | 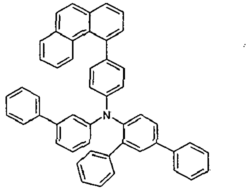 | 71% |
| **2-4** | 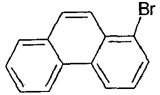 | 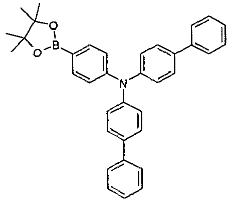 | 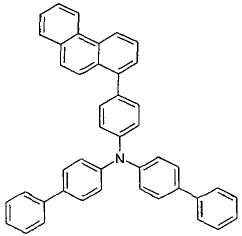 | 82% |
| | (CAS-Nr: 51958-51-1) | | | |
| **2-5** | 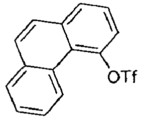 | 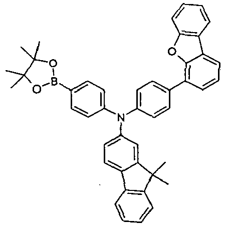 | 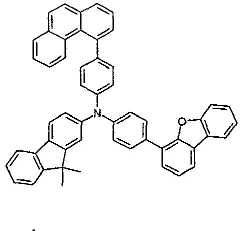 | 89% |
| **2-6** | 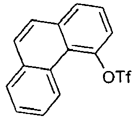 | 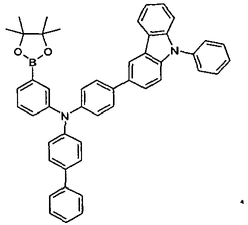 | 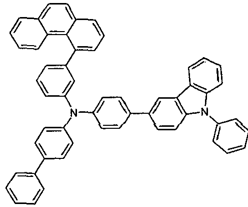 | 69% |
| **2-7** | 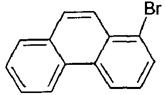 | 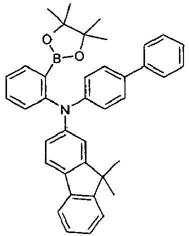 | 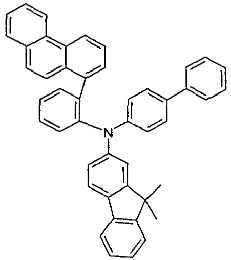 | 88% |
| | (CAS-Nr: 51958-51-1) | | | |
| **2-8** | 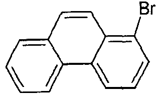 | 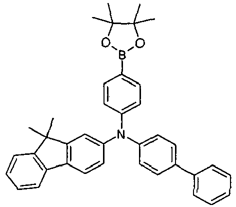 | 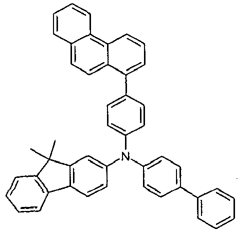 | 84% |
| | (CAS-Nr: 51958-51-1) | | | |
| **2-9** | 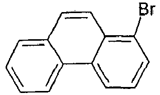 | 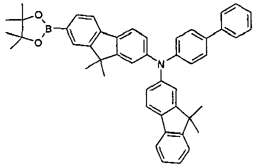 | 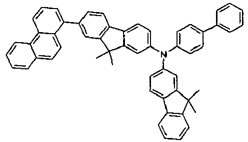 | 87% |
| | (CAS-Nr: 51958-51-1) | | | |

### A-3) Verbindungen des Typs (3)

### Synthese von Biphenyl-4-yl-(9,9-dimethyl-9H-fluoren-2-yl)-phenanthren-1-yl-amin (Verbindung (3-1))

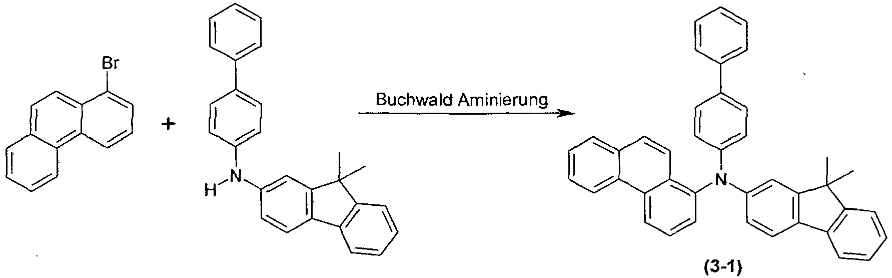

20 g 1-Brom-phenanthren (CAS-Nr: 51958-51-1) (78 mmol), 26,7 g Biphenyl-4-yl-(9,9-dimethyl-9H-fluoren-2-yl)-amin (74 mol) werden in 500 mL Toluol gelöst: Die Lösung wird entgast und mit N₂ gesättigt. Danach wird sie mit 3,1 mL (3,1 mmol) einer Tri-tert-Butylphosphin-Lösung und 0,35 g (1,56 mmol) Palladium(II)acetat versetzt. Anschließend werden 11,6 g Natrium-tert-butylat (117 mmol) zugegeben. Die Reaktionsmischung wird 3 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9 % (HPLC). Die Ausbeute beträgt 33 g (80% der Theorie).

Analog dazu werden folgende Verbindungen (3-2) bis (3-6) hergestellt:

| | **Edukt 1** | **Edukt 2** | **Produkt** | **Ausbeute** |
|---|---|---|---|---|
| **3-2** | 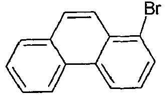 | 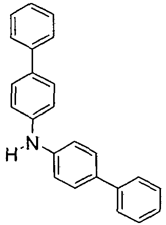 | 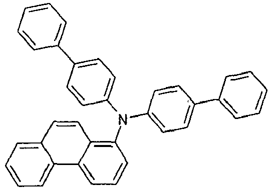 | 65% |
| **3-3** | 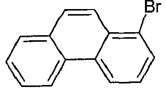 | 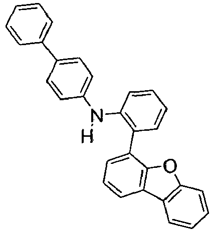 | 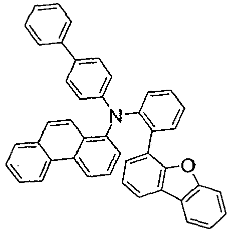 | 75% |
| **3-4** | 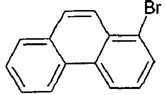 | 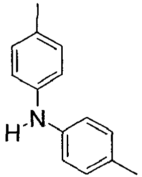 | 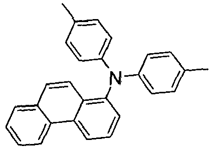 | 78% |
| **3-5** | 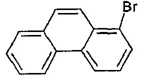 | 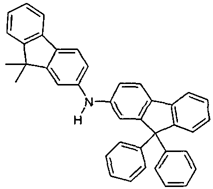 | 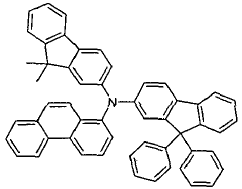 | 81% |
| **3-6** | 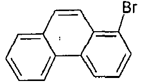 | 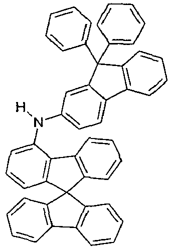 | 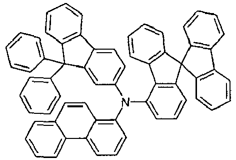 | 69% |

### A-4 Verbindungen des Typs (4)

### Synthese von N*4*-Biphenyl-4-yl-N*4*-(9,9-dimethyl-9H-fluoren-2-yl)-N*1*,N*1*-di-p-tolyl-phenanthren-1,4-diamin (4-1)

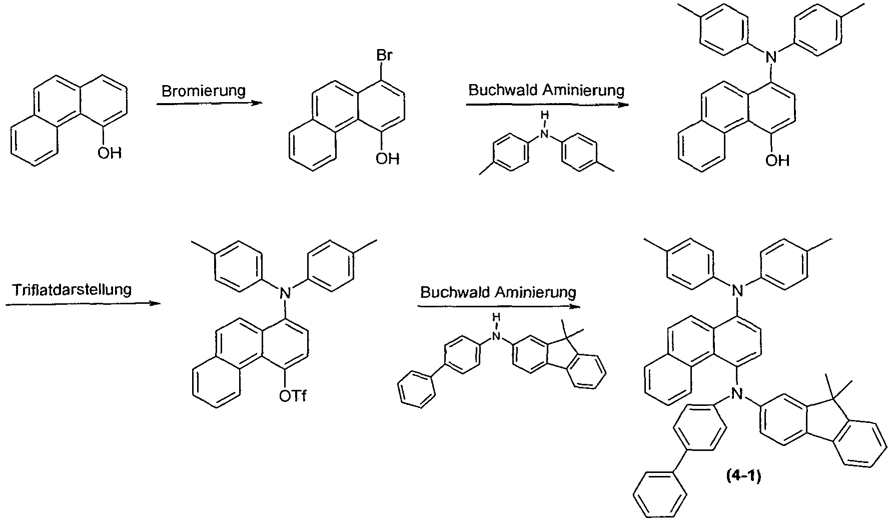

### Vorstufe: 1-Bromo-phenanthren-4-ol

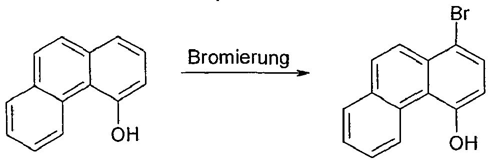

40,0 g (06 mmol) 4-Phenanthrol werden in 500 mL Acetonitril vorgelegt. Anschließend tropft man unter Lichtausschluss bei -15 °C eine Lösung aus 38,5 g (216 mmol) NBS in 100 ml CH₃CN hinzu, lässt auf RT kommen und rührt 4 h weiter bei dieser Temperatur. Anschließend wird die Mischung mit 250 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt.
Ausbeute: 42,5 g (154 mmol), 75 % d. Th.

Zwischenstufe: 1-(Di-p-tolyl-amino)-phenanthren-4-ol Analog zu der oben unter A-3) beschriebenen Synthese werden auch die folgenden Verbindungen hergestellt:

| **Edukt 1** | **Edukt 2** | **Produkt** | **Ausbeute** |
|---|---|---|---|
| 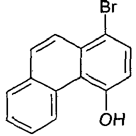 | 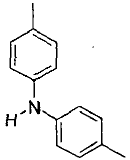 | 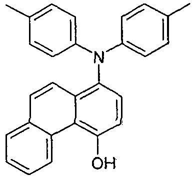 | 76% |
| 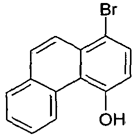 | 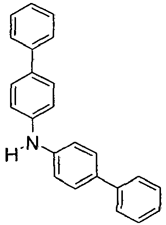 | 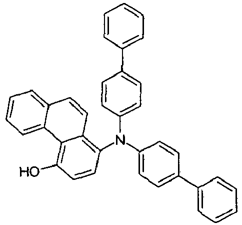 | 69% |
| 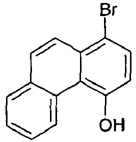 | 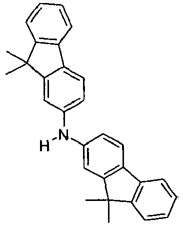 | 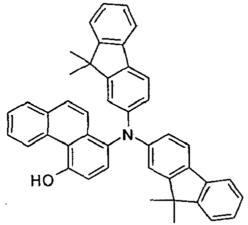 | 78% |

Analog zu der beschriebenen Synthese der Zwischenstufe Trifluormethansulfonsäure-phenanthren-4-yl-ester werden auch die folgenden Verbindungen hergestellt:

| **Edukt 1** | **Produkt** | **Ausbeute** |
|---|---|---|
| 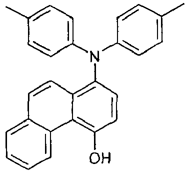 | 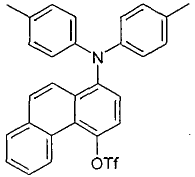 | 86% |
| 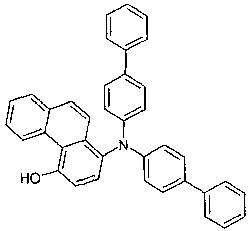 | 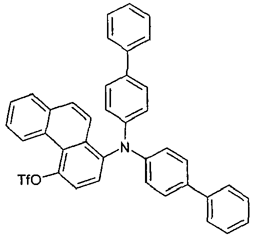 | 89% |
| 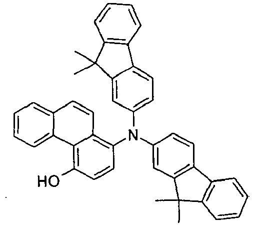 | 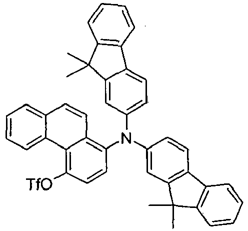 | 93% |

Analog wie oben für Verbindungen der Formel (**1-1**) beschrieben, werden die folgenden Verbindungen (**4-2**) bis (**4-4**) hergestellt:

| | **Edukt 1** | **Edukt 2** | **Produkt** | **Ausbeute** |
|---|---|---|---|---|
| **4-2** | 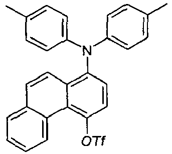 | 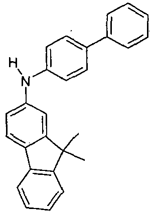 | 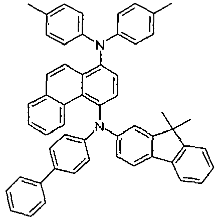 | 68 % |
| **4-3** | 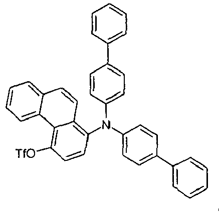 | 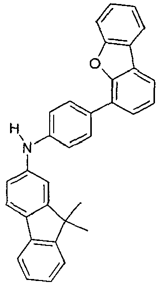 | 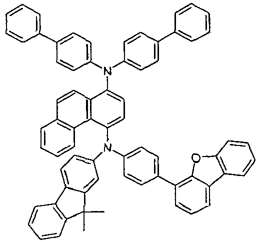 | 61% |
| **4-4** | 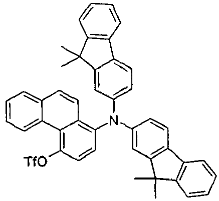 | 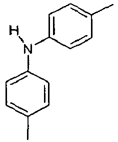 | 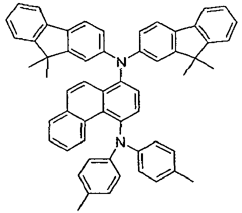 | 69% |

### A-5) Verbindungen des Typs (5)

### Bis-(9,9-dimethyl-9H-fluoren-2-yl)-(4-phenyl-phenanthren-1-yl)-amin (5-1)

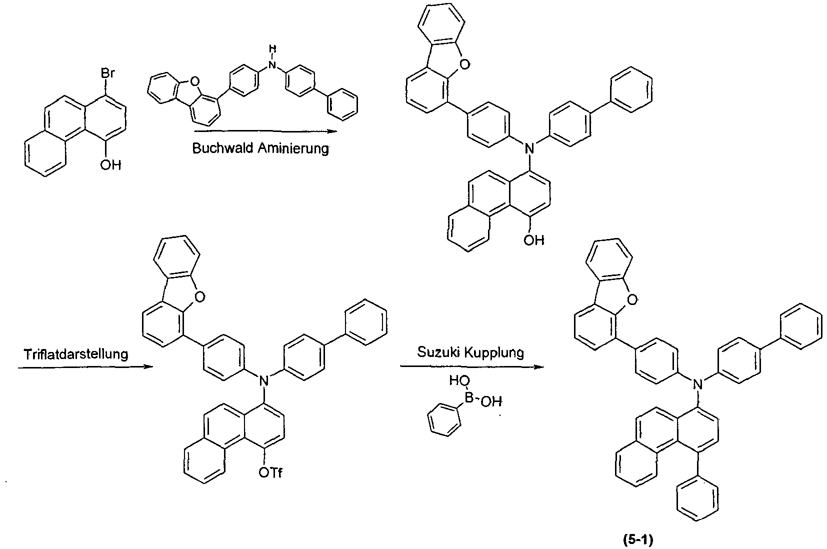

Analog wie oben unter **(2-1)** beschrieben werden auch die folgenden Verbindungen **(5-2)** bis **(5-4)** hergestellt

| | **Edukt 1** | **Edukt 2** | **Produkt** | **Ausbeute** |
|---|---|---|---|---|
| **5-2** | 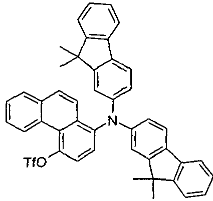 | 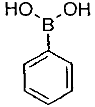 | 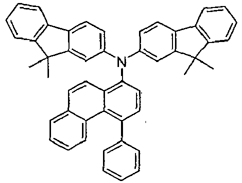 | 68 % |
| **5-3** | 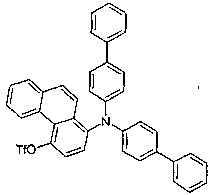 | 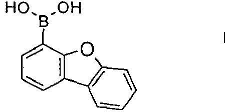 | 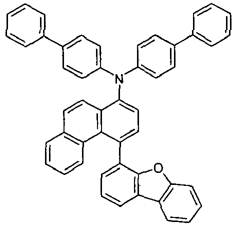 | 61 % |
| **5-4** | 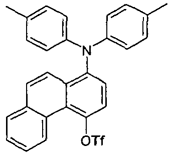 | 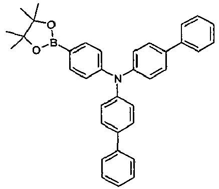 | 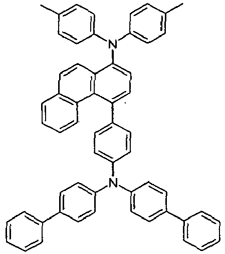 | 69 % |

### A-6) Verbindungen des Typs (6)

### Biphenyl-4-yl-{1-[4-(bis-biphenyl-4-yl-amino)-phenyl]-phenanthren-4-yl}-(9,9-dimethyl-9H-fluoren-2-yl)-amin (6-1)

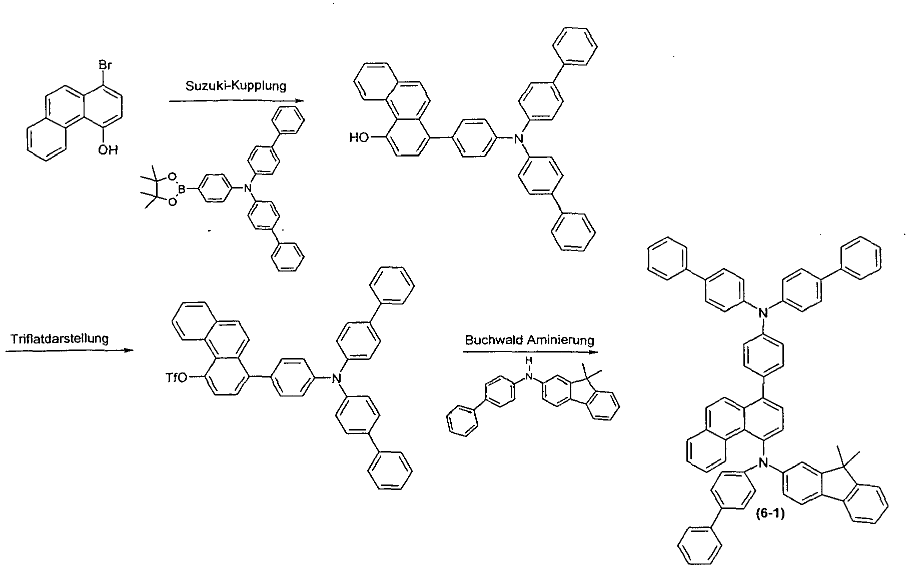

Analog zu der oben unter A-2) beschriebenen Synthese werden auch die folgenden Verbindungen hergestellt.

| **Edukt 1** | **Edukt 2** | **Produkt** | **Ausbeute** |
|---|---|---|---|
| 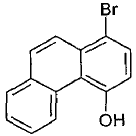 | 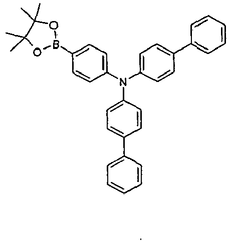 | 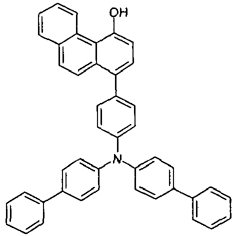 | 82% |
| 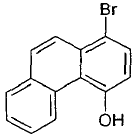 | 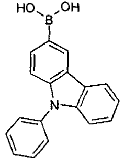 | 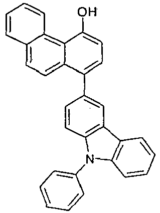 | 85% |
| 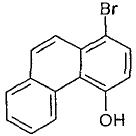 | 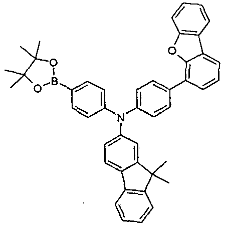 | 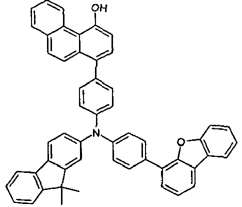 | 69% |

Analog zu der beschriebenen Synthese der Zwischenstufe Trifluormethansulfonsäure-phenanthren-4-yl-ester werden auch die folgenden Verbindungen hergestellt:

| **Edukt 1** | **Produkt** | **Ausbeute** |
|---|---|---|
| 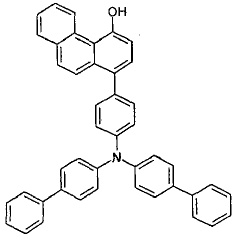 | 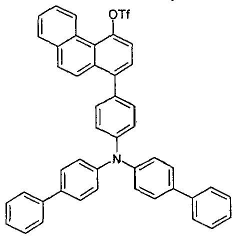 | 81% |
| 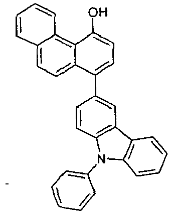 | 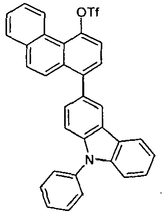 | 93% |
| 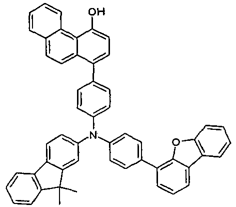 | 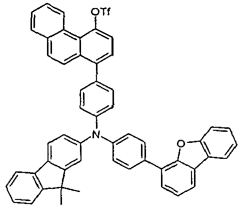 | 89% |

Analog zur oben für **(1-1)** beschriebenen Synthese werden die folgenden Verbindungen **(6-2)** bis **(6-4)** hergestellt:

| | **Edukt 1** | **Edukt 2** | **Produkt** | **Ausbeute** |
|---|---|---|---|---|
| **6-2** | 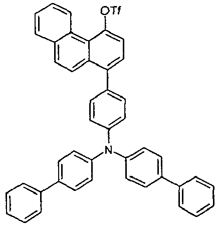 | 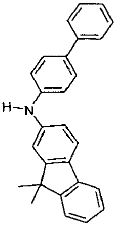 | 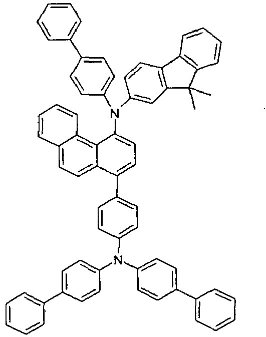 | 58 % |
| **6-3** | 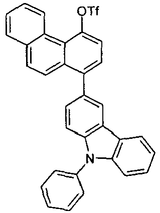 | 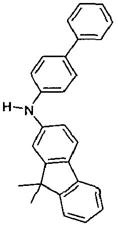 | 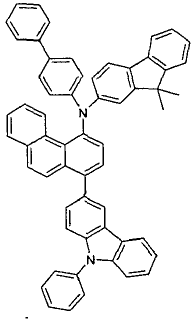 | 52% |
| **6-4** | 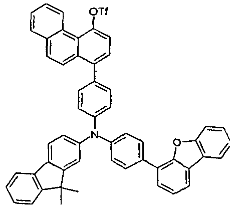 | 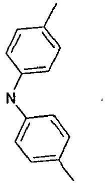 | 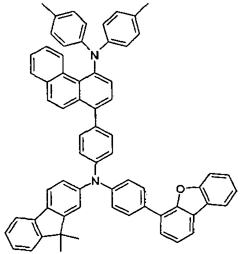 | 63% |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (z.B. Materialien) angepasst wird.

In den folgenden erfindungsgemäßen Beispielen E1 - E9 und in den Referenzbeispielen V1-V4 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben folgenden Schichtaufbau: Substrat / p-dotierte Lochtransportschicht (HIL1) / Lochtransportschicht (HTL) / p-dotierte Lochtransportschicht (HIL2) / Lochtransportschicht (EBL) / Emissionsschicht (EML) / Elektronentransportschicht (ETL) / Elektroneninjektionsschicht (EIL) und abschließend eine Kathode. Bei den Beispielen V3, V4 und E4-E9 sind jeweils die Schichten HIL2 und EBL weggelassen. Die Kathode wird durch eine 100 nm dicke Aluminiumschicht gebildet. Die zur Herstellung der OLEDs benötigten Materialien sind in Tabelle 1 gezeigt, die verschiedenen Bauteilaufbauten in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, emittierende Verbindung), der dem Matrixmaterial bzw. den Matrixmaterialien durch Coverdampfung in einem bestimmten Volumenanteil beigemischt wird. Eine Angabe wie H1:SEB(5%) bedeutet hierbei, dass das Material H1 in einem Volumenanteil von 95 % und SEB in einem Volumenanteil von 5 % in der Schicht vorliegt. Analog können auch die Elektronentransportschichten oder die Lochinjektionsschichten aus einer Mischung von zwei oder mehr Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 10 mA/cm² bezeichnet die externe Quanteneffizienz bei einer Stromdichte von 10mA/cm². LD80 @ 60 mA/cm² ist die Lebensdauer, bis zu der die OLED bei einer Starthelligkeit bei konstantem Strom von 60 mA/cm² auf 80 % der Anfangsintensität abgefallen ist.

| ***Tabelle 1: Strukturen der verwendeten Materialien*** | | |
|---|---|---|
| 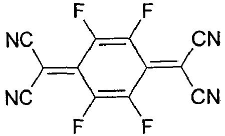 | 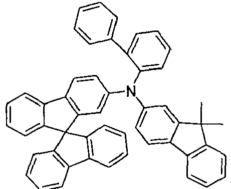 | 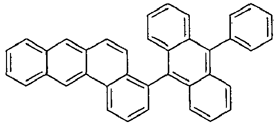 |
| F4TCNQ | HIM | H1 |
| 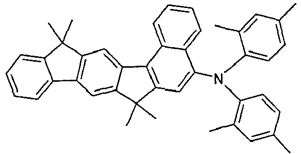 | 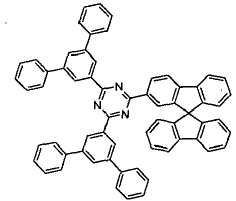 | 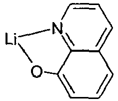 |
| SEB | ETM | LiQ |
| 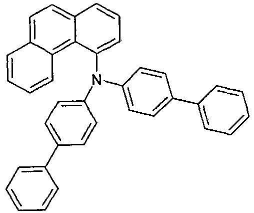 | 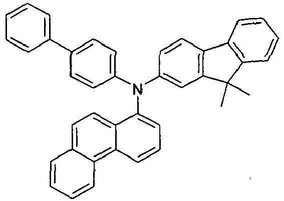 | 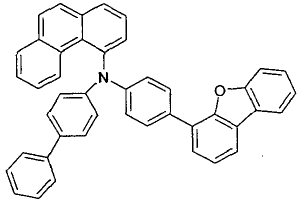 |
| HTM1 | HTM2 | HTM3 |
| 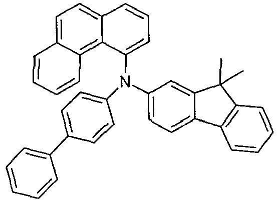 | 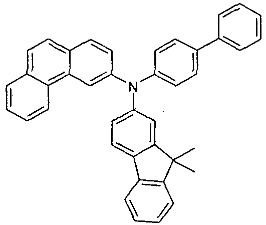 | 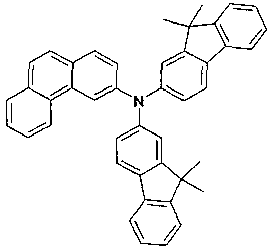 |
| HTM4 | HTMV1 | HTMV2 |
| 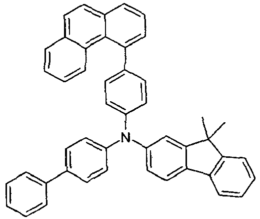 | 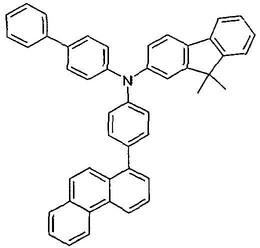 | |
| HTM5 | HTM6 | |

| ***Tabelle 2: Aufbau der OLEDs*** | | | | | | | |
|---|---|---|---|---|---|---|---|
| ***Exp.*** | ***HIL1*** | ***HTL*** | ***HIL2*** | ***EBL*** | ***EML*** | ***ETL*** | ***EIL*** |
| | *Dicke* / *nm* | *Dicke* / *nm* | *Dicke* / *nm* | *Dicke* / *nm* | *Dicke* / *nm* | *Dicke* / *nm* | *Dicke* / *nm* |
| *V1* | *HIM:F4TCNQ(5%) 20 nm* | *HIM 155 nm* | *HTMV1:F4TCNQ(5%) 20 nm* | *HTMV1 20 nm* | *H1: SEB(5%) 20 nm* | *ETM:LiQ(50%) 30 nm* | *LiQ 1 nm* |
| *V2* | *HIM:F4TCNQ(5%) 20 nm* | *HIM 155 nm* | *HTMV2: F4TCNQ(5%) 20 nm* | *HTMV2 20 nm* | *H1:SEB(5%) 20 nm* | *ETM:LiQ(50%) 30 nm* | *LiQ 1 nm* |
| *E1* | *HIM:F4TCNQ(5%) 20 nm* | *HIM 155 nm* | *HTM1:F4TCNQ(5%) 20 nm* | *HTM1 20 nm* | *H1:SEB1(5%) 20 nm* | *ETM:LiQ(50%) 30 nm* | *LiQ 1 nm* |
| *E2* | *HIM:F4TCNQ(5%) 20 nm* | *HIM 155 nm* | *HTM2:F4TCNQ(5%) 20 nm* | *HTM2 20 nm* | *H1:SEB1(5%) 20 nm* | *ETM:LiQ(50%) 30 nm* | *LiQ 1 nm* |
| *E3* | *HIM:F4TCNQ(5%) 20 nm* | *HIM 155 nm* | *HTM3:F4TCNQ(5%) 20 nm* | *HTM3 20 nm* | *H1:SEB1(5%) 20 nm* | *ETM:LiQ(50%) 30 nm* | *LiQ 1 nm* |
| *V3* | *HTMV1:F4TCNQ(5%) 20 nm* | *HTMV1 180 nm* | -- | -- | *H1:SEB1(5%) 20 nm* | *ETM:LiQ(50%) 30 nm* | *LiQ 1 nm* |
| *V4* | *HTMV2:F4TCNQ(5%) 20 nm* | *HTMV2 180 nm* | -- | - | *H1:SEB1(5%) 20 nm* | *ETM:LiQ(50%) 30 nm* | *LiQ 1 nm* |
| *E4* | *HTM4: F4TCNQ(5%) 20 nm* | *HTM4 180 nm* | -- | -- | *H1:SEB1(5%) 20 nm* | *ETM:LiQ(50%) 30 nm* | *LiQ 1 nm* |
| *E5* | *HTM3: F4TCNQ(5%) 20 nm* | *HTM3 180 nm* | -- | -- | *H1:SEB1(5%) 20 nm* | *ETM:LiQ(50%) 30 nm* | *LiQ 1 nm* |
| *E6* | *HTM2:F4TCNQ(5%) 20 nm* | *HTM2 180 nm* | -- | -- | *H1:SEB1(5%) 20 nm* | *ETM: LiQ(50%) 30 nm* | *LiQ 1 nm* |
| E7 | *HTM1:F4TCNQ(5%) 20 nm* | *HTM1 180 nm* | -- | -- | *H1:SEB1(5%) 20 nm* | *ETM:LiQ(50%) 30 nm* | *LiQ. 1 nm* |
| *E8* | *HTMS:F4TCNQ(5%) 20 nm* | *HTM5 180 nm* | -- | -- | *H1:SEB1(5%) 20 nm* | *ETM:LiQ(50%) 30 nm* | *LiQ 1 nm* |
| *E9* | *HTM6:F4TCNQ(5%) 20 nm* | *HTM6 180 nm* | -- | -- | *H1:SEB1(5%) 20 nm* | *ETM:LiQ*(*50*%) *30 nm* | *LiQ 1 nm* |

### Beispiel 1

Im Beispiel 1 werden drei erfindungsgemäße Substanzen (HTM1, HTM2 und HTM3) und zwei Referenzsubstanzen (HTMV1, HTMV2) in einer OLED mit blau fluoreszierender emittierender Schicht verglichen. Die Verbindungen werden jeweils in lochtransportierenden Schichten der OLED eingesetzt.

Die Referenzprobe V1, die eine 3-Phenanthren-Verbindung enthält, wird mit zwei erfindungsgemäßen Bauteilen verglichen, die eine 4-Phenanthren- (E1) bzw. eine 1-Phenanthren-Verbindung (E2) enthalten. Die Lebensdauer LD80 bei 60 mA/cm² ist bei den erfindungsgemäßen Proben E1 (357 h) und E2 (381h) deutlich besser als die Referenzprobe V1 (128 h).

Die externe Quanteneffizienz bei 10mA/cm² der erfindungsgemäßen Verbindung aus Probe E3 (einer 4-Phenanthren-Verbindung) ist mit 7,7% deutlich besser als die der Referenzprobe V2 mit lediglich 6,6%. Die Referenzprobe V2 enthält eine 3-Phenanthren-Verbindung.

### Beispiel 2

Bei OLEDs mit blau fluoreszierender emittierender Schicht (in reduzierter Bauweise; direkte Injektion der Löcher aus der HTL in die EML) zeigen die Referenzproben V3 (6,8 %) und V4 (4,2%) niedrigere Quanteneffizienz bei 10 mA/cm² als die erfindungsgemäßen Proben E4 (7,9 %) und E5 (7,7%). Auch ist die Lebensdauer (80%) bei 60 mA/cm² der erfindungsgemäßen Proben E6 (356 h) und E7 (218h) größer als bei den Referenzen V3 (106 h) und V4 (43h). Auch in diesem Beispiel enthalten die Referenzproben 3-Phenanthren-Verbindungen. Die erfindungsgemäßen Proben E4, E5, E6 und E7 enthalten 1-Phenanthen-Verbindungen bzw. 4-Phenanthren-Verbindungen.

### Beispiel 3

Weiterhin werden zwei Bauteile E8 und E9 hergestellt, die die erfindungsgemäßen Verbindungen HTM5 bzw. HTM6 enthalten. Diese beiden Verbindungen sind dadurch gekennzeichnet, dass sie eine Phenylgruppe zwischen der Phenanthren-Gruppe und der Diarylaminogruppe aufweisen. Die Bauteile werden wie diejenigen des Beispiels 2 mit reduzierter Bauweise hergestellt. Für die Bauteile E8 und E9 werden externe Quanteneffizienzen von 7,3% bzw. 8,1% gemessen.

## Patentansprüche

1. Verbindung der Formel (I) 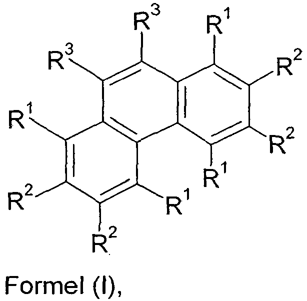 wobei für die auftretenden Symbole gilt:
R¹ ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁶ substituiert sein können, heteroaromatischen Ringsysteme mit 5 bis 40 aromatischen Ringatomen, die mit einem oder mehreren Resten R⁶ substituiert sein können, Gruppen der Formel (A) 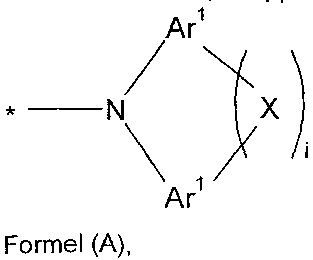 und Gruppen der Formel (B) 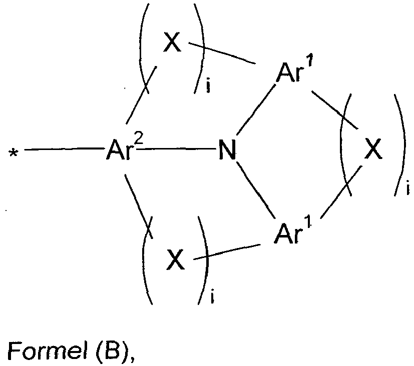 wobei die genannten Alkyl-, Alkoxy-, Alkenyl- und Alkinylgruppen jeweils mit einem oder mehreren Resten R⁶ substituiert sein können, und
wobei eine oder mehrere CH₂-Gruppen in den genannten Alkyl-, Alkoxy-, Alkenyl- und Alkinylgruppen durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können, und
wobei die mit * markierte Bindung in Formel (A) und Formel (B) jeweils die Bindung an die Phenanthren-Einheit bezeichnet;
R²,R³ sind bei jedem Auftreten gleich oder verschieden gewählt aus H, D, F, C(=O)R⁶, CN, Si(R⁶)₃, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁶ substituiert sein können, und heteroaromatischen Ringsysteme mit 5 bis 40 aromatischen Ringatomen, die mit einem oder mehreren Resten R⁶ substituiert sein können,
wobei die genannten Alkyl-, Alkoxy-, Alkenyl- und Alkinylgruppen jeweils mit einem oder mehreren Resten R⁶ substituiert sein können, und
wobei eine oder mehrere CH₂-Gruppen in den genannten Alkyl-, Alkoxy-, Alkenyl- und Alkinylgruppen durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R⁶ ist bei jedem Auftreten gleich oder verschieden gewählt aus H, D, F, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁷ substituiert sein können, und heteroaromatischen Ringsysteme mit 5 bis 40 aromatischen Ringatomen, die mit einem oder mehreren Resten R⁷ substituiert sein können,
wobei die genannten Alkyl-, Alkoxy-, Alkenyl- und Alkinylgruppen jeweils mit einem oder mehreren Resten R⁷ substituiert sein können,
wobei eine oder mehrere CH₂-Gruppen in den genannten Alkyl-, Alkoxy-, Alkenyl- und Alkinylgruppen durch -R⁷C=CR⁷-, -C≡C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können, und
wobei zwei oder mehr Substituenten R⁶ miteinander verknüpft sein können und einen Ring bilden können;
R⁷ ist bei jedem Auftreten gleich oder verschieden gewählt aus H, D, F, CN, und aliphatischen, aromatischen oder heteroaromatischen organischen Resten mit 1 bis 20 C-Atomen, in denen auch ein oder mehrere H-Atome durch D, F oder CN ersetzt sein können;
Ar¹ ist bei jedem Auftreten gleich oder verschieden gewählt aus aromatischen Ringsystemen mit 6 bis 40 aromatischen Ringatomen, die mit einem oder mehreren Resten R⁴ substituiert sein können, und heteroaromatischen Ringsystemen mit 5 bis 40 aromatischen Ringatomen; die mit einem oder mehreren Resten R⁴ substituiert sein können;
Ar² ist bei jedem Auftreten gleich oder verschieden gewählt aus aromatischen Ringsystemen mit 6 bis 40 aromatischen Ringatomen, die mit einem oder mehreren Resten R⁴ substituiert sein können, und heteroaromatischen Ringsystemen mit 5 bis 40 aromatischen Ringatomen, die mit einem oder mehreren Resten R⁴ substituiert sein können;
R⁴ ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁶ substituiert sein können, und heteroaromatischen Ringsystemen mit 5 bis 40 aromatischen Ringatomen, die mit einem oder mehreren Resten R⁶ substituiert sein können,
wobei die genannten Alkyl-, Alkoxy-, Alkenyl- und Alkinylgruppen jeweils mit einem oder mehreren Resten R⁶ substituiert sein können,
wobei eine oder mehrere CH₂-Gruppen in den genannten Alkyl-, Alkoxy-, Alkenyl- und Alkinylgruppen durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können, und
wobei zwei oder mehr Substituenten R⁴ miteinander verknüpft sein können und einen Ring bilden können;
X ist bei jedem Auftreten gleich oder verschieden gewählt aus Einfachbindungen, BR⁵, C(R⁵)₂, -C(R⁵)₂-C(R⁵)₂-, -C(R⁵)=C(R⁵)-, -C(R⁵)₂-O-, -C(R⁵)₂-NR⁵-, Si(R⁵)₂, C=O, NR⁵, PR⁵, P(=O)R⁵, O, S, S=O, SO₂ und wahlweise mit Resten R⁵ substituiertem ortho-Phenylen;
R⁵ ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, die mit einem oder mehreren Resten R⁶ substituiert sein können, und heteroaromatischen Ringsystemen mit 5 bis 40 aromatischen Ringatomen, die mit einem oder mehreren Resten R⁶ substituiert sein können,
wobei die genannten Alkyl-, Alkoxy-, Alkenyl- und Alkinylgruppen jeweils mit einem oder mehreren Resten R⁶ substituiert sein können,
wobei eine oder mehrere CH₂-Gruppen in den genannten Alkyl-, Alkoxy-, Alkenyl- und Alkinylgruppen durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können, und
wobei zwei oder mehr Substituenten R⁵ miteinander verknüpft sein können und einen Ring bilden können;
i ist bei jedem Auftreten gleich oder verschieden 0 oder 1,
wobei mindestens ein Rest R¹ in der Verbindung der Formel (I) gewählt ist aus Gruppen der Formel (A) und Gruppen der Formel (B).

2. Verbindung nach Anspruch 1, **dadurch gekennzeichnet, dass** genau ein Rest R¹ in der Verbindung der Formel (I) gewählt ist aus Gruppen der Formel (A) und Gruppen der Formel (B), oder dass genau zwei Reste R¹ in der Verbindung der Formel (I) gewählt sind aus Gruppen der Formel (A) und Gruppen der Formel (B).

3. Verbindung nach Anspruch 1 oder 2, **dadurch gekennzeichnet, dass** genau zwei Reste R¹ in der Verbindung der Formel (I) gewählt sind aus Gruppen der Formel (A) und Gruppen der Formel (B), wobei genau einer dieser Reste R¹ in 1-Position am Phenanthren gebunden ist und genau einer dieser Reste R¹ in 4-Position am Phenanthren gebunden ist.

4. Verbindung nach einem oder mehreren der Ansprüche 1 bis 3, **dadurch gekennzeichnet, dass** ein oder zwei Indices i pro Gruppe der Formel (B) gleich 1 sind.

5. Verbindung nach einem oder mehreren der Ansprüche 1 bis 3, **dadurch gekennzeichnet, dass** der Index i bei jedem Auftreten gleich 0 ist.

6. Verbindung nach einem oder mehreren der Ansprüche 1 bis 5, **dadurch gekennzeichnet, dass** die Gruppe (A) bei jedem Auftreten gleich oder verschieden gewählt ist aus den Formeln (A-1) bis (A-7)
| | |
|---|---|
| 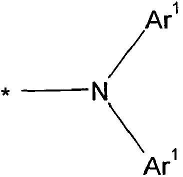 | 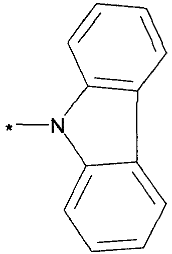 |
| Formel (A-1) | Formel (A-2) |
| 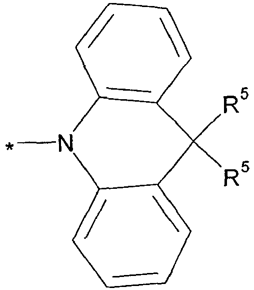 | 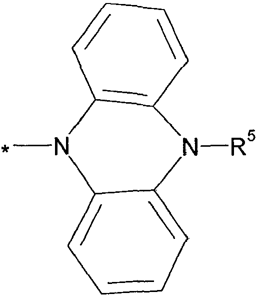 |
| Formel (A-3) | Formel (A-4) |
| | |
| 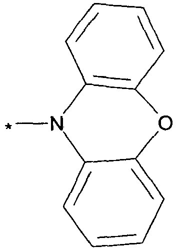 | 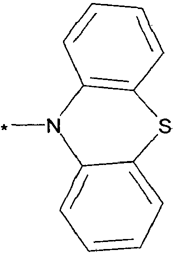 |
| Formel (A-5) | Formel (A-6) |
| | |
| 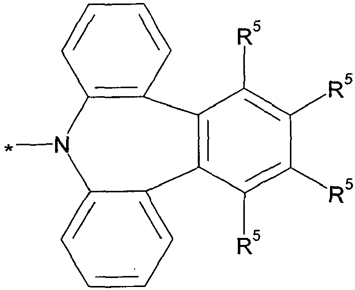 | |
| Formel (A-7) | |
welche an den freien Positionen jeweils mit Resten R⁴ substituiert sein können, und wobei die mit * markierte Bindung die Anbindungsposition an den Phenanthrenyl-Grundkörper darstellt.

7. Verbindung nach einem oder mehreren der Ansprüche 1 bis 6, **dadurch gekennzeichnet, dass** die Gruppe (B) bei jedem Auftreten gleich oder verschieden gewählt ist aus den folgenden Formeln (B-1) bis (B-15)
| | |
|---|---|
| 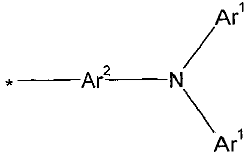 | 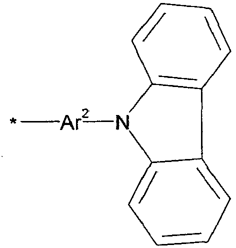 |
| Formel (B-1) | Formel (B-2) |
| | |
| 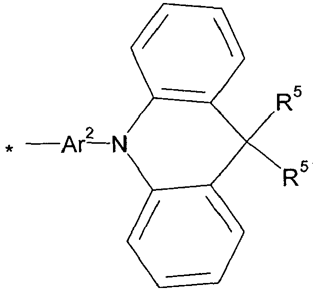 | 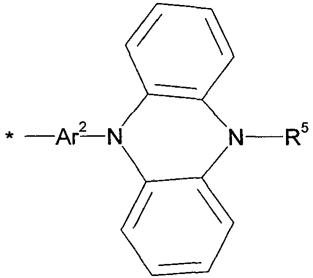 |
| Formel (B-3) | Formel (B-4) |
| | |
| 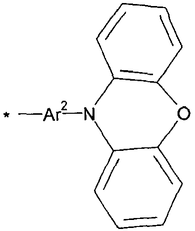 | 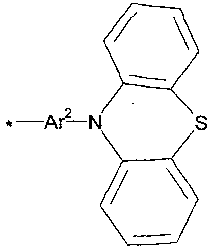 |
| Formel (B-5) | Formel (B-6) |
| 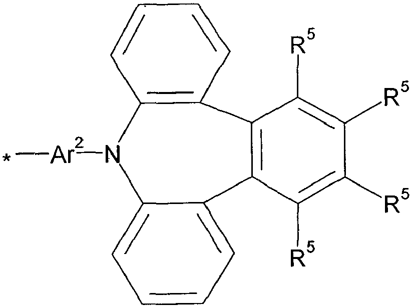 | 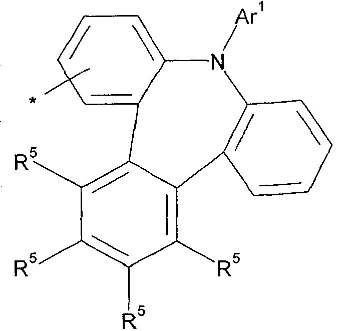 |
| Formel(B-7) | Formel (B-8) |
| | |
| 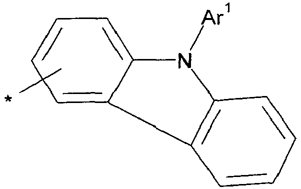 | 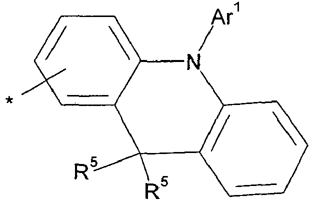 |
| Formeln (B-9) | Formel (B-10) |
| | |
| 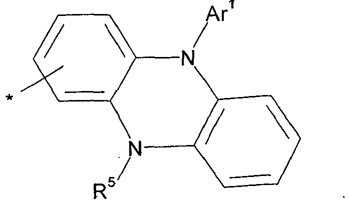 | 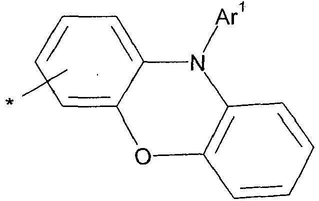 |
| Formel (B-11) | Formel (B-12) |
| | |
| 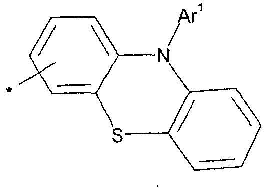 | 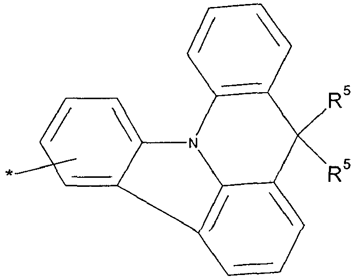 |
| Formel (B-13) | Formel (B-14) |
| 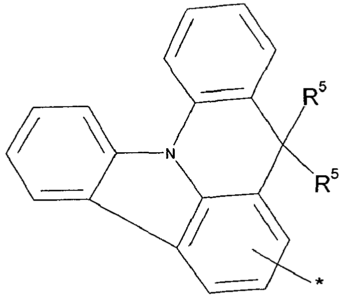 | |
| Formel (B-15) | |
welche an den freien Positionen jeweils mit Resten R⁴ substituiert sein können, und wobei die mit * markierte Bindung die Anbindungsposition an den Phenanthrenyl-Grundkörper darstellt.

8. Verbindung nach einem oder mehreren der Ansprüche 1 bis 7, **dadurch gekennzeichnet, dass** Ar¹ bei jedem Auftreten gleich oder verschieden gewählt ist aus aromatischen Ringsystemen mit 12 bis 24 aromatischen Ringatomen, die mit einem oder mehreren Resten R⁴ substituiert sein können.

9. Verbindung nach einem oder mehreren der Ansprüche 1 bis 8, **dadurch gekennzeichnet, dass** zwei Gruppen Ar¹, die an dasselbe Stickstoffatom in Formel (A) bzw. Formel (B) gebunden sind, nicht gleich gewählt sind.

10. Verbindung nach einem oder mehreren der Ansprüche 1 bis 9, **dadurch gekennzeichnet, dass** die Gruppen Ar¹ jeweils mindestens eine Gruppe enthalten, die gewählt ist aus Benzol, Naphthalin, Phenanthren, Fluoranthen, Biphenyl, Terphenyl, Quaterphenyl, Fluoren, Spirobifluoren, Furan, Benzofuran, Isobenzofuran, Dibenzofuran, Thiophen, Benzothiophen, Isobenzothiophen, Dibenzothiophen, Indol, Isoindol, Carbazol, Indolocarbazol, Indenocarbazol, Pyridin, Chinolin, Isochinolin, Acridin, Phenanthridin, Benzimidazol, Pyrimidin, Pyrazin, und Triazin, wobei die genannten Gruppen jeweils mit einem oder mehreren Resten R⁴ substituiert sein können.

11. Verbindung nach einem oder mehreren der Ansprüche 1 bis 10, **dadurch gekennzeichnet, dass** Ar² bei jedem Auftreten gleich oder verschieden gewählt ist aus aromatischen Ringsystemen mit 6 bis 13 aromatischen Ringatomen, die mit einem oder mehreren Resten R⁴ substituiert sein können.

12. Verbindung nach einem oder mehreren der Ansprüche 1 bis 11, **dadurch gekennzeichnet, dass** Gruppen X bevorzugt gewählt sind aus Einfachbindungen, C(R⁵)₂, -C(R⁵)₂-C(R⁵)₂-, -C(R⁵)=C(R⁵)-, Si(R⁵)₂, C=O, NR⁵, O, S, S=O, SO₂ und wahlweise mit Resten R⁵ substituiertem ortho-Phenylen.

13. Verbindung nach einem oder mehreren der Ansprüche 1 bis 12, **dadurch gekennzeichnet, dass** R⁴ und R⁵ bei jedem Auftreten gleich oder verschieden gewählt sind aus H, D, F, CN, Si(R⁶)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 24 aromatischen Ringatomen, die mit einem oder mehreren Resten R⁶ substituiert sein können, und heteroaromatischen Ringsystemen mit 5 bis 24 aromatischen Ringatomen, die mit einem oder mehreren Resten R⁶ substituiert sein können,
wobei die genannten Alkyl- und Alkoxygruppen jeweils mit einem oder mehreren Resten R⁶ substituiert sein können und wobei in den genannten Alkyl- und Alkoxygruppen eine oder mehrere CH₂-Gruppen durch -C≡C-, -R⁶C=CR⁶-, Si(R⁶)₂, C=O, C=NR⁶, -NR⁶-, -O-,-S-, -C(=O)O- oder -C(=O)NR⁶- ersetzt sein können.

14. Verbindung nach einem oder mehreren der Ansprüche 1 bis 13, **dadurch gekennzeichnet, dass** R⁶ bei jedem Auftreten gleich oder verschieden gewählt ist aus H, D, F, CN, Si(R⁷)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 24 aromatischen Ringatomen, die mit einem oder mehreren Resten R⁷ substituiert sein können, und heteroaromatischen Ringsystemen mit 5 bis 24 aromatischen Ringatomen, die mit einem oder mehreren Resten R⁷ substituiert sein können,
wobei die genannten Alkyl- und Alkoxygruppen jeweils mit einem oder mehreren Resten R⁷ substituiert sein können und wobei in den genannten Alkyl- und Alkoxygruppen eine oder mehrere CH₂-Gruppen durch -C≡C-, -R⁷C=CR⁷-, Si(R⁷)₂, C=O, C=NR⁷, -NR-, -O-,-S-, -C(=O)O- oder -C(=O)NR⁷- ersetzt sein können.

15. Verfahren zur Herstellung einer Verbindung nach einem oder mehreren der Ansprüche 1 bis 14, **dadurch gekennzeichnet, dass** eine in 1- und/oder in 4-Position mit einer Abgangsgruppe substituierte Phenanthrenverbindung in einer Kupplungsreaktion mit einer Diarylaminoverbindung oder mit einer mit einer Abgangsgruppe substituierten Triarylaminoverbindung umgesetzt wird.

16. Oligomer, Polymer oder Dendrimer, enthaltend eine oder mehrere Verbindungen nach einem oder mehreren der Ansprüche 1 bis 14, wobei die Bindung(en) zum Polymer, Oligomer oder Dendrimer an beliebigen, in Formel (I) mit R¹, R², R³, R⁴, R⁵, R⁶ oder R⁷ substituierten Positionen lokalisiert sein können.

17. Formulierung, enthaltend mindestens eine Verbindung nach einem oder mehreren der Ansprüche 1 bis 14 oder ein Polymer, Oligomer oder Dendrimer nach Anspruch 16, sowie mindestens ein Lösungsmittel.

18. Elektronische Vorrichtung, ausgewählt aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), enthaltend mindestens eine Verbindung nach einem oder mehreren der Ansprüche 1 bis 14.

19. Elektronische Vorrichtung nach Anspruch 18, ausgewählt aus organischen Elektrolumineszenzvorrichtungen, enthaltend Kathode, Anode und mindestens eine organische Schicht, enthaltend mindestens eine Verbindung nach einem oder mehreren der Ansprüche 1 bis 14.

20. Elektronische Vorrichtung nach Anspruch 19, ausgewählt aus organischen Elektrolumineszenzvorrichtungen, **dadurch gekennzeichnet, dass** die Verbindung nach einem oder mehreren der Ansprüche 1 bis 14 als Lochtransportmaterial in einer Lochtransportschicht, einer Elektronenblockierschicht oder einer Lochinjektionsschicht vorliegt, oder dass sie als emittierende Verbindung in einer emittierenden Schicht vorliegt, oder dass sie als Matrixverbindung zusammen mit einer oder mehreren emittierenden Verbindungen in einer emittierenden Schicht vorliegt.

## Claims

1. A compound of the formula (I) 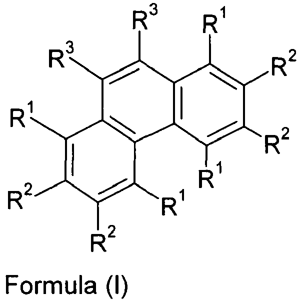 where the symbols that occur are as follows:
R¹ is the same or different at each instance and is selected from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems which have 6 to 40 aromatic ring atoms and may be substituted by one or more R⁶ radicals, heteroaromatic ring systems which have 5 to 40 aromatic ring atoms and may be substituted by one or more R⁶ radicals, groups of the formula (A) 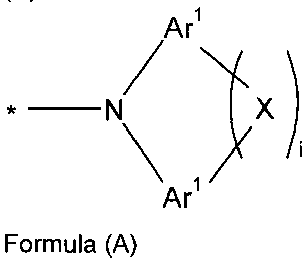 and groups of the formula (B) 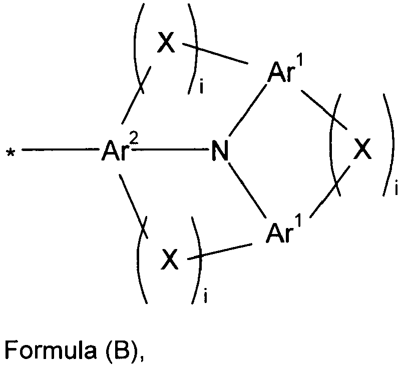 where the alkyl, alkoxy, alkenyl and alkynyl groups mentioned may each be substituted by one or more R⁶ radicals, and
where one or more CH₂ groups in the alkyl, alkoxy, alkenyl and alkynyl groups mentioned may be replaced by -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO or SO₂, and
where the bond marked with * in each of formula (A) and formula (B) denotes the bond to the phenanthrene unit;
R², R³ are the same or different at each instance and are selected from H, D, F, C(=O)R⁶, CN, Si(R⁶)₃, P(=O)(R⁶)₂, OR⁶, S(=O)R⁶, S(=O)₂R⁶, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems which have 6 to 40 aromatic ring atoms and may be substituted by one or more R⁶ radicals, and heteroaromatic ring systems which have 5 to 40 aromatic ring atoms and may be substituted by one or more R⁶ radicals,
where the alkyl, alkoxy, alkenyl and alkynyl groups mentioned may each be substituted by one or more R⁶ radicals, and
where one or more CH₂ groups in the alkyl, alkoxy, alkenyl and alkynyl groups mentioned may be replaced by -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO or SO₂;
R⁶ is the same or different at each instance and is selected from H, D, F, C(=O)R⁷, CN, Si(R⁷)₃, N(R⁷)₂, P(=O)(R⁷)₂, OR⁷, S(=O)R⁷, S(=O)₂R⁷, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems which have 6 to 40 aromatic ring atoms and may be substituted by one or more R⁷ radicals, and heteroaromatic ring systems which have 5 to 40 aromatic ring atoms and may be substituted by one or more R⁷ radicals,
where the alkyl, alkoxy, alkenyl and alkynyl groups mentioned may each be substituted by one or more R⁷ radicals,
where one or more CH₂ groups in the alkyl, alkoxy, alkenyl and alkynyl groups mentioned may be replaced by -R⁷C=CR⁷-, -C≡C-, Si(R⁷)₂, C=O, C=NR⁷, -C(=O)O-, -C(=O)NR⁷-, NR⁷, P(=O)(R⁷), -O-, -S-, SO or SO₂, and
where two or more R⁶ substituents may be joined to one another and may form a ring;
R⁷ is the same or different at each instance and is selected from H, D, F, CN and aliphatic, aromatic or heteroaromatic organic radicals having 1 to 20 carbon atoms, in which one or more hydrogen atoms may also be replaced by D, F or CN;
Ar¹ is the same or different at each instance and is selected from aromatic ring systems which have 6 to 40 aromatic ring atoms and may be substituted by one or more R⁴ radicals, and heteroaromatic ring systems which have 5 to 40 aromatic ring atoms and may be substituted by one or more R⁴ radicals;
Ar² is the same or different at each instance and is selected from aromatic ring systems which have 6 to 40 aromatic ring atoms and may be substituted by one or more R⁴ radicals, and heteroaromatic ring systems which have 5 to 40 aromatic ring atoms and may be substituted by one or more R⁴ radicals;
R⁴ is the same or different at each instance and is selected from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems which have 6 to 40 aromatic ring atoms and may be substituted by one or more R⁶ radicals, and heteroaromatic ring systems which have 5 to 40 aromatic ring atoms and may be substituted by one or more R⁶ radicals,
where the alkyl, alkoxy, alkenyl and alkynyl groups mentioned may each be substituted by one or more R⁶ radicals,
where one or more CH₂ groups in the alkyl, alkoxy, alkenyl and alkynyl groups mentioned may be replaced by -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO or SO₂, and
where two or more R⁴ substituents may be joined to one another and may form a ring;
X is the same or different at each instance and is selected from single bonds, BR⁵, C(R⁵)₂, -C(R⁵)₂-C(R⁵)₂-, -C(R⁵)=C(R⁵)-, -C(R⁵)₂-O-, -C(R⁵)₂-NR⁵-, Si(R⁵)₂, C=O, NR⁵, PR⁵, P(=O)R⁵, O, S, S=O, SO₂ and ortho-phenylene optionally substituted by R⁵ radicals;
R⁵ is the same or different at each instance and is selected from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems which have 6 to 40 aromatic ring atoms and may be substituted by one or more R⁶ radicals, and heteroaromatic ring systems which have 5 to 40 aromatic ring atoms and may be substituted by one or more R⁶ radicals,
where the alkyl, alkoxy, alkenyl and alkynyl groups mentioned may each be substituted by one or more R⁶ radicals,
where one or more CH₂ groups in the alkyl, alkoxy, alkenyl and alkynyl groups mentioned may be replaced by -R⁶C=CR⁶-, -C≡C-, Si(R⁶)₂, C=O, C=NR⁶, -C(=O)O-, , -C(=O)NR⁶-, NR⁶, P(=O)(R⁶), -O-, -S-, SO or SO₂, and
where two or more R⁵ substituents may be joined to one another and may form a ring;
i is the same or different at each instance and is 0 or 1,
where at least one R¹ radical in the compound of the formula (I) is selected from groups of the formula (A) and groups of the formula (B).

2. A compound as claimed in claim 1, **characterized in that** exactly one R¹ radical in the compound of the formula (I) is selected from groups of the formula (A) and groups of the formula (B), or **in that** exactly two R¹ radicals in the compound of the formula (I) are selected from groups of the formula (A) and groups of the formula (B).

3. A compound as claimed in claim 1 or 2, **characterized in that** exactly two R¹ radicals in the compound of the formula (I) are selected from groups of the formula (A) and groups of the formula (B), where exactly one of these R¹ radicals is bonded in the 1 position of the phenanthrene and exactly one of these R¹ radicals is bonded in the 4 position of the phenanthrene.

4. A compound as claimed in one or more of claims 1 to 3, **characterized in that** one or two indices i per group of the formula (B) are 1.

5. A compound as claimed in one or more of claims 1 to 3, **characterized in that** the index i at each instance is 0.

6. A compound as claimed in one or more of claims 1 to 5, **characterized in that** the (A) group is the same or different at each instance and is selected from the formulae (A-1) to (A-7)
| | |
|---|---|
| 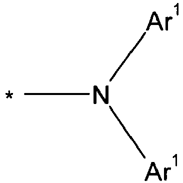 | 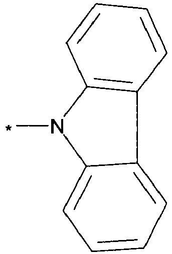 |
| Formula (A-1) | Formula (A-2) |
| | |
| 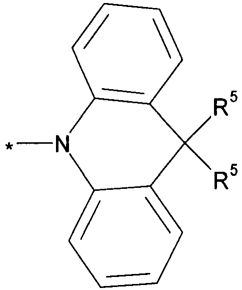 | 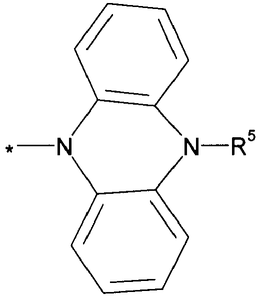 |
| Formula (A-3) | Formula (A-4) |
| | |
| 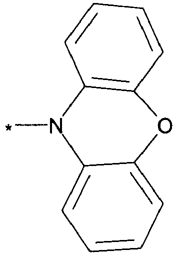 | 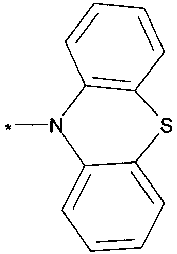 |
| Formula (A-5) | Formula (A-6) |
| 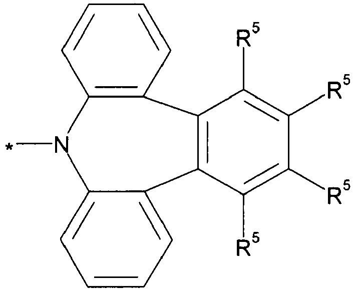 | |
| Formula (A-7) | |
which may each be substituted by R⁴ radicals at the unoccupied positions, and where the bond marked with * represents the position of attachment to the phenanthrenyl base skeleton.

7. A compound as claimed in one or more of claims 1 to 6, **characterized in that** the (B) group is the same or different at each instance and is selected from the following formulae (B-1) to (B-15):
| | |
|---|---|
| 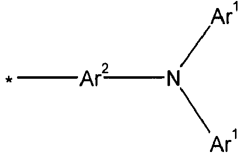 | 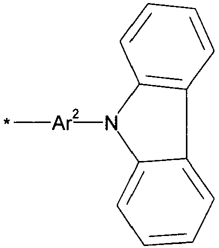 |
| Formula (B-1) | Formula (B-2) |
| 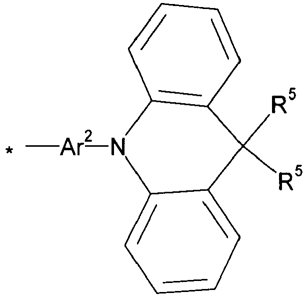 | 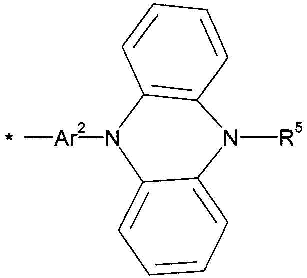 |
| Formula (B-3) | Formula (B-4) |
| | |
| 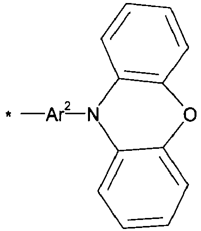 | 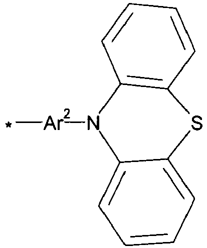 |
| Formula (B-5) | Formula (B-6) |
| | |
| 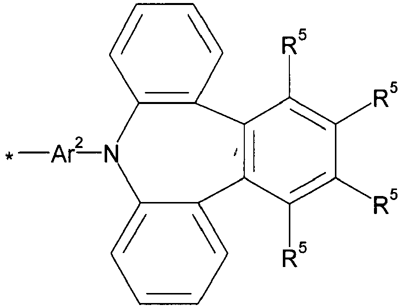 | 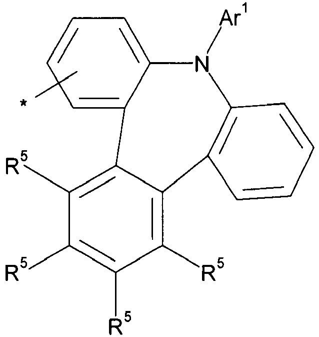 |
| Formula (B-7) | Formula (B-8) |
| | |
| 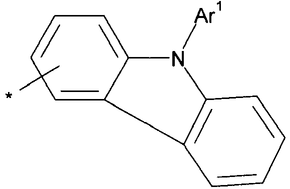 | 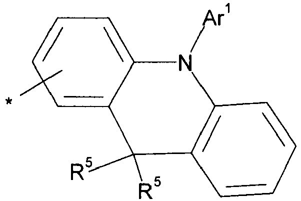 |
| Formula (B-9) | Formula (B-10) |
| | |
| 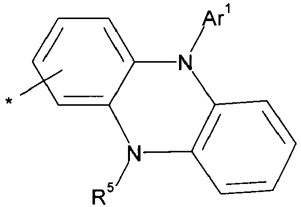 | 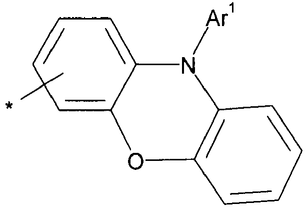 |
| Formula (B-11) | Formula (B-12) |
| | |
| 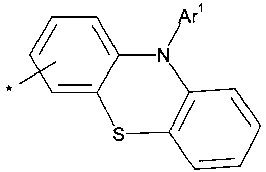 | 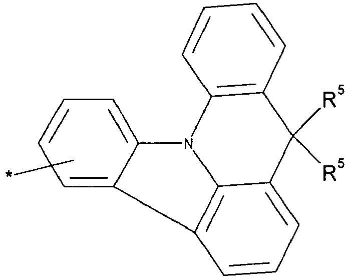 |
| Formula (B-13) | Formula (B-14) |
| | |
| 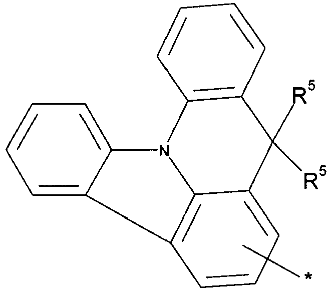 | |
| Formula (B-15) | |
which may each be substituted by R⁴ radicals at the unoccupied positions, and where the bond marked with * represents the position of attachment to the phenanthrenyl base skeleton.

8. A compound as claimed in one or more of claims 1 to 7, **characterized in that** Ar¹ is the same or different at each instance and is selected from aromatic ring systems which have 12 to 24 aromatic ring atoms and may be substituted by one or more R⁴ radicals.

9. A compound as claimed in one or more of claims 1 to 8, **characterized in that** two Ar¹ groups bonded to the same nitrogen atom in formula (A) or formula (B) are not the same.

10. A compound as claimed in one or more of claims 1 to 9, **characterized in that** the Ar¹ groups each contain at least one group selected from benzene, naphthalene, phenanthrene, fluoranthene, biphenyl, terphenyl, quaterphenyl, fluorene, spirobifluorene, furan, benzofuran, isobenzofuran, dibenzofuran, thiophene, benzothiophene, isobenzothiophene, dibenzothiophene, indole, isoindole, carbazole, indolocarbazole, indenocarbazole, pyridine, quinoline, isoquinoline, acridine, phenanthridine, benzimidazole, pyrimidine, pyrazine and triazine, where the groups mentioned may each be substituted by one or more R⁴ radicals.

11. A compound as claimed in one or more of claims 1 to 10, **characterized in that** Ar² is the same or different at each instance and is selected from aromatic ring systems which have 6 to 13 aromatic ring atoms and may be substituted by one or more R⁴ radicals.

12. A compound as claimed in one or more of claims 1 to 11, **characterized in that** X groups are preferably selected from single bonds, C(R⁵)₂, -C(R⁵)₂-C(R⁵)₂-, -C(R⁵)=C(R⁵)-, Si(R⁵)₂, C=O, NR⁵, O, S, S=O, SO₂ and ortho-phenylene optionally substituted by R⁵ radicals.

13. A compound as claimed in one or more of claims 1 to 12, **characterized in that** R⁴ and R⁵ are the same or different at each instance and are selected from H, D, F, CN, Si(R⁶)₃, straight-chain alkyl or alkoxy groups having 1 to 20 carbon atoms, branched or cyclic alkyl or alkoxy groups having 3 to 20 carbon atoms, aromatic ring systems which have 6 to 24 aromatic ring atoms and may be substituted by one or more R⁶ radicals, and heteroaromatic ring systems which have 5 to 24 aromatic ring atoms and may be substituted by one or more R⁶ radicals,
where the alkyl and alkoxy groups mentioned may each be substituted by one or more R⁶ radicals and where one or more CH₂ groups in the alkyl and alkoxy groups mentioned may be replaced by -C≡C-, -R⁶C=CR⁶-, Si(R⁶)₂, C=O, C=NR⁶, -NR⁶-, -O-, -S-, -C(=O)O- or -C(=O)NR⁶-.

14. A compound as claimed in one or more of claims 1 to 13, **characterized in that** R⁶ is the same or different at each instance and is selected from H, D, F, CN, Si(R⁷)₃, straight-chain alkyl or alkoxy groups having 1 to 20 carbon atoms, branched or cyclic alkyl or alkoxy groups having 3 to 20 carbon atoms, aromatic ring systems which have 6 to 24 aromatic ring atoms and may be substituted by one or more R⁷ radicals, and heteroaromatic ring systems which have 5 to 24 aromatic ring atoms and may be substituted by one or more R⁷ radicals,
where the alkyl and alkoxy groups mentioned may each be substituted by one or more R⁷ radicals and where one or more CH₂ groups in the alkyl and alkoxy groups mentioned may be replaced by -C≡C-, -R⁷C=CR⁷-, Si(R⁷)₂, C=O, C=NR⁷, -NR⁷- -O-, -S-, -C(=O)O- or -C(=O)NR⁷-.

15. A process for preparing a compound as claimed in one or more of claims 1 to 14, **characterized in that** a phenanthrene compound substituted by a leaving group in the 1 and/or 4 position is reacted in a coupling reaction with a diarylamino compound or with a triarylamino compound substituted by a leaving group.

16. An oligomer, polymer or dendrimer containing one or more compounds as claimed in one or more of claims 1 to 14, wherein the bond(s) to the polymer, oligomer or dendrimer may be localized at any desired positions substituted by R¹, R², R³, R⁴, R⁵, R⁶ or R⁷ in formula (I).

17. A formulation comprising at least one compound as claimed in one or more of claims 1 to 14, or a polymer, oligomer or dendrimer as claimed in claim 16, and at least one solvent.

18. An electronic device selected from organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), comprising at least one compound as claimed in one or more of claims 1 to 14.

19. The electronic device as claimed in claim 18, selected from organic electroluminescent devices comprising cathode, anode and at least one organic layer comprising at least one compound as claimed in one or more of claims 1 to 14.

20. The electronic device as claimed in claim 19, selected from organic electroluminescent devices, **characterized in that** the compound as claimed in one or more of claims 1 to 14 is present as hole transport material in a hole transport layer, an electron blocker layer or a hole injection layer, or **in that** it is present as emitting compound in an emitting layer, or **in that** it is present as matrix compound together with one or more emitting compounds in an emitting layer.

## Revendications

1. Composé de la formule (I) : 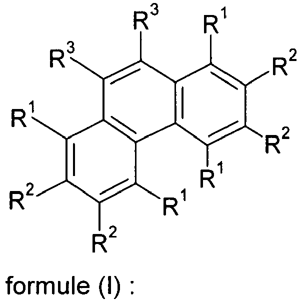 formule dans laquelle ce qui suit s'applique aux symboles présents :
R¹ est sélectionné pour chaque occurrence, de manière identique ou différente, parmi H, D, F, C(=O)R⁶, CN, Si(R⁶)₃, N(R⁶)₂, P(=O)(R⁶)₂, OR⁶, S(=O)R⁶, S(=O)₂R⁶, des groupes alkyle ou alcoxy en chaîne droite qui comportent 1 à 20 atome(s) de C, des groupes alkyle ou alcoxy ramifiés ou cycliques qui comportent 3 à 20 atomes de C, des groupes alkényle ou alkynyle qui comportent 2 à 20 atomes de C, des systèmes de cycle aromatique qui comportent 6 à 40 atomes de cycle aromatique, lesquels peuvent être substitués par un radical ou plusieurs radicaux R⁶, des systèmes de cycle hétéroaromatique qui comportent 5 à 40 atomes de cycle aromatique, lesquels peuvent être substitués par un radical ou plusieurs radicaux R⁶, des groupes de la formule (A) : 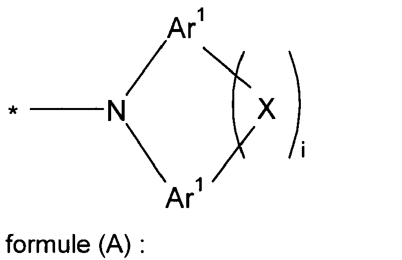 et des groupes de la formule (B) : 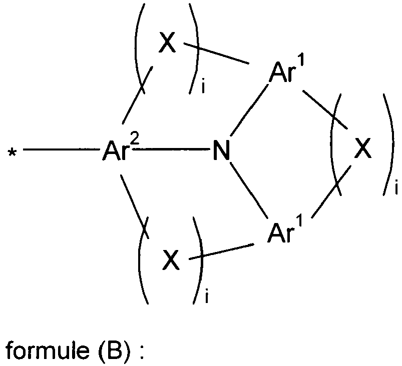 formules dans lesquelles lesdits groupes alkyle, alcoxy, alkényle et alkynyle peuvent chacun être substitués par un radical ou plusieurs radicaux R⁶ ; et
formules dans lesquelles un ou plusieurs groupe(s) CH₂ dans lesdits groupes alkyle, alcoxy, alkényle et alkynyle peut/ peuvent être remplacé(s) par -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO ou SO₂, et
formules dans lesquelles la liaison marquée par * dans la formule (A) et dans la formule (B), dans chaque cas, représente la liaison sur l'unité phénanthrène ;
R², R³ sont sélectionnés pour chaque occurrence, de manière identique ou différente, parmi H, D, F, C(=O)R⁶, CN, Si(R⁶)₃, P(=O)(R⁶)₂, OR⁶, S(=O)R⁶, S(=O)₂R⁶, des groupes alkyle ou alcoxy en chaîne droite qui comportent 1 à 20 atome(s) de C, des groupes alkyle ou alcoxy ramifiés ou cycliques qui comportent 3 à 20 atomes de C, des groupes alkényle ou alkynyle qui comportent 2 à 20 atomes de C, des systèmes de cycle aromatique qui comportent 6 à 40 atomes de cycle aromatique, lesquels peuvent être substitués par un radical ou plusieurs radicaux R⁶, et des systèmes de cycle hétéroaromatique qui comportent 5 à 40 atomes de cycle aromatique, lesquels peuvent être substitués par un radical ou plusieurs radicaux R⁶ ;
formules dans lesquelles lesdits groupes alkyle, alcoxy, alkényle et alkynyle peuvent chacun être substitués par un radical ou plusieurs radicaux R⁶ ; et
formules dans lesquelles un ou plusieurs groupe(s) CH₂ dans lesdits groupes alkyle, alcoxy, alkényle et alkynyle peut/ peuvent être remplacé(s) par -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO ou SO₂ ;
R⁶ est sélectionné pour chaque occurrence, de manière identique ou différente, parmi H, D, F, C(=O)R⁷, CN, Si(R⁷)₃, N(R⁷)₂, P(=O)(R⁷)₂, OR⁷, S(=O)R⁷, S(=O)₂R⁷, des groupes alkyle ou alcoxy en chaîne droite qui comportent 1 à 20 atome(s) de C, ou des groupes alkyle ou alcoxy ramifiés ou cycliques qui comportent 3 à 20 atomes de C, des groupes alkényle ou alkynyle qui comportent 2 à 20 atomes de C, des systèmes de cycle aromatique qui comportent 6 à 40 atomes de cycle aromatique, lesquels peuvent être substitués par un radical ou plusieurs radicaux R⁷, et des systèmes de cycle hétéroaromatique qui comportent 5 à 40 atomes de cycle aromatique, lesquels peuvent être substitués par un radical ou plusieurs radicaux R⁷ ;
formules dans lesquelles lesdits groupes alkyle, alcoxy, alkényle et alkynyle peuvent chacun être substitués par un radical ou plusieurs radicaux R⁷ ;
formules dans lesquelles un ou plusieurs groupe(s) CH₂ dans lesdits groupes alkyle, alcoxy, alkényle et alkynyle peut/ peuvent être remplacé(s) par -R⁷C=CR⁷-, -C≡C-, Si(R⁷)₂, C=O, C=NR⁷, -C(=O)O-, -C(=O)NR⁷-, NR⁷, P(=O)(R⁷), -O-, -S-, SO ou SO₂ ; et
formules dans lesquelles deux substituants R⁶ ou plus peuvent être liés l'un à l'autre ou les uns aux autres et peuvent former un cycle ;
R⁷ est sélectionné pour chaque occurrence, de manière identique ou différente, parmi H, D, F, CN, et des radicaux organiques aliphatiques, aromatiques ou hétéroaromatiques qui comportent 1 à 20 atome(s) de C, où, en outre, un ou plusieurs atome(s) de H peut/peuvent être remplacé(s) par D, F ouCN;
Ar¹ est sélectionné pour chaque occurrence, de manière identique ou différente, parmi des systèmes de cycle aromatique qui comportent 6 à 40 atomes de cycle aromatique, lesquels peuvent être substitués par un radical ou plusieurs radicaux R⁴, et des systèmes de cycle hétéroaromatique qui comportent 5 à 40 atomes de cycle aromatique, lesquels peuvent être substitués par un radical ou plusieurs radicaux R⁴ ;
Ar² est sélectionné pour chaque occurrence, de manière identique ou différente, parmi des systèmes de cycle aromatique qui comportent 6 à 40 atomes de cycle aromatique, lesquels peuvent être substitués par un radical ou plusieurs radicaux R⁴, et des systèmes de cycle hétéroaromatique qui comportent 5 à 40 atomes de cycle aromatique, lesquels peuvent être substitués par un radical ou plusieurs radicaux R⁴ ;
R⁴ est sélectionné pour chaque occurrence, de manière identique ou différente, parmi H, D, F, C(=O)R⁶, CN, Si(R⁶)₃, N(R⁶)₂, P(=O)(R⁶)₂, OR⁶, S(=O)R⁶, S(=O)₂R⁶, des groupes alkyle ou alcoxy en chaîne droite qui comportent 1 à 20 atome(s) de C, des groupes alkyle ou alcoxy ramifiés ou cycliques qui comportent 3 à 20 atomes de C, des groupes alkényle ou alkynyle qui comportent 2 à 20 atomes de C, des systèmes de cycle aromatique qui comportent 6 à 40 atomes de cycle aromatique, lesquels peuvent être substitués par un radical ou plusieurs radicaux R⁶, et des systèmes de cycle hétéroaromatique qui comportent 5 à 40 atomes de cycle aromatique, lesquels peuvent être substitués par un radical ou plusieurs radicaux R⁶ ;
formules dans lesquelles lesdits groupes alkyle, alcoxy, alkényle et alkynyle peuvent chacun être substitués par un radical ou plusieurs radicaux R⁶ ;
formules dans lesquelles un ou plusieurs groupe(s) CH₂ dans lesdits groupes alkyle, alcoxy, alkényle et alkynyle peut/ peuvent être remplacé(s) par -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO ou SO₂ ; et
formules dans lesquelles deux substituants R⁴ ou plus peuvent être liés l'un à l'autre ou les uns aux autres et peuvent former un cycle ;
X est sélectionné pour chaque occurrence, de manière identique ou différente, parmi des liaisons simples, BR⁵, C(R⁵)₂, -C(R⁵)₂-C(R⁵)₂-, -C(R⁵)=C(R⁵)-, -C(R⁵)₂-O-, -C(R⁵)₂-NR⁵-, Si(R⁵)₂, C=O, NR⁵, PR⁵, P(=O)R⁵, O, S, S=O, SO₂ et un ortho-phénylène, lequel est en option substitué par des radicaux R⁵ ;
R⁵ est sélectionné pour chaque occurrence, de manière identique ou différente, parmi H, D, F, C(=O)R⁶, CN, Si(R⁶)₃, N(R⁶)₂, P(=O)(R⁶)₂, OR⁶, S(=O)R⁶, S(=O)₂R⁶, des groupes alkyle ou alcoxy en chaîne droite qui comportent 1 à 20 atome(s) de C, des groupes alkyle ou alcoxy ramifiés ou cycliques qui comportent 3 à 20 atomes de C, des groupes alkényle ou alkynyle qui comportent 2 à 20 atomes de C, des systèmes de cycle aromatique qui comportent 6 à 40 atomes de cycle aromatique, lesquels peuvent être substitués par un radical ou plusieurs radicaux R⁶, et des systèmes de cycle hétéroaromatique qui comportent 5 à 40 atomes de cycle aromatique, lesquels peuvent être substitués par un radical ou plusieurs radicaux R⁶ ;
formules dans lesquelles lesdits groupes alkyle, alcoxy, alkényle et alkynyle peuvent chacun être substitués par un radical ou plusieurs radicaux R⁶ ;
formules dans lesquelles un ou plusieurs groupe(s) CH₂ dans lesdits groupes alkyle, alcoxy, alkényle et alkynyle peut/ peuvent être remplacé(s) par -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO ou SO₂ ; et
formules dans lesquelles deux substituants R⁵ ou plus peuvent être liés l'un à l'autre ou les uns aux autres et peuvent former un cycle ;
i est pour chaque occurrence, de manière identique ou différente, 0 ou 1,
formules dans lesquelles au moins un radical R¹ dans le composé de la formule (I) est sélectionné parmi des groupes de la formule (A) et des groupes de la formule (B).

2. Composé selon la revendication 1, **caractérisé en ce que** précisément un radical R¹ dans le composé de la formule (I) est sélectionné parmi des groupes de la formule (A) et des groupes de la formule (B), ou **en ce que** précisément 2 radicaux R¹ dans le composé de la formule (I) sont sélectionnés parmi des groupes de la formule (A) et des groupes de la formule (B).

3. Composé selon la revendication 1 ou 2, **caractérisé en ce que** précisément 2 radicaux R¹ dans le composé de la formule (I) sont sélectionnés parmi des groupes de la formule (A) et des groupes de la formule (B), où précisément l'un de ces radicaux R¹ est lié au phénanthrène à la position 1 et précisément l'un de ces radicaux R¹ est lié au phénanthrène à la position 4.

4. Composé selon une ou plusieurs des revendications 1 à 3, **caractérisé en ce qu'**un ou deux indice(s) i par groupe de la formule (B) est/ sont égal/égaux à 1.

5. Composé selon une ou plusieurs des revendications 1 à 3, **caractérisé en ce que** l'indice i est pour chaque occurrence égal à 0.

6. Composé selon une ou plusieurs des revendications 1 à 5, **caractérisé en ce que** le groupe (A) est sélectionné pour chaque occurrence, de manière identique ou différente, parmi les formules (A-1) à (A-7) :
| | |
|---|---|
| 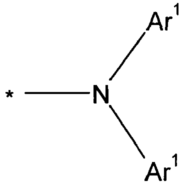 | 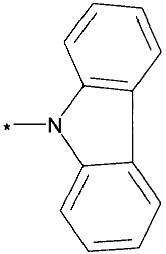 |
| formule (A-1) | formule (A-2) |
| | |
| 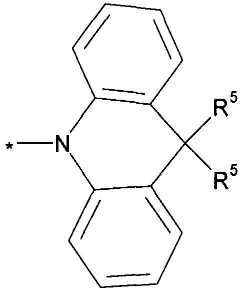 | 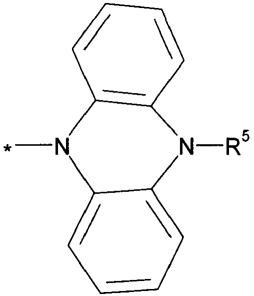 |
| formule (A-3) | formule (A-4) |
| 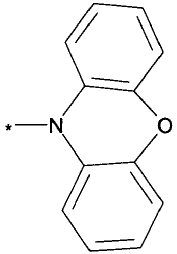 | 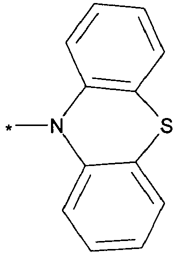 |
| formule (A-5) | formule (A-6) |
| | |
| 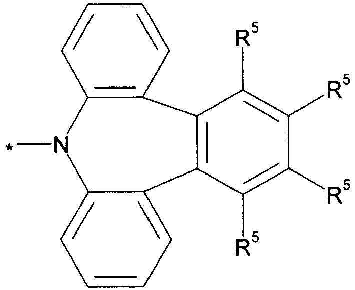 | |
| formule (A-7) | |
lequel peut dans chaque cas être substitué au niveau des positions libres par des radicaux R⁴, et où la liaison marquée par * représente la position de liaison sur l'ossature phénanthrényle.

7. Composé selon une ou plusieurs des revendications 1 à 6, **caractérisé en ce que** le groupe (B) est sélectionné pour chaque occurrence, de manière identique ou différente, parmi les formules (B-1) à (B-15) qui suivent :
| | |
|---|---|
| 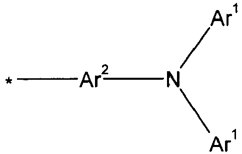 | 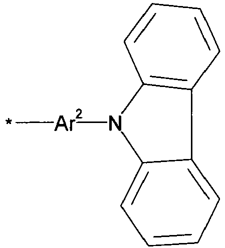 |
| formule (B-1) | formule (B-2) |
| | |
| 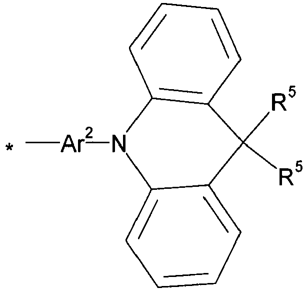 | 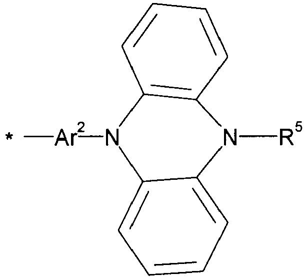 |
| formule (B-3) | formule (B-4) |
| | |
| 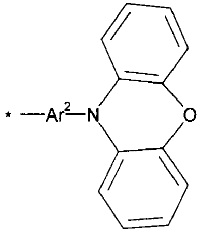 | 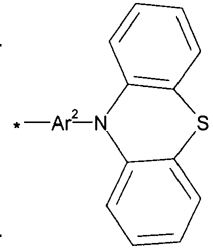 |
| formule (B-5) | formule (B-6) |
| 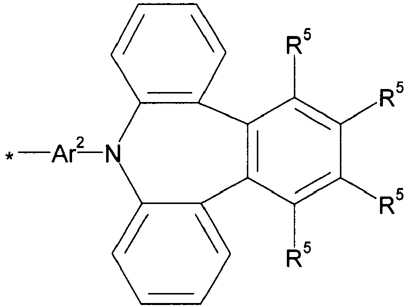 | 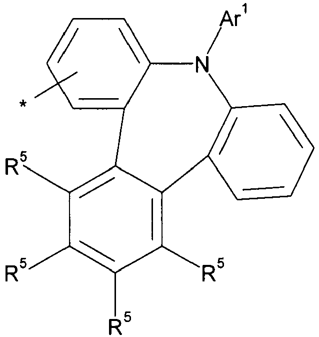 |
| formule (B-7) | formule (B-8) |
| | |
| 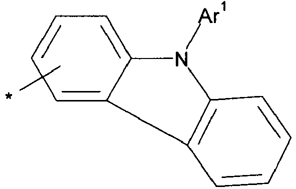 | 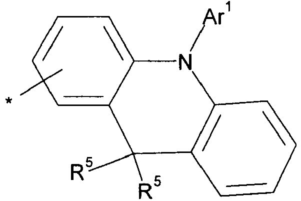 |
| formule (B-9) | formule (B-10) |
| | |
| 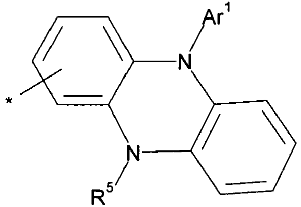 | 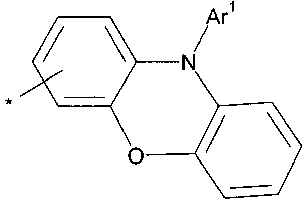 |
| formule (B-11) | formule (B-12) |
| | |
| 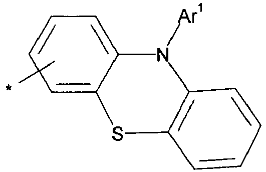 | 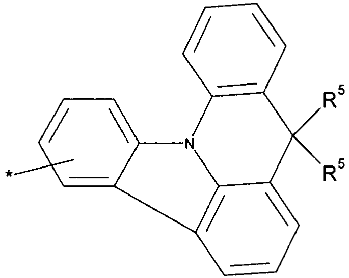 |
| formule (B-13) | formule (B-14) |
| 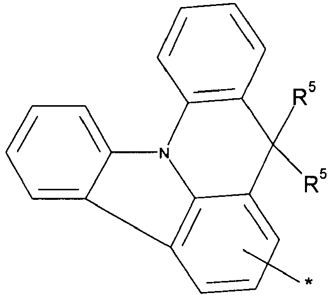 | |
| formule (B-15) | |
lequel peut dans chaque cas être substitué au niveau des positions libres par des radicaux R⁴, et où la liaison marquée par * représente la position de liaison sur l'ossature phénanthrényle.

8. Composé selon une ou plusieurs des revendications 1 à 7, **caractérisé en ce que** Ar¹ est sélectionné pour chaque occurrence, de manière identique ou différente, parmi des systèmes de cycle aromatique qui comportent 12 à 24 atomes de cycle aromatique, lesquels peuvent être substitués par un radical ou plusieurs radicaux R⁴.

9. Composé selon une ou plusieurs des revendications 1 à 8, **caractérisé en ce que** deux groupes Ar¹ qui sont liés au même atome d'azote dans la formule (A) ou dans la formule (B) ne sont pas sélectionnés de manière identique.

10. Composé selon une ou plusieurs des revendications 1 à 9, **caractérisé en ce que** les groupes Ar¹ contiennent chacun au moins un groupe qui est sélectionné parmi benzène, naphtalène, phénanthrène, fluoranthène, biphényle, terphényle, quaterphényle, fluorène, spirobifluoréne, furane, benzofurane, isobenzofurane, dibenzofurane, thiophène, benzothiophène, isobenzothiophène, dibenzothiophène, indole, isoindole, carbazole, indolocarbazole, indénocarbazole, pyridine, quinoline, isoquinoline, acridine, phénanthridine, benzimidazole, pyrimidine, pyrazine et triazine, où lesdits groupes peuvent chacun être substitués par un radical ou plusieurs radicaux R⁴.

11. Composé selon une ou plusieurs des revendications 1 à 10, **caractérisé en ce que** Ar² est sélectionné pour chaque occurrence, de manière identique ou différente, parmi des systèmes de cycle aromatique qui comportent 6 à 13 atomes de cycle aromatique, lesquels peuvent être substitués par un radical ou plusieurs radicaux R⁴.

12. Composé selon une ou plusieurs des revendications 1 à 11, **caractérisé en ce que** des groupes X sont de préférence sélectionnés parmi des liaisons, C(R⁵)₂, -C(R⁵)₂-C(R⁵)₂-, -C(R⁵)=C(R⁵)-, Si(R⁵)₂, C=O, NR⁵, O, S, S=O, SO₂ et ortho-phénylène, lequel est en option substitué par des radicaux R⁵.

13. Composé selon une ou plusieurs des revendications 1 à 12, **caractérisé en ce que** R⁴ et R⁵ sont sélectionnés pour chaque occurrence, de manière identique ou différente, parmi H, D, F, CN, Si(R⁶)₃, des groupes alkyle ou alcoxy en chaîne droite qui comportent 1 à 20 atome(s) de C, des groupes alkyle ou alcoxy ramifiés ou cycliques qui comportent 3 à 20 atomes de C, des systèmes de cycle aromatique qui comportent 6 à 24 atomes de cycle aromatique, lesquels peuvent être substitués par un radical ou plusieurs radicaux R⁶, et des systèmes de cycle hétéroaromatique qui comportent 5 à 24 atomes de cycle aromatique, lesquels peuvent être substitués par un radical ou plusieurs radicaux R⁶ ;
où lesdits groupes alkyle et alcoxy peuvent chacun être substitués par un radical ou plusieurs radicaux R⁶ et où un ou plusieurs groupe(s) CH₂ dans lesdits groupes alkyle et alcoxy peut/peuvent être remplacé(s) par -C≡C-, -R⁶C=CR⁶-, Si(R⁶)₂, C=O, C=NR⁶, -NR⁶-, -O-, -S-, -C(=O)O- ou -C(=O)NR⁶-.

14. Composé selon une ou plusieurs des revendications 1 à 13, **caractérisé en ce que** R⁶ est sélectionné pour chaque occurrence, de manière identique ou différente, parmi H, D, F, CN, Si(R⁷)₃, des groupes alkyle ou alcoxy en chaîne droite qui comportent 1 à 20 atome(s) de C, des groupes alkyle ou alcoxy ramifiés ou cycliques qui comportent 3 à 20 atomes de C, des systèmes de cycle aromatique qui comportent 6 à 24 atomes de cycle aromatique, lesquels peuvent être substitués par un radical ou plusieurs radicaux R⁷, et des systèmes de cycle hétéroaromatique qui comportent 5 à 24 atomes de cycle aromatique, lesquels peuvent être substitués par un radical ou plusieurs radicaux R⁷ ;
où lesdits groupes alkyle et alcoxy peuvent chacun être substitués par un radical ou plusieurs radicaux R⁷ et où un ou plusieurs groupe(s) CH₂ dans lesdits groupes alkyle et alcoxy peut/peuvent être remplacé(s) par -C=C-, -R⁷C=CR⁷-, Si(R⁷)₂, C=O, C=NR⁷, -NR⁷-, -O-, -S-, -C(=O)O- ou -C(=O)NR⁷-.

15. Procédé pour la préparation d'un composé selon une ou plusieurs des revendications 1 à 14, **caractérisé en ce qu'**un composé phénanthrène qui est substitué à la position/aux positions 1 et/ou 4 par un groupe partant est amené à réagir selon une réaction de couplage avec un composé diarylamino ou avec un composé triarylamino qui est substitué par un groupe partant.

16. Oligomère, polymère ou dendrimère, contenant un ou plusieurs composé(s) selon une ou plusieurs des revendications 1 à 14, dans lequel la/les liaison(s) sur le polymère, l'oligomère ou le dendrimère peut/peuvent être localisée(s) au niveau de n'importe quelles positions souhaitées dans la formule (I) qui sont substituées par R¹, R², R³, R⁴, R⁵, R⁶ ou R⁷.

17. Formulation comprenant au moins un composé selon une ou plusieurs des revendications 1 à 14 ou polymère, oligomère ou dendrimère selon la revendication 16, et au moins un solvant.

18. Dispositif électronique sélectionné parmi les circuits intégrés organiques (OIC), les transistors à effet de champ organiques (OFET), les transistors à film mince organiques (OTFT), les transistors à émission de lumière organiques (OLET), les cellules solaires organiques (OSC), les détecteurs optiques organiques, les photorécepteurs organiques, les dispositifs à extinction de champ organiques (OFQD), les cellules électrochimiques à émission de lumière organiques (OLEC), les diodes laser organiques (O-laser) et les dispositifs électroluminescents organiques (OLED), contenant au moins un composé selon une ou plusieurs des revendications 1 à 14.

19. Dispositif électronique selon la revendication 18, sélectionné parmi les dispositifs électroluminescents organiques qui contiennent une cathode, une anode et au moins une couche organique comprenant au moins un composé selon une ou plusieurs des revendications 1 à 14.

20. Dispositif électronique selon la revendication 19, sélectionné parmi les dispositifs électroluminescents organiques, **caractérisé en ce que** le composé selon une ou plusieurs des revendications 1 à 14 est présent en tant que matériau de transport de trous dans une couche de transport de trous, une couche de blocage d'électrons ou une couche d'injection de trous, ou **en ce qu'**il est présent en tant que composé d'émission dans une couche d'émission, ou **en ce qu'**il est présent en tant que composé de matrice en association avec un ou plusieurs composé(s) d'émission dans une couche d'émission.
